(19) Europäisches Patentamt
European Patent Office
Office européen des brevets

(11) EP 1 016 646 A1

(12) DEMANDE DE BREVET EUROPEEN

(43) Date de publication:
**05.07.2000 Bulletin 2000/27**

(21) Numéro de dépôt: **00200918.1**

(22) Date de dépôt: **11.12.1992**

(51) Int Cl.⁷: **C07C 59/48**, C07C 59/64, C07C 69/732, C07C 239/20, C07C 255/57, C07D 277/40

(84) Etats contractants désignés:
**AT BE CH DE DK ES FR GB GR IE IT LI LU NL PT SE**

(30) Priorité: **12.12.1991 FR 9115416**
**28.09.1992 FR 9211520**

(62) Numéro(s) de document de la (des) demande(s) initiale(s) en application de l'article 76 CBE:
**92403361.6 / 0 551 034**

(71) Demandeur: **HOECHST MARION ROUSSEL**
**92800 Puteaux (FR)**

(72) Inventeurs:
- **Aszodi, Jozsef**
  **77340 Pontault Combault (FR)**
- **Chantot, Jean-François**
  **77410 Gressy en France (FR)**
- **Fauveau, Patrick**
  **93190 Livry Gargan (FR)**
- **D'Ambrieres, Gouin**
  **75020 Paris (FR)**
- **Humbert, Daniel**
  **94120 Fontenay sous Bois (FR)**

(74) Mandataire: **Vieillefosse, Jean-Claude et al**
**102, Route de Noisy**
**93235 Romainville Cedex (FR)**

Remarques:
Cette demande a été déposée le 14 - 03 - 2000 comme demande divisionnaire de la demande mentionnée sous le code INID 62.

# (54) Nouveaux produits intermédiaires pour la préparation de céphalosporines comportant en position 7 un radical benzyloxyimino substitué

(57) L'invention a pour objet les produits de formules

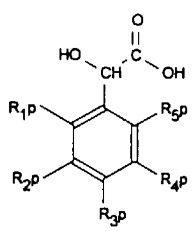
(III)

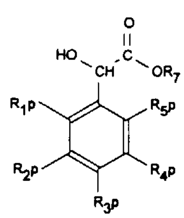
(IV)

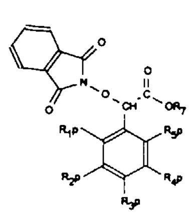
(V)

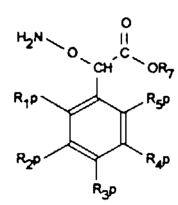
(VI)

EP 1 016 646 A1

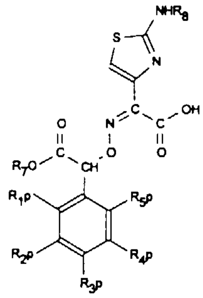

(VIII)

intermédiaires pour la préparation des produits de formule générale (I) :

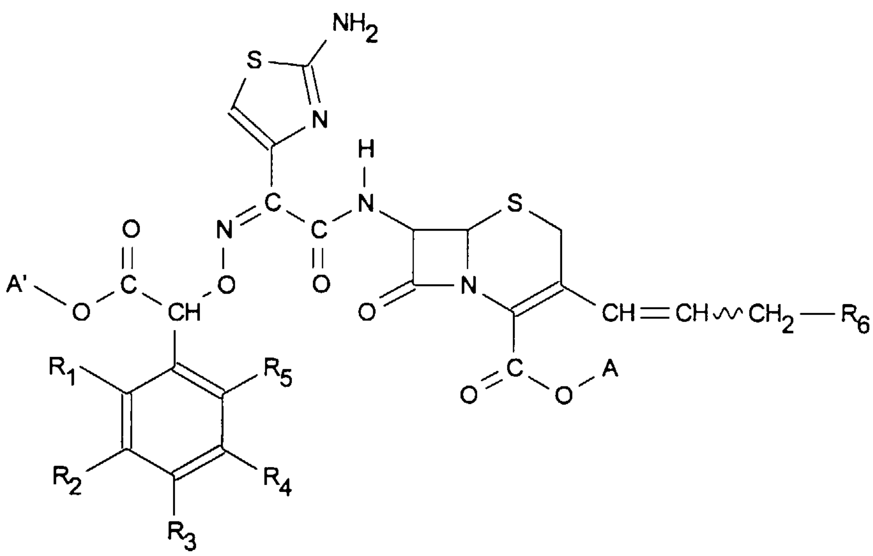

(I)

décrits dans la demande principale EP-A-0551034.

R1p, R2p, R3p, R4p et R5p ont respectivement les valeurs de R1, R2, R3, R4 et R5 décrites dans la demande principale ou une fonction protégée, R3 ou R5 étant identique à R4, étant entendu que soit R1, R2 ou R3, soit R2, R4 ou R5 est nécessairement F, Br, I ou CN ; soit R1 et R5 sont différents d'hydrogène ; soit R2 = R5 = Cl ; R7 est un reste d'ester clivable et R8 est hydrogène ou un groupe protecteur.

## Description

La présente invention concerne de nouveaux produits intermédiaires pour la préparation de céphalosporines comportant sur la chaîne latérale en position 7, un radical benzyloxyimino substitué.

L'art antérieur et, plus particulièrement les documents EP-A-315518, EP-A-266060, WO-A-86/05787 et GB-A-2134522 décrivent des composés de type cephalosporines dont le radical amino en position 7 est substitué par une chaîne de type aminothiazolyl 2-hydroxy ou méthoxy iminoacétamido et qui soit comportent en position 3 un radical propényl substitué par un ammonium quaternaire, soit comportent en position 3 un radical de type carbamoyloxyméthyl ou triazolopyrimidothiométhyl et, sur le radical méthoxyimino une fonction carboxy libre, estérifiée ou salifiée et un groupement de type 3,4-dihydroxyphényl.

Par ailleurs, la demande européenne EP-A-462009 décrit des composés comportant en position 3 un radical propényl substitué par un ammonium quaternaire et, sur le radical méthoxyimino, une fonction carboxy et un groupement 3,4-dihydroxyphényl.

L'art antérieur ne décrit pas, par contre, de composés comportant à la fois en position 3 un radical propényl substitué par un ammonium quaternaire et sur le radical méthoxyimino, une fonction carboxy, et, notamment, un groupement de type 3,4-dihydroxyphényl substitué sur un ou plusieurs des autres sommets du cycle phényle.

La demanderesse a ainsi découvert et décrit dans la demande principale EP-A-0551034 les produits de formule générale (I) :

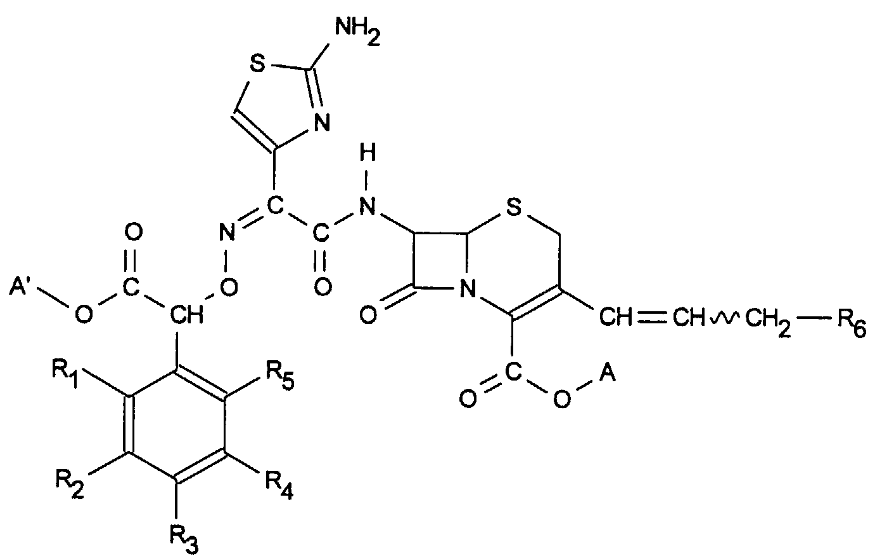

(I)

isomère <u>syn</u>, sous forme (R) ou (S) ou d'un mélange (R,S), sous forme de sel interne ou de sels avec les acides minéraux ou organiques ou de sels d'un métal alcalin, alcalino-terreux, de magnésium, d'ammonium ou d'une base organique aminée, formule dans laquelle :

$R_1$, $R_2$, $R_3$ et $R_5$ identiques ou différents représentent un atome d'hydrogène, un atome d'halogène ou un des radicaux choisis parmi le radical hydroxy, alkyle renfermant de 1 à 4 atomes de carbone, éventuellement substitué par un ou plusieurs atomes d'halogène, alkyloxy renfermant de un à quatre atomes de carbone, mercapto, alkylthio renfermant de 1 à 4 atomes de carbone, nitro, cyano, amino, alkylamino renfermant de 1 à 4 atomes de carbone, dialkylamino renfermant de 2 à 8 atomes de carbone, carbamoyle, (alkylamino) carbonyle renfermant de 2 à 5 atomes de carbone, (dialkylamino) carbonyle renfermant de trois à neuf atomes de carbone, carboxy, alcoxycarbonyle renfermant de 2 à 5 atomes de carbone, acyloxy renfermant de 1 à 8 atomes de carbone,

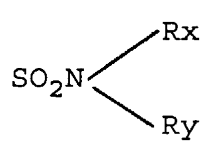

dans lequel Rx et Ry identiques ou différents représentent un atome d'hydrogène ou un radical alkyle renfermant

de 1 à 4 atomes de carbone,

$R_4$ représente un radical hydroxy ou un radical acyloxy renfermant de 1 à 8 atomes de carbone,

étant entendu que : soit $R_3$, soit R5, représente nécessairement le même radical hydroxy ou acyloxy renfermant de 1 à 8 atomes de carbone que R4 et que, lorsque $R_3$ représente un radical hydroxy ou un radical acyloxy renfermant de 1 à 8 atomes de carbone, $R_1$, $R_2$ et $R_5$ ne représentent pas ensemble un atome d'hydrogène,

A et A' sont identiques ou différents et représentent un atome d'hydrogène ou le cation d'un sel selon ci-dessus,

le trait ondulé signifie que le groupement $CH_2R_6$ peut se trouver dans la position E ou Z et $R_6$ représente sous forme d'ammonium quaternaire, un des radicaux suivants :

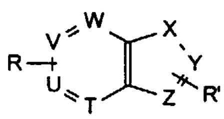
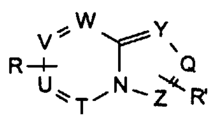
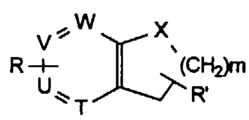
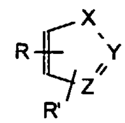

m = 1, 2 ou 3

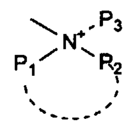

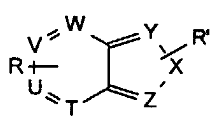
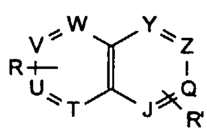
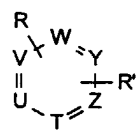
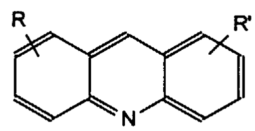

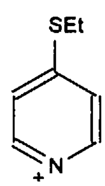
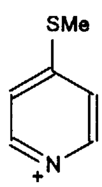
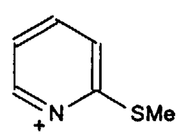
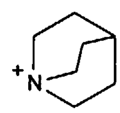
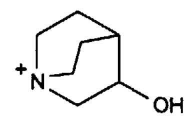

étant entendu que $R_6$ est lié à la chaîne par le ou l'un des atomes d'azote qu'il comporte,dans lesquels X représente $CH_2$, NH, O ou S ;

Q, J, Y, T, U, V, W et Z identiques ou différents représentent indépendamment les uns des autres CH ou N, étant entendu que chacun de ces radicaux cycliques contient de un à cinq hétéroatomes, qu'au moins un de ces hétéroatomes est l'atome d'azote et que ces radicaux cycliques peuvent être substitués par un ou plusieurs radicaux R ou R' ;

R et R', identiques ou différents représentent un atome d'halogène, un radical alkyle renfermant de 1 à 4 atomes de carbone, un radical alkyloxy renfermant de 1 à 4 atomes de carbone, un atome d'halogène, un radical cyano, $CO_2$-$Q_1$, CO-$NQ_1(Q_2)$, $NQ_1(Q_2)$, $SO_2$-$NQ_1(Q_2)$, CS-$NH_2$, NH-CO-$Q_1$, CH=N-OH, CH=N-O-$Q_1$, $CH_2$-CN, $CH_2$-S-$Q_1$ dans lesquels $Q_1$ et $Q_2$ identiques ou différents représentent un atome d'hydrogène ou un radical alkyle renfermant de 1 à 4 atomes de carbone,

$P_1$, $P_2$ et $P_3$ identiques ou différents représentent un radical alkyle renfermant au plus 4 atomes de carbone, éventuellement substitué par un des substituants indiqués ci-dessus pour R et R', le trait pointillé indiquant que $P_1$ et $P_2$ peuvent éventuellement former avec l'atome d'azote auquel ils sont liés, un hétérocycle à 5 ou 6 chaînons choisi parmi les valeurs pyrrolidine, morpholine et pipéridine,ou $P_1$, $P_2$ et $P_3$ forment avec $N^+$ un radical $Me_2N^+CH_2CH_2OH$.

Par radical alkyle renfermant de 1 à 4 atomes de carbone, on entend les radicaux, méthyle, éthyle, propyle, isopropyle, butyle, isobutyle, sec-butyle ou tert-butyle.

Par radical alkyloxy renfermant de 1 à 4 atomes de carbone, on entend les radicaux, méthoxy, éthoxy, propoxy,

iso-propoxy, butoxy, isobutoxy, sec-butoxy ou tert-butoxy.

Par radical alkylthio renfermant de 1 à 4 atomes de carbone, on entend les radicaux, méthylthio, éthylthio, propylthio, isopropylthio, butylthio, isobutylthio, secbutylthio ou tert-butylthio.

Par radical alkylamino renfermant de 1 à 4 atomes de carbone, on entend les radicaux, méthylamino, éthylamino, propylamino, isopropylamino, butylamino, isobutylamino, secbutylamino ou tert-butylamino.

Par radical dialkylamino renfermant de 2 à 8 atomes de carbone, on entend notamment les radicaux, diméthylamino, diéthylamino, dipropylamino, diisopropylamino, dibutylamino, diisobutylamino, éthyl méthylamino, propyl méthylamino, butyl méthylamino, propyl éthylamino.

Par radical (alkylamino) carbonyle renfermant de 2 à 5 atomes de carbone, on entend notamment les radicaux, (méthylamino) carbonyle, (éthylamino) carbonyle, (propylamino) carbonyle, (isopropylamino) carbonyle, (butylamino) carbonyle. Par radical alkoxycarbonyle renfermant de 2 à 5 atomes de carbone, on entend notamment les radicaux, méthoxycarbonyle, éthoxycarbonyle.

Par radical (dialkylamino) carbonyle renfermant de 3 à 9 atomes de carbone, on entend notamment les radicaux, (diméthylamino) carbonyle, (diéthylamino) carbonyle, (dipropylamino) carbonyle.

Par radical acyloxy renfermant de 1 à 8 atomes de carbone, on entend notamment les radicaux acétoxy, propionyloxy ou benzoyloxy.

Par atome d'halogène, on entend atome de fluor, chlore, brome ou iode.

Lorsque $R_4$ représente un radical acyloxy, il s'agit notamment des radicaux acétoxy, propionyloxy ou benzoyloxy. Parmi les valeurs de A et A' on peut citer un équivalent de sodium, de potassium, de lithium, de calcium, de magnésium ou d'ammonium. On peut citer, parmi les bases organiques, la méthylamine, la propylamine, la triméthylamine, la diéthylamine, la triéthylamine, la N,N-diméthyléthanolamine, le tris[(hydroxyméthyl) amino] méthane, l'éthanolamine, la pyridine, la picoline, la dicyclohexylamine, la morpholine, la benzylamine, la procaïne, la lysine, l'arginine, l'histidine, la N-méthylglucamine.

Les produits de formule (I) peuvent également se présenter sous forme de sel interne pur, sous forme salifiée ou sous forme associée aux acides de la solution. Parmi les acides avec lesquels on peut salifier le ou les produits de formule (I), on peut citer entre autres, les acides acétique, trifluoroacétique, maléïque, tartrique, méthanesulfonique, benzènesulfonique, para-toluènesulfonique, phosphorique, sulfurique, chlorhydrique, bromhydrique, iodhydrique.

Dans un mode préféré de l'invention, A' représente un atome d'hydrogène et $CO_2A$ représente $CO_2^-$.

Parmi les produits de formule générale (I) telle que définie ci-dessus, on peut citer en particulier ceux dans laquelle $R_6$ représente un des radicaux suivants :

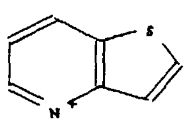
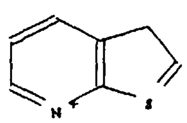
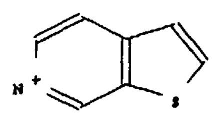
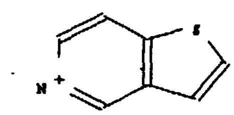

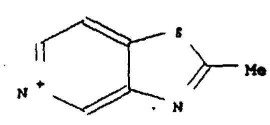
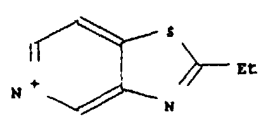
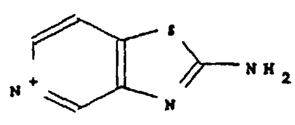
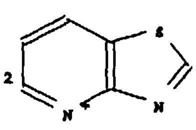

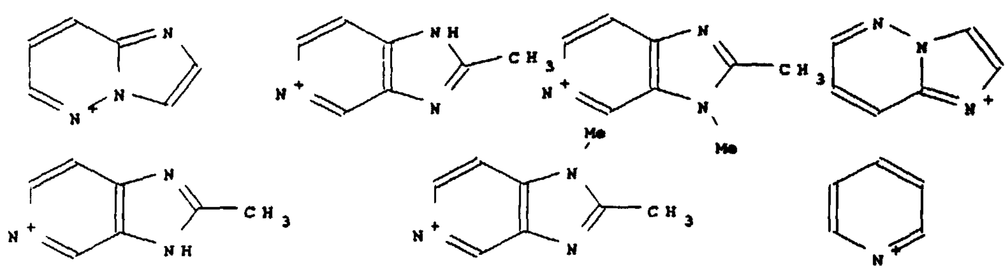

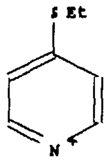
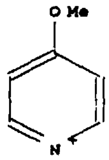
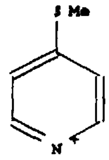
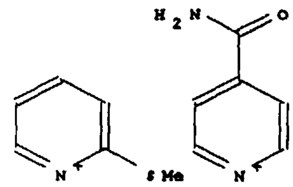
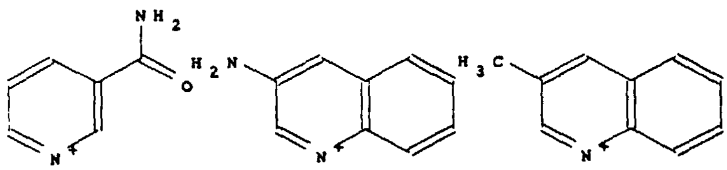

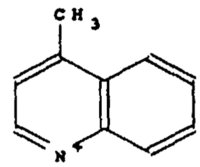
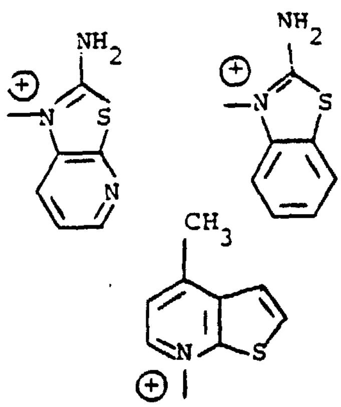

ou encore l'un des radicaux suivants :

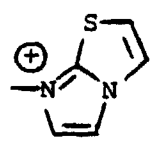
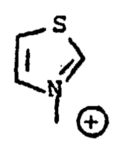
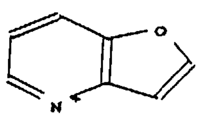

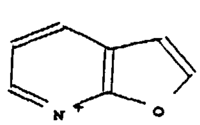
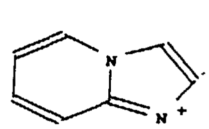
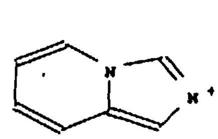
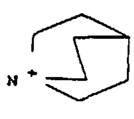

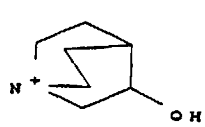
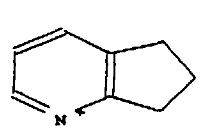
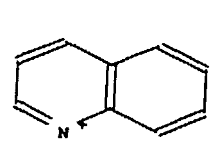
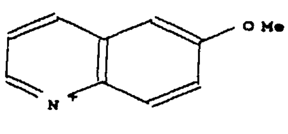

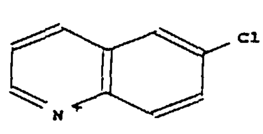
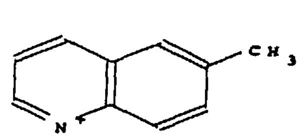

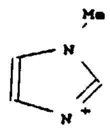
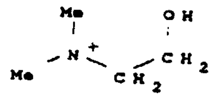
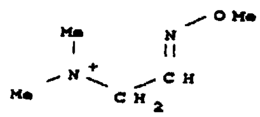
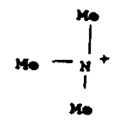

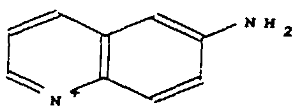
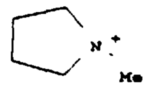
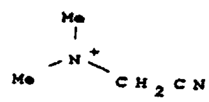
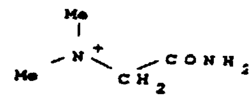

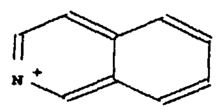

Parmi les produits de formule générale (I) telle que définie ci-dessus, on peut citer plus particulièrement ceux dans laquelle $R_6$ représente le radical quinoléinium, isoquinoléinium, 4-(méthylthio) pyridinium, thièno[2,3-b]pyridinium, imidazo(1,2-a)pyridinium ou le radical 6,7-dihydro-5H-pyrindinium, ceux dans laquelle $R_3$ et $R_4$ représentent chacun un radical hydroxy et ceux dans laquelle $R_2$ et $R_5$ représentent chacun un atome de chlore ou de fluor, ceux dans lesquels $R_1$ et $R_2$ représentent chacun un atome de fluor et ceux dans lesquels $R_2$ représente un radical méthoxy et l'un de $R_1$ ou $R_5$ représente un atome de chlore, et

tout particulièrement, les produits dont les noms suivent :

- le sel interne de (6R-(3-(E) 6alpha, 7béta-(Z))) 1-(3-(7-(((2-amino 4-thiazolyl) ((carboxy (2,3-difluoro 4,5-dihydroxy phényl) méthoxy) imino) acétyl) amino) 2-carboxy 8-oxo 5-thia 1-azabicyclo[4,2,0]oct-2-èn-3-yl) 2-propényl) imidazo(1,2-a) pyridinium,
- le sel interne de (6R-(3-(E) 6alpha, 7béta-(Z))) 1-(3-(7-(((2-amino 4-thiazolyl) ((carboxy (2,5-difluoro 3,4-dihydroxyphényl) méthoxy) imino) acétyl) amino) 2-carboxy 8-oxo 5-thia 1-azabicyclo[4,2,0]oct-2-èn-3-yl) 2-propényl) imidazo(1,2-a) pyridinium,
- le sel interne de (6R-(3-(E) 6alpha, 7béta-(Z))) 1-(3-(7-(((2-amino 4-thiazolyl) ((carboxy (2-chloro 4,5-dihydroxy 3-méthoxy phényl) méthoxy) imino) acétyl) amino) 2-carboxy 8-oxo 5-thia 1-azabicyclo[4,2,0]oct-2-èn-3-yl) 2-propényl) quinolinium,
- le sel interne de (6R-(3-(E) 6alpha, 7béta-(Z))) 1-(3-(7-(((2-amino 4-thiazolyl) ((carboxy (2,5-dichloro 3,4-dihydroxy phényl) méthoxy) imino) acétyl) amino) 2-carboxy 8-oxo 5-thia 1-azabicyclo[4,2,0]oct-2-èn-3-yl) 2-propényl) 6,7-dihydro 5-H-pyrindinium,
- le sel interne de (6R-(3-(E) 6alpha, 7béta-(Z(S*)))) 1-(3-(7-(((2-amino 4-thiazolyl) ((carboxy (3,4-dihydroxy 5-fluorophényl) méthoxy) imino) acétyl) amino) 2-carboxy 8-oxo 5-thia 1-azabicyclo[4,2,0]oct-2-èn-3-yl) 2-propényl) quinoléinium,
- le sel interne de [6R-[3(E), 6alpha, 7béta(Z)]] 1-[3-[7-[[(2-amino 4-thiazolyl) [[carboxy (2-chloro 3,4-dihydroxyphényl) méthoxy] imino] acétyl] amino] 2-carboxy 8-oxo 5-thia 1-azabicyclo[4,2,0]oct-2-en-3-yl] 2-propènyl] quinoléinium (R) ou (S) ou d'un mélange (R+S),
- le sel interne de [6R-[3(E), 6alpha, 7béta(Z)]] 1-[3-[7-[[(2-amino 4-thiazolyl) [[carboxy (3-cyano 4,5-dihydroxy phényl) méthoxy] imino] acétyl] amino] 2-carboxy 8-oxo 5-thia 1-azabicyclo[4,2,0]oct-2-en-3-yl] 2-propènyl] quinoléinium (R) ou (S) ou d'un mélange (R+S),
- le sel interne de [6R-[3(E), 6alpha, 7béta(Z)]] 1-[3-[7-[[(2-amino 4-thiazolyl) [[carboxy (3-fluoro 4,5-dihydroxy phényl) méthoxy] imino] acétyl] amino] 2-carboxy 8-oxo 5-thia 1-azabicyclo[4,2,0]oct-2-en-3-yl] 2-propènyl] quinoléinium (R) ou (S) ou d'un mélange (R+S),
- le sel interne de [[6R-[3(E), 6alpha, 7béta(Z)]] 7-[3-[7-[[(2-amino 4-thiazolyl) [[carboxy (2,5-dichloro 3,4-dihydroxy phényl) méthoxy] imino] acétyl] amino] 2-carboxy 8-oxo 5-thia 1-azabicyclo[4,2,0]oct-2-en-3-yl] 2-propènyl] thièno [2,3-b]pyridinium (R) ou (S) ou d'un mélange (R+S),
- le sel interne de [6R-[3(E), 6alpha, 7béta(Z)]] 1-[3-[7-[[(2-amino 4-thiazolyl) [[carboxy (2,5-dichloro 3,4-dihydroxy

phényl) méthoxy] imino] acétyl] amino] 2-carboxy 8-oxo 5-thia 1-azabicyclo[4,2,0]oct-2-en-3-yl] 2-propènyl] quinoléinium (R) ou (S) ou d'un mélange (R+S),

- le sel interne de [6R-[3(E), 6alpha, 7béta[Z(S*)]]] 1-[3-[7-[[(2-amino 4-thiazolyl) [[carboxy (3-cyano 4,5-dihydroxy phényl) méthoxy] imino] acétyl] amino] 2-carboxy 8-oxo 5-thia 1-azabicyclo[4,2,0]oct-2-en-3-yl] 2-propènyl] thièno [2,3-b]pyridinium,
- le sel interne de [6R-[3(E), 6alpha, 7béta(Z)]] 1-[3-[7-[[(2-amino 4-thiazolyl) [[carboxy (2,5-dichloro 3,4-dihydroxy phényl) méthoxy] imino] acétyl] amino] 2-carboxy 8-oxo 5-thia 1-azabicyclo[4,2,0]oct-2-en-3-yl] 2-propènyl] isoquinoléinium (R) ou (S) ou d'un mélange (R+S),
- le sel interne de [6R-[3(E), 6alpha, 7béta(Z)]] 1-[3-[7-[[(2-amino 4-thiazolyl) [[carboxy (2,5-dichloro 3,4-dihydroxy phényl) méthoxy] imino] acétyl] amino] 2-carboxy 8-oxo 5-thia 1-azabicyclo[4,2,0]oct-2-en-3-yl] 2-propènyl] 4-(méthylthio) pyridinium (R) ou (S) ou d'un mélange (R+S).

Le procédé de préparation des produits de formule (I) telle que définie ci-dessus est caractérisé en ce que l'aldéhyde aromatique de formule (II) :

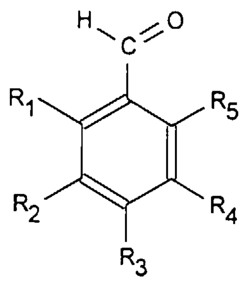

(II)

dans laquelle $R_1$, $R_2$, $R_3$, $R_4$ et $R_5$ sont tels que définis ci-dessus, est si nécessaire protégé en ses fonctions réactives et transformé ainsi en aldéhyde aromatique de formule $(II)_p$ :

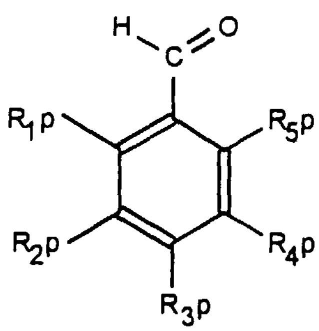

(II)p

dans laquelle $R_{1p}$, $R_{2p}$, $R_{3p}$, $R_{4p}$ et $R_{5p}$ représentent respectivement les valeurs de $R_1$, $R_2$, $R_3$, $R_4$ et $R_5$ telles que définies précédemment ou une fonction réactive protégée, ledit aldéhyde de formule (II)p est homologué en alpha-hydroxy acide de formule (III) :

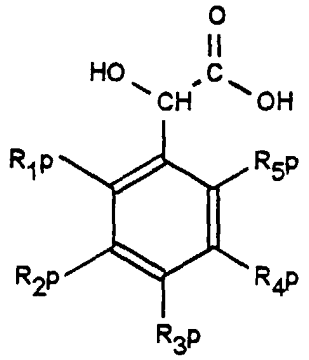

(III)

acide que l'on estérifie en alpha-hydroxy ester de formule (IV) :

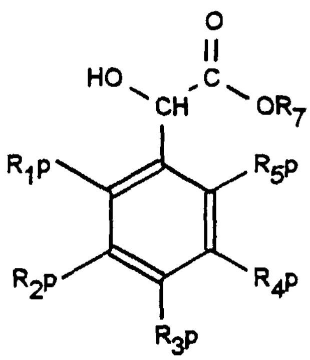

(IV)

dans laquelle $R_7$ représente le reste d'un ester aisément clivable, ledit alpha-hydroxy ester est traité par de la N-hydroxy phtalamide pour conduire au dérivé de formule (V) :

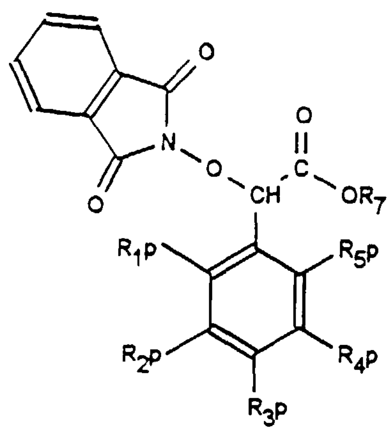

(V)

dérivé de formule (V) que l'on réduit en hydroxylamine O-substitué de formule (VI) :

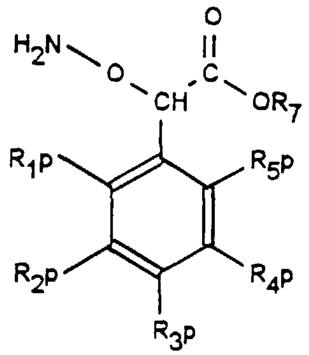

(VI)

produit de formule (VI) que l'on condense avec un dérivé de l'acide (2-amino thiazol-4-yl) glyoxylique de formule (VII) :

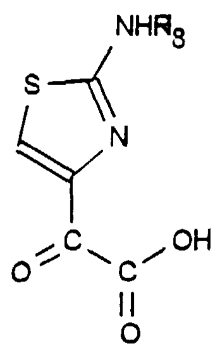

(VII)

dans laquelle $R_8$ représente un atome d'hydrogène ou un groupe protecteur de la fonction amine, pour former le dérivé de l'acide alpha-hydroximino acétique de formule (VIII) :

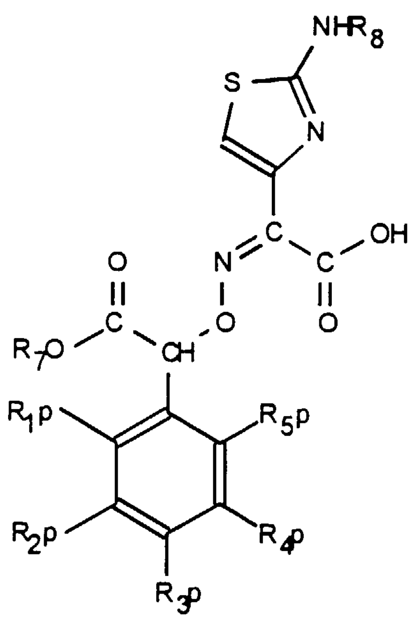

(VIII)

produit de formule (VIII) ou un dérivé fonctionnel de cet acide que l'on amidifie avec un ester du chlorhydrate de l'acide 7-amino 3-(3-chloro 1-propènyl) 8-oxo 5-thia 1-aza-bicyclo[4,2,0]oct-2-èn-2-carboxylique de formule (IX) :

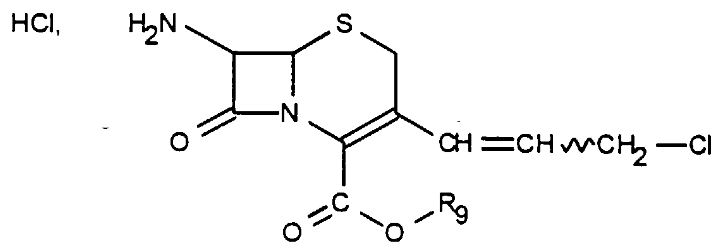

(IX)

ou de ses sels, dans laquelle $R_9$ représente le reste d'un ester aisément clivable, pour conduire au dérivé de l'acide 7-(N-substitué amido) 3-(3-chloro 1-propènyl) 8-oxo 5-thia 1-azabicyclo[4,2,0]oct-2-èn-2-carboxylique de formule (X) :

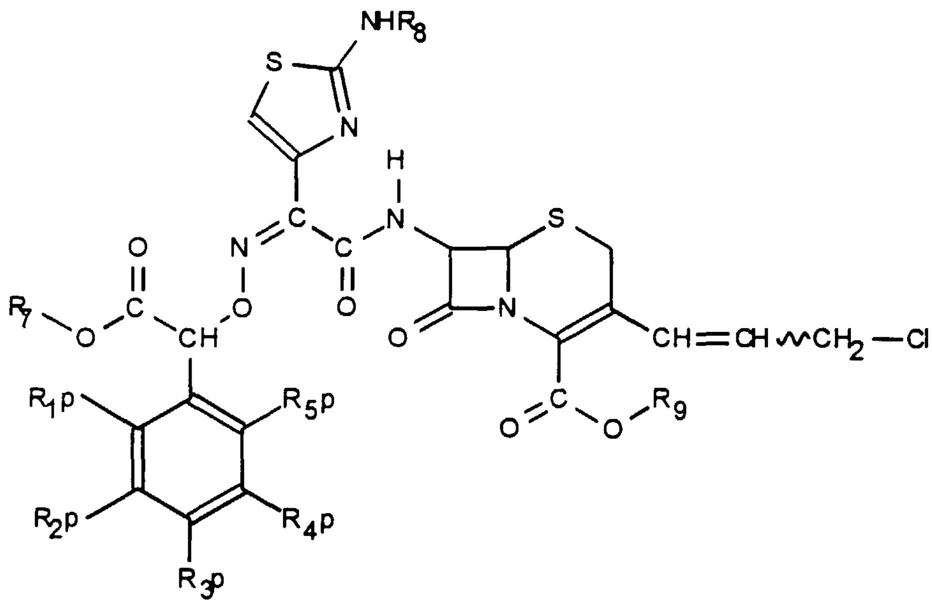

(X)

produit de formule (X) que l'on transforme en analogue 3-(3-iodo propènylé) de formule (XI) :

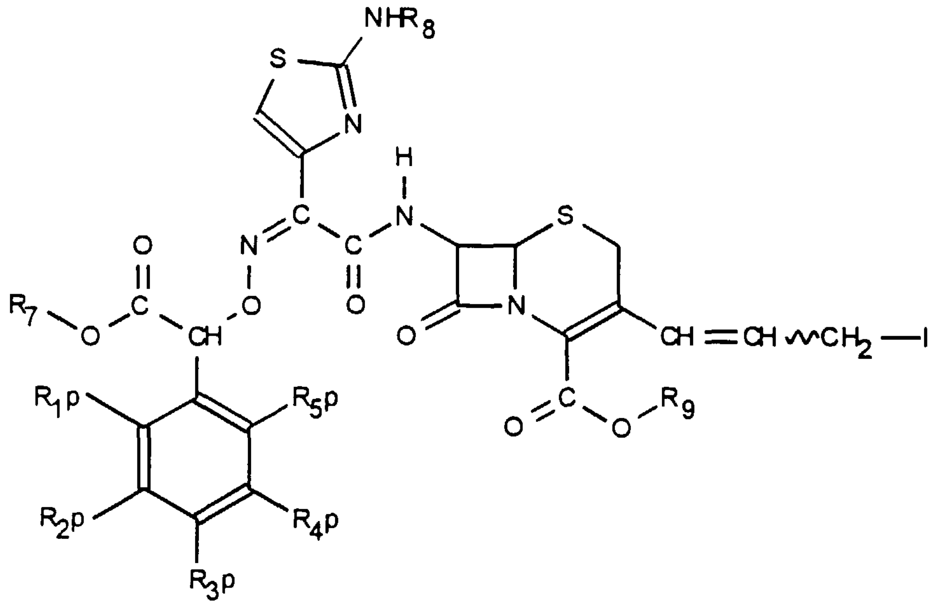

(XI)

produit de formule (XI) que l'on traite avec une base de formule $R_6$ pour obtenir le produit de formule (XII) :

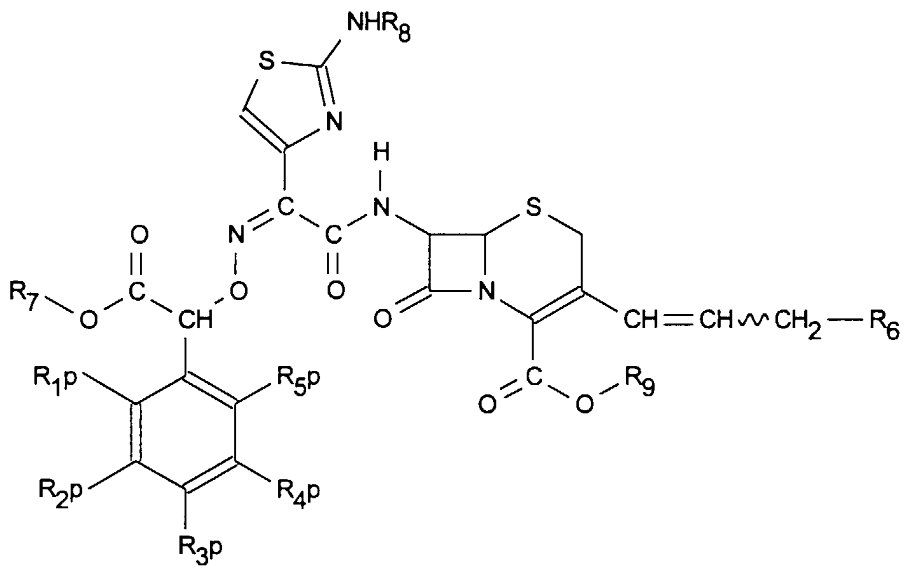

(XII)

produit de formule (XII) à partir duquel si désiré, l'on isole les isomères (E) ou (Z) ou transforme les isomères (Z) en isomère (E) et produit de formule (XII) que l'on soumet à une ou plusieurs des réactions suivantes, dans un ordre quelconque :

a) coupure par hydrolyse ou par action de la thiourée de tout ou partie des groupements esters ou des groupements de protection du radical amino ou des radicaux hydroxyles,
b) estérification ou salification par une base du ou des radicaux carboxyliques,
c) salification par un acide du radical amino,
d) séparation des produits sous forme de mélange R,S en R ou S.

Les produits de formule I peuvent encore être préparés par une variante du procédé décrit ci-dessus, caractérisé en ce que l'hydroxylamine O-substitué de formule (VI) est condensé au produit de formule (XIII) :

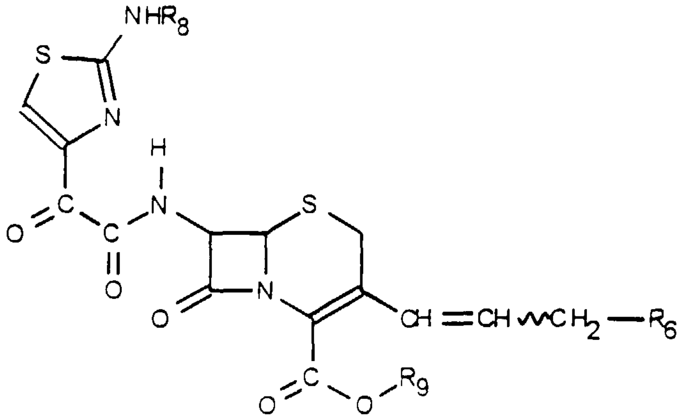

(XIII)

pour conduire au produit de formule (XII) telle que définie précédemment.

Les fonctions hydroxy protégées que peuvent représenter $R_{1p}$, $R_{2p}$, $R_{3p}$, $R_{4p}$ et $R_{5p}$, sont choisies parmi les groupes acyloxy tel que par exemple formyloxy, acétoxy, propionyloxy, chloroacétoxy, bromoacétoxy, dichloroacétoxy, trichloroacétoxy, trifluoroacétoxy, méthoxyacétoxy, phénoxyacétoxy, benzoyloxy, benzoylformoxy, p-nitro benzoyloxy. On peut citer également les groupements éthoxycarbonyloxy, méthoxycarbonyloxy, propoxycarbonyloxy, 2,2,2-trichloro éthoxycarbonyloxy, benzyloxycarbonyloxy, tert-butoxycarbonyloxy, 1-cyclopropyl éthoxycarbonyloxy, phtaloyloxy, butyryloxy, isobutyryloxy, valéryloxy, isovaléryloxy, oxalyloxy, succinyloxy et pivaloyloxy, phénylacétoxy, phénylpropionyloxy, mésyloxy, chlorobenzoyloxy, para-nitrobenzoyloxy, para-tert-butyl benzoyloxy, caprylyloxy, acryloyloxy, méthylcarbamoyloxy, phénylcarbamoyloxy, naphtylcarbamoyloxy.

On peut également citer les radicaux phénoxy, 4-chloro phénoxy, tolyloxy ou tert-butyl phénoxy, tétrahydropyrannyloxy, tétrahydrothiopyrannyloxy, méthoxytétrahydropyrannyloxy, trityloxy, benzyloxy, 4-méthoxy benzyloxy, benzhydryloxy, trichloroéthoxy, 1-méthyl 1-méthoxyéthoxy, ou les radicaux alkoxy alkoxy-méthyle tel que méthoxy éthoxy méthyle.

Deux radicaux hydroxy adjacents peuvent être protégés en formant un radical méthylènedioxy, isopropylènedioxy, 1,1-cyclohexyl bis(oxy), diphénylméthylènedioxy, carbonate ou hydroxy borannylbis(oxy).

Les fonctions hydroxy protégées que peuvent représenter $R_{1p}$, $R_{2p}$, $R_{3p}$, $R_{4p}$ et $R_{5p}$, sont choisies de préférence parmi les groupes méthoxyéthoxyméthoxy, propionyloxyméthoxy, acétoxyméthoxy, butoxy, isobutoxy, tert-butoxy, pentoxy, hexyloxy, butyryloxyméthoxy, valéryloxyméthoxy, pivaloyloxyméthoxy, 2-acétoxy éthoxy, 2-propionyloxy éthoxy, 2-butyryloxy éthoxy, 2-iodoéthoxy, 2,2,2-trichloro éthoxy, vinyloxy, allyloxy, éthynyloxy, propynyloxy, benzyloxy, 4-méthoxy benzyloxy, 4-nitro benzyloxy, phényléthoxy, trityloxy, diphénylméthyloxy ou 3,4-diméthoxyphénoxy.

On préfère particulièrement le groupement 2-méthoxy éthoxyméthoxy (MEM-O).

Les restes de groupements ester facilement clivables que représentent $R_7$ et $R_9$, sont choisis parmi les groupements butyle, isobutyle, tert-butyle, pentyle, hexyle, méthoxyméthyle, éthoxyméthyle, isopropyloxyméthyle, alphaméthoxy éthyle, alpha-éthoxy éthyle, méthylthiométhyle, éthylthiométhyle, isopropylthiométhyle, pivaloyloxyméthyle, acétoxyméthyle, propionyloxyméthyle, butyryloxyméthyle, isobutyryloxyméthyle, valéryloxyméthyle, isovaléryloxyméthyle, tert-butylcarbonyloxyméthyle, hexadécanoyloxyméthyle, pivaloyloxyméthyl, propionyloxyéthyle, isovaléryloxyéthyle, 1-acétoxy éthyle, 2-acétoxy éthyle, 1-propionyloxy éthyle, 2-propionyloxy éthyle, 1-butyryloxy éthyle, 2-butyryloxy éthyle, 1-(tert-butylcarbonyloxy) éthyle, 1-acétoxy propyle, 1-hexadécanoyloxy éthyle, 1-propionyloxy propyle, 1-méthoxycarbonyloxy éthyle, méthoxycarbonyloxyméthyle, 1-acétoxy butyle, 1-acétoxy hexyle, 1-acétoxy heptyle, phtalidyle, 5,6-diméthoxy phtalidyle, tert-butylcarbonylméthyle, vinyle, allyle, 2-chloro allyle, éthynyle, propynyle, méthoxycarbonylméthyle, benzyle, 4-méthoxy benzyle, 4-nitro benzyle, phénéthyle, trityle, diphényl méthyle, phényle, 4-chloro phényle, tolyle, tert-butyl phényle, 3,4-diméthoxy phényle, méthoxyéthoxyméthyle, diméthylaminoéthyle, cyanométhyle, tert-butoxycarbonylméthyle, 2,2-éthylènedioxy éthyle, cyanoéthyle, 2,2-diméthoxy éthyle ; 2-chloro éthoxyméthyle, (2-hydroxy éthoxy) éthyle, 2,3-époxy propyle, 3-diméthylamino 2-hydroxy propyle, 2-hydroxy éthyle, 2-méthylaminoéthoxyméthyle, (2-amino éthoxy) méthyle, 3-méthoxy 2,4-thiadiazol-5-yle, tétrahydropyrann-2-yle, 1-méthoxy 1-méthyl éthyle, 2-hydroxy 1-méthyl éthyle, iso-propyle, carbamoylméthyle, chlorométhyle, 2-chloro éthyle, 2,2,2-trichloro éthyle, 2-iodo éthyle, acétyle, méthyle, 2-méthylthio éthyle, thiocyanatométhyle, 2-chloro 1-acétoxy éthyle, 2-bromo 1-acétoxy éthyle, 2-fluoro 1-acétoxy éthyle, 2-méthoxy 1-acétoxy éthyle, 2-méthyl 1-acétoxy propyle, 1-méthyl 1-acétoxy éthyle, 1-(méthoxyacétoxy) éthyle, 1-acétyl carbonyloxyéthyle, 1-hydroxy acétoxyéthyle, 1-(2-thiényl) carbonyloxyéthyle, 1-(2-furyl) carbonyloxyéthyle, 1-(5-nitro 2-furyl) carbonyloxyéthyle, 1-(2-pyrrolyl) carbonyloxyéthyle, 1-(propionyloxycarbonyloxy) éthyle, 1-(propoxycarbonyloxy) éthyle, 1-(isopropoxycarbonyloxy) éthyle,-1-(méthoxyéthoxycarbonyloxy) éthyle, 1-(allyloxycarbonyloxy) éthyle, isopropoxycarbonyl méthyle, 1-[(2,3-époxy propyl)

oxycarbonyloxy] éthyle, 1-[(2-furyl) méthoxycarbonyloxy] éthyle, 1-[(2-fluoro éthoxy) carbonyloxy] éthyle, 1-(méthoxycarbonyloxy) propyle, 1-(méthoxycarbonyloxy) 1-méthyl éthyle, (méthoxycarbonyloxy) chlorométhyle, 1-(méthoxycarbonyloxy) 2-chloro éthyle, 1-(méthoxy carbonyloxy) 2-méthoxy éthyle, 1-(méthoxycarbonyloxy) allyle ou un reste 5-méthyl 2-oxo 1,3-dioxol-4-yle ;

On préfère le radical diphénylméthyle pour $R_7$ et 4-méthoxy benzyle ou diphénylméthyle pour $R_9$.

Le groupement protecteur du radical amino que peut représenter $R_8$ peut être par exemple un groupe carbamoyle méthyl carbamoyle, phénylcarbamoyle, naphtylcarbamoyle, ainsi que les thiocarbamoyles correspondants, un radical alkyle de 1 à 6 atomes de carbone substitué ou non substitué tel que, préférentiellement, trichloroéthyle, tert-butyle ou tert-amyle, un radical aralkyle tel que benzyle, 4-méthoxy benzyle, phénéthyle, trityle, 3,4-diméthoxy benzyle ou benzhydryle, un radical acyle aliphatique, aromatique ou hétérocyclique substitué ou non, tel que par exemple formyle, acétyle, propionyle, butyryle, isobutyryle, valéryle, iso-valéryle, chloroacétyle, dichloroacétyle, trichloroacétyle, bromoacétyle, trifluoroacétyle benzoyle, toluolyle, naphtoyle, chlorobenzoyle, para-nitro benzoyle, para-tert-butyl benzoyle, phénoxyacétyle, caprylyle, décanoyle, acryloyle, phtaloyle, mésyle, phénylacétyle, phénylpropionyle, oxalyle, succinyle, pivaloyle, un radical alcoxycarbonyle ou cycloalcoxycarbonyle inférieur tel que par exemple, méthoxycarbonyle, éthoxycarbonyle, propoxycarbonyle, 1-cyclopropyléthoxycarbonyle, isopropoxycarbonyle, butoxycarbonyle, tert-butoxycarbonyle, pentoxycarbonyle, hexyloxycarbonyle, trichloroéthoxy carbonyle, un groupe aralcoxycarbonyle, tel que benzyloxycarbonyle.

On préfère le groupement trityle.

La liste ci-dessus n'est pas limitative, il est évident que d'autres groupements protecteurs des amines, groupements connus en particulier dans la chimie des peptides, peuvent également être utilisés.

Le dérivé fonctionnel de l'acide de formule (VIII) peut être par exemple un halogénure, un anhydride symétrique ou mixte, l'amide, l'azide ou un ester activé.

Comme exemple d'anhydride mixte on peut citer par exemple celui formé avec le chloroformiate d'isobutyle et celui formé avec le chlorure de pivaloyle et les anhydrides mixtes carboxylique-sulfonique formé par exemple avec le chlorure de para-toluène sulfonyle.

Comme exemple d'ester activé, on peut mentionner l'ester formé avec le 2,4-dinitrophénol et celui formé avec l'hydroxybenzothiazole.

Comme exemple d'halogénure, on peut citer le chlorure ou le bromure.

L'anhydride peut être formé in situ par action de carbodiimide N,N'-disubstitué, par exemple le N,N-dicyclohexylcarbodiimide.

La réaction d'acylation est conduite de préférence dans un solvant organique tel que le chlorure de méthylène. On peut cependant utiliser d'autres solvants tels que le tétrahydrofuranne, le chloroforme ou le diméthylformamide.

Lorsqu'on utilise un halogénure d'acide et de manière générale lorsqu'une molécule d'acide halohydrique est libérée au cours de la réaction, on réalise la réaction de préférence en présence d'une base telle que la soude, la potasse, les carbonates et carbonates acides de sodium ou de potassium, l'acétate de sodium, la triéthylamine, la pyridine, la morpholine ou la N-méthylmorpholine.

La température de réaction est en général inférieure ou égale à la température ambiante.

On peut également faire agir directement un produit de formule (VIII) avec un produit de formule (IX) en présence d'un carbodiimide telle que le diisopropylcarbodiimide ou le 1-(3-diméthylamino propyl) 3-éthyl carbodiimide (EDC). Un exemple d'une telle préparation est donnée plus loin dans la partie expérimentale.

L'action des réactifs capable d'introduire le radical $R_6$ sur le produit de formule (XI) est effectuée dans les conditions suivantes :

On peut effectuer in situ ou après isolement du produit de formule X, une substitution de l'atome de chlore par un atome d'iode en présence d'iodure de sodium, puis ajoute ensuite le réactif de formule $R_6$, en présence ou non d'un solvant organique tel que le dichlorométhane, l'acétonitrile ou le tétrahydrofuranne.

On peut également faire agir directement le réactif de formule $R_6$ désiré sur le produit de formule (X), en présence de tétrafluoroborate d'argent.

L'isomérie des produits de formule (XII) peut être différente de celle des produits de formule (X) ou (XI) utilisés au départ. Dans le cas où l'on isole l'isomère Z, on peut transformer cet isomère en isomère E selon les méthodes usuelles, notamment par action de l'iode.

Selon les valeurs de $R_7$, $R_8$, $R_9$, $R_{1p}$, $R_{2p}$, $R_{3p}$, $R_{4p}$ et $R_{5p}$, l'action sur le produit de formule (XII) d'un ou plusieurs agents d'hydrolyse, d'hydrogénolyse ou de la thiourée a pour but d'éliminer le radical $R_8$ lorsque celui-ci représente un radical protecteur du radical amino, de transformer les radicaux $R_{1p}$, $R_{2p}$, $R_{3p}$, $R_{4p}$ et $R_{5p}$ respectivement en radicaux $R_1$, $R_2$, $R_3$, $R_4$ et $R_5$ lorsque ceux-ci représentent un groupement protecteur des radicaux hydroxyle et/ou d'éliminer les radicaux $R_7$ et $R_9$ lorsque ceux-ci représentent, parmi les groupements esters facilement clivables l'un de ceux que l'on désire éliminer.

Cependant, il est bien entendu possible d'éliminer $R_8$ et de transformer les radicaux $R_{1p}$, $R_{2p}$, $R_{3p}$, $R_{4p}$ et $R_{5p}$ respectivement en radicaux $R_1$, $R_2$, $R_3$, $R_4$ et $R_5$ lorsque ceux-ci représentent un groupement protecteur des radicaux

hydroxyle sans toucher aux substituants $R_7$ et $R_9$ lorsque ceux-ci doivent être conservés. La nature des réactifs à mettre en jeu dans un tel cas est bien connu de l'homme du métier. Des exemples de telles réactions sont donnés plus loin dans la partie expérimentale. On trouvera par exemple une description des différentes méthodes d'élimination des différents groupements protecteurs dans la demande de brevet français B.F. 2.499.995.

Etant donné la nature des groupements protecteurs préférés que l'on utilise : trityle pour $R_8$, 2-méthoxy éthoxy méthyle pour protéger les fonctions hydroxy que peuvent représenter un ou plusieurs des substituants $R_1$, $R_2$, $R_3$, $R_4$ et $R_5$, diphénylméthyle pour $R_7$ et 4-méthoxy benzyle ou diphénylméthyle pour $R_9$, on utilise de préférence l'acide trifluoroacétique sans solvant ou dans un solvant tel que l'anisole ou un mélange de solvants tels que anisole/chlorure de méthylène. On obtient alors un sel avec l'acide trifluoroacétique. On peut revenir à la base libre par action d'une base telle que le carbonate de triéthylamine.

La salification des produits peut être effectuée selon les méthodes usuelles ; elle peut par exemple, être obtenue par action, sur un produit sous forme acide ou sur un solvat, par exemple, le solvat éthanolique ou un hydrate de cet acide, d'une base minérale telle que l'hydroxyde de sodium ou de potassium, le carbonate ou le carbonate acide de sodium ou de potassium. On peut également utiliser les sels d'acides minéraux tels que le phosphate trisodique. On peut également faire appel à des sels d'acides organiques tels que par exemple, les sels de sodium d'acides carboxyliques aliphatiques, linéaires ou ramifiés, saturés ou insaturés de 1 à 18 et de préférence de 2 à 10 atomes de carbone. Les chaînes aliphatiques de ces acides peuvent être interrompues par un ou plusieurs hétéroatomes tels que l'oxygène ou le soufre ou substituées par des radicaux aryle tels que phényle, thiényle ou furyle, par un ou plusieurs radicaux hydroxyles ou par un ou plusieurs atomes d'halogène tels que fluor, chlore ou brome, préférentiellement chlore, par un ou plusieurs radicaux carboxyliques ou alkoxycarbonyles inférieurs, de préférence méthoxycarbonyle, éthoxycarbonyle ou propyloxycarbonyle, par un ou plusieurs radicaux aryloxy, de préférence phénoxy.

De plus, on peut utiliser comme acides organiques, des acides aromatiques suffisamment solubles comme, par exemple, des acides benzoïques substitués, de préférence par des radicaux alkyles inférieurs.

Comme exemples de tels acides organiques, on peut mentionner les acides formique, acétique, acrylique, butyrique, adipique, isobutyrique, n-caproïque, isocaproïque, chloropropioniques, crotonique, phényl acétique, (2-thiényl) acétique, (3-thiényl) acétique, (4-éthyl phényl) acétique, glutarique, l'ester monoéthylique de l'acide adipique, les acides hexanoïque, heptanoïque, décanoïque, oléïque, stéarique, palmitique, 3-hydroxy propionique, 3-méthoxy propionique, 3-méthyl thiobutyrique, 4-chloro butyrique, 4-phényl butyrique, 3-phénoxy butyrique, 4-éthyl benzoïque, 1-propyl benzoïque.

On utilise cependant de préférence comme sels de sodium, l'acétate de sodium, le 2-éthyl hexanoate de sodium ou le diéthyl acétate de sodium.

La salification peut également être obtenue par action d'une base organique comme la triéthylamine, la diéthylamine, la triméthylamine, la propylamine, la N,N-diméthyl éthanolamine, le tris[(hydroxyméthyl) amino] méthane, la méthylamine, l'éthanolamine, la pyridine, la picoline, la dicyclohexyl amine, la morpholine et la benzylamine.

Elle peut également être obtenue par action de l'arginine, de la lysine, de la procaïne, de l'histidine, de la N-méthyl glucamine.

Cette salification est réalisée de préférence dans un solvant ou un mélange de solvants tels que l'eau, l'éther éthylique, le méthanol, l'éthanol ou l'acétone.

Les sels sont obtenus sous forme amorphe ou cristallisée selon les conditions réactionnelles employées.

Les sels cristallisés sont préparés de préférence en faisant réagir les acides libres avec l'un des sels des acides carboxyliques aliphatiques mentionnés ci-dessus, de préférence, avec l'acétate de sodium.

La salification des produits par les acides minéraux ou organiques est effectuée dans les conditions usuelles.

L'estérification éventuelle des produits est effectuée dans les conditions classiques. On opère en général en faisant réagir l'acide de formule (I) ou un dérivé fonctionnel avec un dérivé de formule :

$$Z\text{-}Re$$

dans laquelle Z représente un radical hydroxyle ou un atome d'halogène tel que le chlore, le brome, l'iode et Re désigne le groupement ester à introduire, groupement dont une liste non exhaustive figure ci-dessus. Dans certains cas, il peut être avantageux d'effectuer une estérification sur un produit dont l'amine et/ou les groupements réactionnels présents sur l'oxyimino sont bloqués avant d'enlever le groupement protecteur de l'amine et du groupement réactionnel présents sur l'oxyimino.

Les produits de formule (I) comportent plusieurs carbones asymétriques. Dans le noyau cephème, qui comporte deux carbones asymétriques, les deux carbones sont dans la configuration R. Par ailleurs le radical présent sur la fonction oxyimino comporte également un carbone asymétrique qui peut être sous forme R ou S ou sous forme d'un mélange R + S. La séparation des deux diastéréoisomères peut être effectuée par les moyens connus de l'homme du métier, par exemple par chromatographie.

Les produits de formule générale (I) possèdent une très bonne activité antibiotique sur les bactéries gram (+) telles que les staphylocoques, les streptocoques et notamment sur les staphylocoques pénicillino-résistants. Leur efficacité sur les bactéries gram (-) notamment sur les bactéries coliformes, les klebsiella, les salmonella, les proteus et les pseudomonas, est particulièrement remarquable.

Ces propriétés rendent aptes lesdits produits ainsi que leurs sels d'acides pharmaceutiquement acceptables à être utilisés comme médicaments dans le traitement des affections à germes sensibles et notamment dans celui des staphylococcies, telles que septicémies à staphylocoques, staphylococcies malignes de la face ou cutanée, pyodermites, plaies septiques ou suppurantes, anthrax, phlegmons, érésipèles, staphylococcies aiguës primitives ou post grippales, broncho-pneumonies, suppurations pulmonaires.

Ces produits peuvent également être utilisés comme médicaments dans le traitement des colibacilloses et infections associées, dans les infections à proteus, à klebsiella et à salmonella et dans d'autres affections provoquées par des bactéries à gram (-).

Parmi les médicaments de formule (I) telle que décrite ci-dessus on peut citer plus particulièrement ceux dans laquelle $R_6$ est choisi parmi les radicaux quinoléinium, isoquinoléinium, 4-(méthylthio) pyridinium, thièno[2,3-b] pyridinium, imidazo (1,2-a) pyridinium et 6,7-dihydro 5H-pyrindinium, et tout spécialement les produits dont les noms suivent :

- le sel interne de (6R-(3-(E) 6alpha, 7béta-(Z))) 1-(3-(7-(((2-amino 4-thiazolyl) ((carboxy (2,3-difluoro 4,5-dihydroxy phényl) méthoxy) imino) acétyl) amino) 2-carboxy 8-oxo 5-thia 1-azabicyclo[4,2,0]oct-2-èn-3-yl) 2-propényl) imidazo(1,2-a) pyridinium,
- le sel interne de (6R-(3-(E) 6alpha, 7béta-(Z))) 1-(3-(7-(((2-amino 4-thiazolyl) ((carboxy (2,5-difluoro 3,4-dihydroxyphényl) méthoxy) imino) acétyl) amino) 2-carboxy 8-oxo 5-thia 1-azabicyclo[4,2,0]oct-2-èn-3-yl) 2-propényl) imidazo(1,2-a) pyridinium,
- le sel interne de (6R-(3-(E) 6alpha, 7béta-(Z))) 1-(3-(7-(((2-amino 4-thiazolyl) ((carboxy (2-chloro 4,5-dihydroxy 3-méthoxy phényl) méthoxy) imino) acétyl) amino) 2-carboxy 8-oxo 5-thia l-azabicyclo[4,2,0]oct-2-èn-3-yl) 2-propényl) quinolinium,
- le sel interne de (6R-(3-(E) 6alpha, 7béta-(Z))) 1-(3-(7-(((2-amino 4-thiazolyl) ((carboxy (2,5-dichloro 3,4-dihydroxy phényl) méthoxy) imino) acétyl) amino) 2-carboxy 8-oxo 5-thia 1-azabicyclo[4,2,0]oct-2-èn-3-yl) 2-propényl) 6,7-dihydro 5-H-pyrindinium,
- le sel interne de (6R-(3-(E) 6alpha, 7béta-(Z(S*)))) 1-(3-(7-(((2-amino 4-thiazolyl) ((carboxy (3,4-dihydroxy 5-fluorophényl) méthoxy) imino) acétyl) amino) 2-carboxy 8-oxo 5-thia 1-azabicyclo[4,2,0]oct-2-èn-3-yl) 2-propényl) quinoléinium,
- le sel interne de [6R-[3(E), 6alpha, 7béta(Z)]] 1-[3-[7-[[(2-amino 4-thiazolyl) [[carboxy (2-chloro 3,4-dihydroxyphényl) méthoxy] imino] acétyl] amino] 2-carboxy 8-oxo 5-thia 1-azabicyclo[4,2,0]oct-2-en-3-yl] 2-propènyl] quinoléinium (R) ou (S) ou d'un mélange (R+S),
- le sel interne de [6R-[3(E), 6alpha, 7béta(Z)]] 1-[3-[7-[[(2-amino 4-thiazolyl) [[carboxy (3-cyano 4,5-dihydroxy phényl) méthoxy] imino] acétyl] amino] 2-carboxy 8-oxo 5-thia 1-azabicyclo[4,2,0]oct-2-en-3-yl] 2-propènyl] quinoléinium (R) ou (S) ou d'un mélange (R+S),
- le sel interne de [6R-[3(E), 6alpha, 7béta(Z)]] 1-[3-[7-[[(2-amino 4-thiazolyl) [[carboxy (3-fluoro 4,5-dihydroxy phényl) méthoxy] imino] acétyl] amino] 2-carboxy 8-oxo 5-thia 1-azabicyclo[4,2,0]oct-2-en-3-yl] 2-propènyl] quinoléinium (R) ou (S) ou d'un mélange (R+S),
- le sel interne de [[6R-[3(E), 6alpha, 7béta(Z)]] 7-[3-[7-[[(2-amino 4-thiazolyl) [[carboxy (2,5-dichloro 3,4-dihydroxy phényl) méthoxy] imino] acétyl] amino] 2-carboxy 8-oxo 5-thia 1-azabicyclo[4,2,0]oct-2-en-3-yl] 2-propènyl] thièno [2,3-b] pyridinium (R) ou (S) ou d'un mélange (R+S),
- le sel interne de [6R-[3(E), 6alpha, 7béta(Z)]] 1-[3-[7-[[(2-amino 4-thiazolyl) [[carboxy (2,5-dichloro 3,4-dihydroxy phényl) méthoxy] imino] acétyl] amino] 2-carboxy 8-oxo 5-thia 1-azabicyclo[4,2,0]oct-2-en-3-yl] 2-propènyl] quinoléinium (R) ou (S) ou d'un mélange (R+S),
- le sel interne de [6R-[3(E), 6alpha, 7béta[Z(S*)]]] 1-[3-[7-[[(2-amino 4-thiazolyl) [[carboxy (3-cyano 4,5-dihydroxy phényl) méthoxy] imino] acétyl] amino] 2-carboxy 8-oxo 5-thia 1-azabicyclo[4,2,0]oct-2-en-3-yl] 2-propènyl] thièno [2,3-b]pyridinium,
- le sel interne de [6R-[3(E), 6alpha, 7béta(Z)]] 1-[3-[7-[[(2-amino 4-thiazolyl) [[carboxy (2,5-dichloro 3,4-dihydroxy phényl) méthoxy] imino] acétyl] amino] 2-carboxy 8-oxo 5-thia 1-azabicyclo[4,2,0]oct-2-en-3-yl] 2-propènyl] isoquinoléinium (R) ou (S) ou d'un mélange (R+S),
- le sel interne de [6R-[3(E), 6alpha, 7béta(Z)]] 1-[3-[7-[[(2-amino 4-thiazolyl) [[carboxy (2,5-dichloro 3,4-dihydroxy phényl) méthoxy] imino] acétyl] amino] 2-carboxy 8-oxo 5-thia 1-azabicyclo[4,2,0]oct-2-en-3-yl] 2-propènyl] 4-(méthylthio) pyridinium (R) ou (S) ou d'un mélange (R+S),

ainsi que leurs sels pharmaceutiquement acceptables.

Les compositions pharmaceutiques renfermant, comme principe actif, au moins un des médicaments définis

ci-dessus,peuvent être administrées par voie buccale, rectale, parentérale, notamment intramusculaire ou par voie locale en application topique sur la peau et les muqueuses.

Les produits de formule (I) et notamment ceux dans laquelle A représente un ester clivable peuvent être administrés par voie orale.

Les compositions ci-dessus peuvent être solides ou liquides et se présenter sous les formes pharmaceutiques couramment utilisées en médecine humaine comme par exemple, les comprimés simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent notamment se présenter sous forme d'une poudre destinée à être dissoute extemporanément dans un véhicule approprié, par exemple, de l'eau stérile apyrogène.

La dose administrée est variable selon l'affection traitée, le sujet en cause, la voie d'administration et le produit considéré. Elle peut être, par exemple, comprise entre 0,250 g et 4 g par jour, par voie orale chez l'homme, avec le produit décrit à l'exemple 1 ou encore comprise entre 0,500 g et 1 g trois fois par jour par voie intramusculaire.

Les produits de formule (I) peuvent également être utilisés comme désinfectants des instruments chirurgicaux.

La présente demande divisionnaire a pour objet, à titre de produits industriels nouveaux et notamment à titre de produits intermédiaires nécessaires à la préparation des produits de formule (I),les produits de formules (III), (IV), (V), (VI) et (VIII), telles que définies précédemment, formules dans lesquelles $R_{1p}$, $R_{2p}$, $R_{3p}$, $R_{4p}$ et $R_{5p}$ représentent respectivement les valeurs de $R_1$, $R_2$, $R_3$, $R_4$ et R5 telles que définies ci-après ou une fonction réactive protégée, $R_1$, $R_2$, $R_3$ et $R_5$ identiques ou différents, représentent un atome d'hydrogène, un atome d'halogène ou un des radicaux choisis parmi le radical hydroxy, alkyle renfermant de 1 à 4 atomes de carbone, éventuellement substitué par un ou plusieurs atomes d'halogène, alkyloxy renfermant de un à quatre atomes de carbone, mercapto, alkylthio renfermant de 1 à 4 atomes de carbone, nitro, cyano, amino, alkylamino renfermant de 1 à 4 atomes de carbone, dialkylamino renfermant de 2 à 8 atomes de carbone, carbamoyle, (alkylamino) carbonyle renfermant de 2 à 5 atomes de carbone, (dialkylamino) carbonyle renfermant de trois à neuf atomes de carbone, carboxy, alcoxycarbonyle renfermant de 2 à 5 atomes de carbone, acyloxy renfermant de 1 à 8 atomes de carbone et

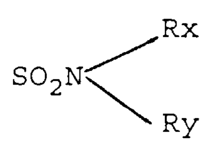

dans lequel Rx et Ry,identiques ou différents, représentent un atome d'hydrogène ou un radical alkyle renfermant de 1 à 4 atomes de carbone,

$R_4$ représente un radical hydroxy ou un radical acyloxy renfermant de 1 à 8 atomes de carbone, $R_3$ ou $R_5$ représentant nécessairement le même radical Hydroxy ou Acyloxy que $R_4$, étant entendu que :

- soit $R_1$,$R_2$ ou $R_3$, soit $R_2$, $R_4$ ou $R_5$ représente nécessairement un atome de Fluor, de Brome, d'Iode ou un radical cyano,
- soit $R_1$ et $R_5$ sont tous les deux différents d'hydrogène,
- soit $R_2$ et $R_5$ représentent tous les deux un atome de chlore,

R7 représente le reste d'un ester aisément clivable et $R_8$ représente un atome d'hydrogène ou un groupe protecteur de la fonction amine.

Les produits de formule (VI) dans lesquels $R_1$p et $R_2$p représentent chacun un atome de fluor et $R_3$p et $R_4$p représentent chacun un radical hydroxy protégé par exemple par une fonction méthoxyéthoxyméthyle (MEM ci-après produits de formule (VIa)) peuvent en particulier être préparés comme suit:

On fait agir du butyllithium et du triméthylborate puis de l'eau oxygénée sur un produit de formule (A) :

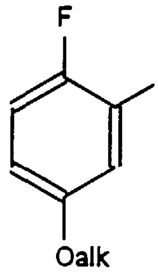

(A)

dans laquelle Alk représente un radical alkyle, de préférence le méthyle pour obtenir le produit de formule (B) :

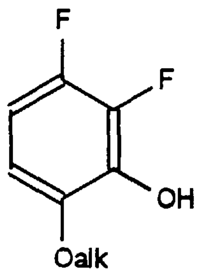

(B)

sur lequel on fait agir de la diméthylamine en présence d'anhydride formique pour obtenir un produit de formule (C) :

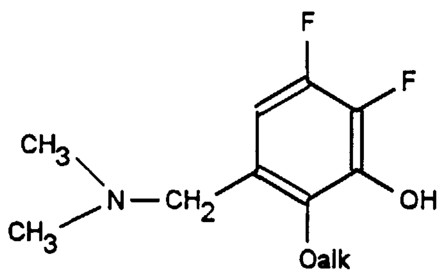

(C)

sur lequel on fait agir de l'iodure de méthyle et de l'hexaméthylène tétramine en présence d'acide acétique pour obtenir un produit de formule ($II_D$) :

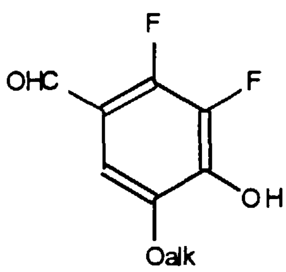

($II_D$)

produit de formule ($II_D$) dont on élimine le radical alkyle en présence de tribromure de bore pour obtenir un produit de formule (E) :

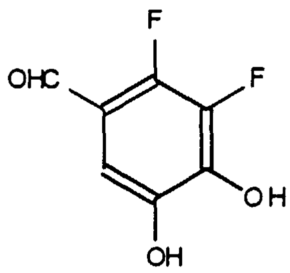

(E)

produit de formule (E) dont on protège les fonctions hydroxyles, par exemple à l'aide d'un groupement (MEM) pour obtenir un produit de formule (F) :

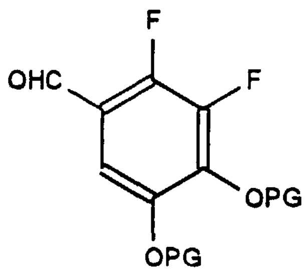

(F)

dans laquelle Gp représente un groupement protecteur du radical hydroxyle, produit que l'on traite avec un dérivé organophosphoré de formule :

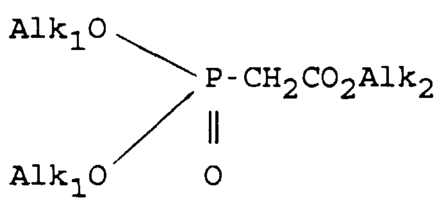

dans laquelle $Alk_1$ et $Alk_2$ identiques ou différents représentent un radical alkyle ayant de 1 à 4 atomes de carbone pour obtenir un produit de formule (G) :

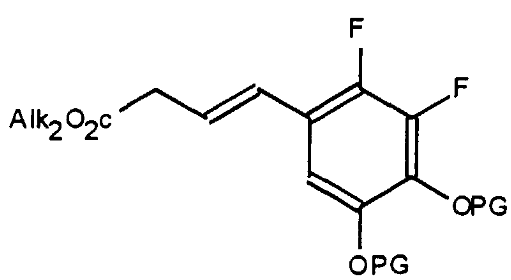

(G)

que l'on traite par un agent réducteur, par exemple l'hydrure de diisobutylaluminium pour obtenir un produit de formule (H) :

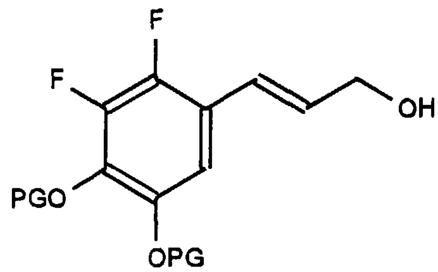

(H)

produit que l'on traite par un agent d'époxydation par exemple l'acide métachloroperbenzoïque pour obtenir un produit de formule (J) :

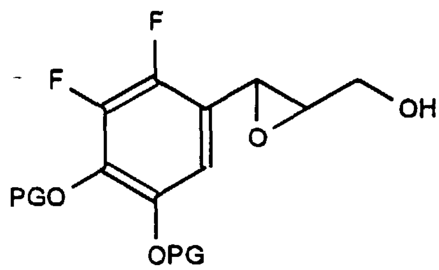

(J)

produit que l'on traite par le chlorure cuivreux en présence de chlorure de lithium pour obtenir un produit de formule (K) :

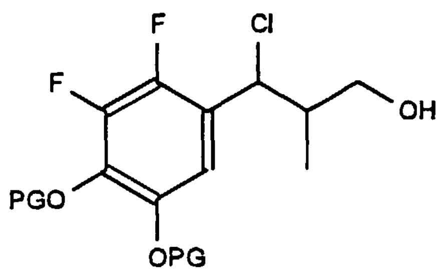

(K)

produit que l'on traite par un réactif d'oxydation, par exemple le métapériodate de sodium puis par un réactif d'estérification pour obtenir un produit de formule (L) :

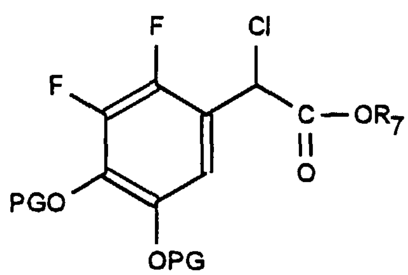

(L)

dans lequel $R_7$ a la signification précédente, produit que l'on traite par la N-hydroxyphtalimide puis par l'hydrazine pour obtenir un produit de formule ($VI_a$) :

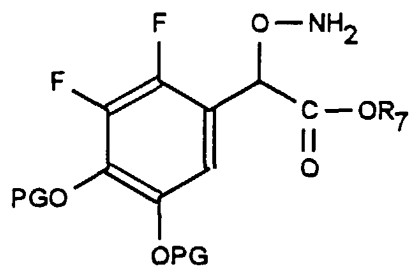

(VIa)

Les produits de formules (II) et (A) sont connus de manière générale ; la plupart sont commercialisés ; d'autres peuvent être préparés à partir de produits commercialisés, selon les méthodes décrites dans les préparations ci-dessous. Pour la préparation des produits de formule (II), on peut aussi utiliser les méthodes décrites dans la littérature, notamment la réduction dite de Rosemund, la réduction des acides benzoïques, ou la formylation des cycles aromatiques comme par exemple la réaction de Vilsmeier-Haack, la réaction de Gatterman-Koch, la réaction de Reimer-Tiemann ou la réaction avec le fluorure de formyle (J. Am. Chem. Soc. 82, 2380 (1960).

Les produits de formules (VII) et (IX) sont également connus dans la littérature, notamment dans les demandes de brevets belge BE864828 et européenne EP0333154.

Les exemples suivants illustrent l'invention sous toutefois la limiter.

**PREPARATION 1 : 2-chloro 3,4-bis[(2-méthoxy éthoxy) méthoxy] benzaldéhyde**

**Stade A : 2-chloro 3,4-dihydroxy benzaldéhyde**

On dissout 27,62 g de 3,4-dihydroxy benzaldéhyde (commercial) dans 450 $cm^3$ d'acide acétique et l'on fait barboter du chlore à température ordinaire jusqu'à consommation de 4,48 $dm^3$ de gaz. L'agitation est maintenue pendant 16 heures, puis la solution est concentrée, refroidie à 0°C, et le précipité filtré, lavé et séché. On recueille ainsi 7,5 g du produit attendu que l'on recristallise dans l'acétate d'éthyle. Point de fusion (PF) = 196°C.

**Stade B : 2-chloro 3,4-bis[(2-méthoxy éthoxy) méthoxy] benzaldéhyde**

On ajoute à une suspension contenant 11,42 g de produit préparé au stade précédent dans 120 $cm^3$ de chlorure de méthylène, 46 $cm^3$ de diisopropyl éthylamine, refroidit à-10°C puis ajoute 30,14 $cm^3$ de chloro (2-méthoxy éthoxy) méthane, agite pendant une heure, dilue par 100 $cm^3$ d'eau, sépare puis lave la phase organique, sèche et concentre. On recupère ainsi 22,6 g du produit attendu. Rf = 0,6 (éluant acétate d'éthyle)
Analyse RMN du proton ($CDCl_3$ 250 MHz en ppm)
3,36 (s), et 3,38 (s) : $-OC\underline{H}_3$
3,58 (m) (4 H), 3,84 (m) (2 H) et 4,06 (m) (2 H) : $O-C\underline{H}_2-C\underline{H}_2-O$
5,29 (s) (2 H) et 5,38 (s) (2 H) : $-O-C\underline{H}_2-O$
7,21 (d) et 7,71 (d) : protons aromatiques (Ar-H) 10,36 (s) : $-C\underline{H}=O$

**PREPARATION 2 : 3-formyl 5,6-bis[(2-méthoxy éthoxy) méthoxy] benzonitrile**

**Stade A : 3-formyl 6-hydroxy 5-méthoxy benzonitrile**

On porte au reflux sous agitation pendant deux heures un mélange constitué de 23,1 g de 3-bromo 4-hydroxy 5-méthoxy benzaldéhyde, 9,3 g de cyanure cuivreux et 140 $cm^3$ de diméthyl acétamide (DMA).
Après refroidissement on verse sur la glace, extrait par le mélange acétate d'éthyle-méthanol (90-10), lave la phase organique, sèche et élimine les solvants. On recueille 18 g de produit brut utilisé tel quel au stade suivant. F = 180°C

**Stade B : 3-formyl 5,6-dihydroxy benzonitrile**

On mélange sous azote 18 g du produit préparé au stade précédent et 400 $cm^3$ de dichlorométhane, refroidit à 0°C, ajoute 150 $cm^3$ d'une solution molaire de tribromure de bore dans le dichlorométhane, laisse 16 heures à température ambiante, concentre, refroidit de nouveau à 0°C et ajoute 250 $cm^3$ d'une solution normale d'acide chlorhydrique. On essore le produit qui cristallise, lave à l'eau, sèche puis recristallise dans le mélange isopropanol-eau (1-2). On recueille ainsi 12 g du produit attendu.

Analyse Infra-rouge (Nujol)
Absorption forte et complexe dans la région -NH/OH
2245 $cm^{-1}$ : C≡N
1700 $cm^{-1}$ : C=O
1602, 1597, 1520 $cm^{-1}$ noyau aromatique
Analyse RMN du proton (DMSO 250 MHz en ppm)
7,45 (d) (J = 2) : Ar-H
7,73 (d) (J = 2) : Ar-H
9,74 : C<u>H</u>=O
11,15 : absorption mobile

**Stade C : 3-formyl 5,6-bis[(2-méthoxy éthoxy) méthoxy] benzonitrile**

On ajoute à une suspension contenant 12,6 g de produit préparé au stade précédent dans 500 $cm^3$ de dichlorométhane, 53,6 $cm^3$ de diisopropyl éthylamine, refroidit à -10°C puis ajoute 35,3 $cm^3$ de chloro (2-méthoxy éthoxy) méthane, agite pendant une heure, dilue par 100 $cm^3$ d'eau, sépare puis lave la phase organique, sèche et concentre. On recupère ainsi
21,4 g du produit attendu.
Analyse Infra-rouge ($CHCl_3$)
Absence d'OH
2235 $cm^{-1}$ : C≡N
1702 et 2730 $cm^{-1}$ : CH=O
1592, 1582 et 1485 $cm^{-1}$ : noyau aromatique
Analyse RMN du proton ($CDCl_3$ 250 MHz en ppm)
3,37 (s) : -OC$\underline{H}_3$
3,58 (m) (4 H), 3,85 (m) (2 H) et 4,06 (m) (2 H) : O-C$\underline{H}_2$-C$\underline{H}_2$-O
5,39 (s) (2 H) et 5,49 (s) (2 H) : -O-C$\underline{H}_2$-O
7,76 (d) (1 H) et 7,92 (d) (1 H) : Ar-H
9,90 (s) : -C<u>H</u>=O

**<u>PREPARATION 3</u> : 3-fluoro 4,5-bis[(2-méthoxy éthoxy) méthoxy] benzaldéhyde**

**Stade A : 3-fluoro 4-hydroxy 5-méthoxy benzaldéhyde**

On agite pendant environ deux heures et demie, à 0°C et sous azote, un mélange constitué de 30,4 g 4-hydroxy 5-méthoxy benzaldéhyde (vanilline), 100 $cm^3$ d'acétonitrile et 250 $cm^3$ de Fréon, on fait barboter pendant 2 heures et demie de l'azote mélangée à 10% de fluor, puis de l'azote seul. Après traite-ment au thiosulfate, acidification par une solution d'acide chlorhydrique dinormale et extraction à l'acétate d'éthyle, on lave la phase organique, sèche, concentre et chromatographie sur silice en éluant par le dichlorométhane ; on recueille
2,1 g du produit recherché. Rf= 0,3

| Microanalyse | FM= $C_7H_7FO_3$ | | |
|---|---|---|---|
| calculés<br>trouvés | C% 56,47<br>56,4 | H% 4,14<br>4,1 | F% 11,16<br>11,0 |

**Stade B : 3-fluoro 4,5-dihydroxy benzaldéhyde**

On mélange sous azote 3,72 g du produit préparé au stade précédent et 60 $cm^3$ de dichlorométhane, refroidit à 0°C, ajoute 32 $cm^3$ d'une solution molaire de tribromure de bore dans le dichlorométhane, laisse 16 heures à température ambiante, concentre, refroidit de nouveau à 0°C et acidifie. On essore le produit qui cristallise, lave à l'eau, sèche. On recueille ainsi 2,83 g du produit brut attendu que l'on utilise tel quel au stade suivant. Rf= 0,2 [éluant : acétone-dichlorométhane (1-9)]

**Stade C : 3-fluoro 4,5-bis[(2-méthoxy éthoxy) méthoxy] benzaldéhyde**

On ajoute à une suspension contenant 2,8 g du produit préparé au stade précédent dans 5,6 $cm^3$ de dichlorométhane, 17 $cm^3$ de diisopropyl éthylamine, refroidit à -10°C puis ajoute 6,5 $cm^3$ de chloro (2-méthoxy éthoxy)

méthane, agite pendant une heure, dilue par 100 $cm^3$ d'eau, sépare puis lave la phase organique, sèche et concentre. On recupère ainsi 5,5 g du produit attendu. Rf = 0,55 [éluant : dichlorométhane-acétone (9-1)]
Analyse Infra-rouge ($CHCl_3$)
Absence d'OH
1696 et 2730 $cm^{-1}$ : CH=O conjugué
Analyse RMN du proton ($CDCl_3$ 200 MHz en ppm)
3,36 (s) et 3,37 (s) : -OC<u>H</u>$_3$
3,60 (m) (4 H), 3,85 (m) (2 H) et 3,96 (m) (2 H) : O-C<u>H</u>$_2$-C<u>H</u>$_2$-O 5,33 (s) et 5,36 (s) : O-C<u>H</u>$_2$-O
7,32 (dd) 7,51 (m) : Ar-H
9,90 (m) : -CH=O

**<u>PREPARATION 4</u> : 3-chloro 4,5-bis[(2-méthoxy éthoxy) méthoxy] benzaldéhyde**

**Stade A : 3-chloro 4,5-dihydroxy benzaldéhyde**

On mélange sous azote 37,2 g de 5-chloro vanilline commerciale et 800 $cm^3$ de dichlorométhane, refroidit à 0°C, ajoute 300 $cm^3$ d'une solution molaire de tribromure de bore dans le dichlorométhane, laisse 16 heures à température ambiante, concentre, refroidit de nouveau à 0°C et acidifie. On essore le produit qui cristallise, lave à l'eau, sèche et recristallise dans le mélange isopropanol-eau (1-2 en v.). On recueille ainsi 26,6 g du produit attendu. Point de fusion (PF) > 260°C
Analyse Infra-rouge (Nujol)
3425 $cm^{-1}$ : -OH + absorption générale
1672-1660 $cm^{-1}$ : C=O
1595, 1588, 1534, 1500 $cm^{-1}$ : cycle aromatique
Analyse RMN du proton (DMSO 250 MHz en ppm)
7,24 (d J=2Hz) et 7,44 (d J=2Hz) : Ar-<u>H</u>
9,90 (m) : -C<u>H</u>=O
10,40 : absorption mobile

**Stade B : 3-chloro 4,5-bis[(2-méthoxy éthoxy) méthoxy] benzaldéhyde**

On ajoute à une suspension contenant 15 g du produit préparé au stade précédent dans 150 $cm^3$ de dichlorométhane, 60,5 $cm^3$ de diisopropyl éthylamine, refroidit à -10°C puis ajoute en 45 minutes, 39,7 $cm^3$ de chloro (2-méthoxy éthoxy) méthane, agite pendant une demi-heure, dilue par 100 $cm^3$ d'eau, sépare puis lave la phase organique, sèche, concentre et chromatographie sur silice en éluant par le mélange dichlorométhane-méthanol (99-1). On recupère ainsi 15,9 g du produit attendu.
Analyse Infra-rouge ($CHCl_3$)
1698 et 2735 $cm^{-1}$ : CH=O conjugué
1591, 1579, 1498 $cm^{-1}$ : cycle aromatique
Analyse RMN du proton ($CDCl_3$ 250 MHz en ppm)
3,38 (s) : -OC<u>H</u>$_3$
3,57 (m) (4 H), 3,87 (m) (2 H) et 4,03 (m) (2 H) : O-C<u>H</u>$_2$-C<u>H</u>$_2$-O 5,36 (s) et 5,38 (s) : O-C<u>H</u>$_2$-O
7,59 (d) 7,62 (d) : Ar-<u>H</u>
9,85 : -C<u>H</u>=O

**<u>PREPARATION 5</u> : 3-nitro 4,5-bis[(2-méthoxy éthoxy) méthoxy] benzaldéhyde**

**Stade A : 3-nitro 4,5-dihydroxy benzaldéhyde**

On mélange sous azote 35 g de 5-nitro vanilline (commerciale) et 1200 $cm^3$ de dichlorométhane, refroidit à 0°C, ajoute 533 $cm^3$ d'une solution molaire de tribromure de bore dans le dichlorométhane, laisse deux jours à température ambiante, concentre, reprend le résidu à froid (bain de glace + méthanol) par 300 $cm^3$ d'acide chlorhydrique dinormale, agite deux à trois heures à cette température puis laisse 16 heures à température ambiante. On extrait à l'acétate d'éthyle, lave la phase organique, sèche et concentre. On recueille ainsi 18 g du produit attendu. (PF) > 260°C
Analyse Infra-rouge (Nujol)
absorption générale NH/OH
1682 $cm^{-1}$ : C=O

1620, 1590, 1580, 1548 et 1525 $cm^{-1}$ : cycle aromatique + $-NO_2$

**Stade B : 3-nitro 4,5-bis[(2-méthoxy éthoxy) méthoxy] benzaldéhyde**

On ajoute à une suspension contenant 18 g du produit préparé au stade précédent dans 50 $cm^3$ de dichlorométhane, 13,6 $cm^3$ de diisopropyl éthylamine, refroidit puis ajoute en 45 minutes, 39,6 $cm^3$ de chloro (2-méthoxy éthoxy) méthane, agite pendant deux heures à 0°C, dilue par 100 $cm^3$ d'eau, sépare puis lave la phase organique, sèche, concentre et chromatographie sur silice en éluant par le mélange dichlorométhane-acétone (9-1). On recupère ainsi 22,1 g du produit attendu. Rf = 0,3
Analyse Infra-rouge ($CHCl_3$)
1704 et $cm^{-1}$ : CH=O
1608, 1578, 1546, 1496 $cm^{-1}$ : cycle aromatique + $NO_2$
Analyse RMN du proton ($CDCl_3$ 300 MHz en ppm)
3,36 et 3,38 : $-OC\underline{H}_3$
3,55 (m), 3,87 (m) : $O-C\underline{H}_2-C\underline{H}_2-O$
5,41 : $O-C\underline{H}_2-O$
7,92 : Ar-$\underline{H}$
9,93 : -C$\underline{H}$=O

**<u>PREPARATION 6</u> : 3-iodo 4,5-bis[(2-méthoxy éthoxy) méthoxy] benzaldéhyde**

**Stade A : 3-iodo 4,5-dihydroxy benzaldéhyde**

On mélange sous azote 37,2 g de 5-iodo vanilline (commerciale) et 360 $cm^3$ de dichlorométhane, refroidit à 0°C, ajoute 135 $cm^3$ d'une solution molaire de tribromure de bore dans le dichlorométhane, laisse 16 heures à température ambiante, concentre, refroidit de nouveau à 0°C et acidifie. On essore le produit qui cristallise, lave à l'eau, sèche et recristallise dans le mélange isopropanol-eau (1-2 en v.). On recueille ainsi 23,4 g du produit attendu.
Analyse Infra-rouge (Nujol)
absorption générale NH/OH
1662 (m) 1640 (F) $cm^{-1}$ : C=O
1588, 1578, 1516 $cm^{-1}$ : cycle aromatique
Analyse RMN du proton (DMSO 250 MHz en ppm)
7,25 (sl) et 7,44 (sl) : Ar-$\underline{H}$
9,68 (m) : -C$\underline{H}$=O
10,46, 10,43 $cm^{-1}$ **:** absorption mobile

**Stade B : 3-iodo 4,5-bis[(2-méthoxy éthoxy) méthoxy] benzaldéhyde**

On ajoute à une suspension contenant 25,4 g du produit préparé au stade précédent dans 640 $cm^3$ de dichlorométhane, 67 $cm^3$ de diisopropyl éthylamine, refroidit à -10°C puis ajoute en 45 minutes, 44 $cm^3$ de chloro (2-méthoxy éthoxy) méthane, agite pendant une demi-heure, dilue par 100 $cm^3$ d'eau, sépare puis lave la phase organique, sèche, concentre et chromatographie sur silice en éluant par le mélange acétate d'éthyle-hexane (1/1). On recupère ainsi 25,5 g du produit attendu.
Analyse Infra-rouge ($CHCl_3$)
Absence d'OH
1698 et 2730 $cm^{-1}$ : CH=O
1588, 1562, $cm^{-1}$ : cycle aromatique
Analyse RMN du proton ($CDCl_3$ 250 MHz en ppm)
3,38 (sl) (6 H) : $-OC\underline{H}_3$
3,58 (m) (4 H), 3,84 (m) (2 H) et 4,05 (m) (2 H) : $O-C\underline{H}_2-C\underline{H}_2-O$
5,33 (sl) et 5,39 (sl) : $O-C\underline{H}_2-O$
7,68 (sl) et 7,96 (sl) : Ar-$\underline{H}$
9,82 : -C$\underline{H}$=O

**<u>PREPARATION 7</u> : 3,4,5-tris[(2-méthoxy éthoxy) méthoxy] benzaldéhyde**

On ajoute à une suspension contenant 8 g de 3,4,5-trihydroxy benzaldéhyde (commercial) dans 160 $cm^3$ de dichlorométhane, 52 $cm^3$ de diisopropyl éthylamine, refroidit à 0-5°C puis ajoute en une heure et demie, 35 $cm^3$ de

chloro (2-méthoxy éthoxy) méthane, agite, dilue par 100 $cm^3$ d'eau, sépare puis lave la phase organique, sèche, et concentre. On recupère ainsi 21,75 g de produit brut que l'on utilise tel quel par la suite.
Analyse Infra-rouge ($CHCl_3$)
Absence d'OH
1696 $cm^{-1}$ : CH=O
1590, 1498 $cm^{-1}$ : cycle aromatique
Analyse RMN du proton ($CDCl_3$ 250 MHz en ppm)
3,36 et 3,38 (9 H) : $-OC\underline{H}_3$
3,57 (m) (6 H), 3,85 (m) (4 H) et 3,99 (m) (2 H) : $O-C\underline{H}_2-C\underline{H}_2-O$ 5,31 (s) (2 H) et 5,34 (s) (4 H) : $O-C\underline{H}_2-O$
7,43 (s) : Ar-H
9,82 : -CH=O

**<u>PREPARATION 8</u> : 2,5-dichloro 3,4-bis[(2-méthoxy éthoxy) méthoxy] benzaldéhyde**

**Stade A : 2,5-dichloro 3,4-dihydroxy benzaldéhyde**

On dissout 27,6 g de 3,4-dihydroxy benzaldéhyde (commercial) dans 300 $cm^3$ d'acide acétique et l'on fait barboter du chlore à température ordinaire jusqu'à consommation de 31,24 g du gaz. L'agitation est maintenue pendant 42 heures, puis la solution est concentrée, refroidie à 0°C, et le précipité filtré, lavé et séché. On recueille ainsi 13,7 g du produit attendu. Point de fusion (PF) 176-178°C ; Rf =0,1 [éluant : acétate d'éthyle-cyclohexane (5-5)]
Analyse RMN du proton (DMSO 250 MHz en ppm)
7,24 (d J=2Hz) et 7,44 (d J=2Hz) : Ar-<u>H</u>
9,90 (m) : -C<u>H</u>=O
10,40 : absorption mobile

**Stade B : 2,5-dichloro 3,4-bis[12-méthoxy éthoxy) méthoxy] benzaldéhyde**

On ajoute à une solution contenant 6,83 g du produit préparé au stade précédent dans 265 $cm^3$ d'acétonitrile, 60,5 $cm^3$ de diisopropyl éthylamine, puis, 11,27 $cm^3$ de chloro (2-méthoxy éthoxy) méthane, agite pendant deux heures à 35°C, évapore à sec reprend au dichlorométhane, lave, sèche et évapore le solvant et chromatographie sur silice en éluant par le mélange acétate d'éthyle-cyclohexane (6-4). On recupère ainsi 10,9 g du produit attendu. PF < 50°C ; Rf = 0,3
Analyse Infra-rouge (PE580)
1690 $cm^{-1}$ : CH=O
1572, 1551 $cm^{-1}$ : cycle aromatique
Analyse RMN du proton ($CDCl_3$ 250 MHz en ppm)
3,37 et 3,38 : $-OC\underline{H}_3$
3,58 et 4,01 (m) (2 H) : $O-C\underline{H}_2-C\underline{H}_2-O$
5,29 et 5,36 (s) : $O-C\underline{H}_2-O$
7,78 (s) : Ar-<u>H</u>
10,36 (s) : -C<u>H</u>=O

**<u>PREPARATION 9</u> : 3-méthoxy 4,5-bis[(2-méthoxy éthoxy) méthoxy] benzaldéhyde**

On ajoute à une suspension contenant 8 g de 3,4-dihydroxy 5-méthoxy benzaldéhyde (commercial) dans 80 $cm^3$ de dichlorométhane, 34 $cm^3$ de diisopropyl éthylamine, refroidit à -5°C, -10°C puis ajoute en une demi-heure, 23 $cm^3$ de chloro (2-méthoxy éthoxy) méthane, agite pendant 16 heures, lave la phase organique, sèche, filtre, concentre et chromatographie sur silice en éluant par le mélange hexane-acétate d'éthyle (50-50). On recupère le produit attendu. Rf =0,4 [éluant : hexane-acétate d'éthyle (30-70)].
Analyse Infra-rouge ($CHCl_3$ sur PE 580)
Absence d'OH
1696 $cm^{-1}$ : CH=O
1588, 1498 $cm^{-1}$ : cycle aromatique
Analyse RMN du proton ($CDCl_3$ 250 MHz en ppm)
3,36 (s) (3 H) et 3,38 (s) (3 H) : $O-CH_2-CH_2-OC\underline{H}_3$
3,57 (m) (4 H), 3,87 (m) (2 H) et 4,00 (m) (2 H) : $O-C\underline{H}_2-C\underline{H}_2-O$
3,90 (s) (3 H) : $Ar-OC\underline{H}_3$
5,31 (s) (2 H) et 5,35 (s) (2 H) : $O-C\underline{H}_2-O$

7,19 (d J=2Hz) (1 H) et 7,38 (d J=2Hz) (1 H) : Ar-H en méta 9,80 (s) (1 H) : -CH=O

**PREPARATION 10 : Iodure de 1-[3-[7béta-[[oxo [2-[(triphénylméthyl) amino] thiazol-4-yl] acétyl] amino] 2-[(4-méthoxy benzyloxy) carbonyl] 8-oxo 5-thia 1-azabicyclo[4,2,0] [oct-2-en-3-yl] 2-propènyl] thièno-[2,3-b]pyridinium**

**Stade A : 7béta-[[oxo [2-[(triphénylméthyl) amino] thiazol-4-yl] acétyl] amino] 3-[(Z+E)-3-chloro 1-propényl) 8-oxo 5-thia 1-azabicyclo[4,2,0]oct-2-èn-2-carboxylate de 4-méthoxy** benzyle.

On agite sous azote, 5 g de chlorhydrate de 7béta-amino 3-[(Z)-3-chloro 1-propényl] 8-oxo 5-thia 1-azabicyclo-[4,2,0]oct-2-èn-2-carboxylate de 4-méthoxy benzyle (décrit dans la demande de brevet européen N° 0333 154), 0,72 g d'acide oxo [2-[(triphénylméthyl) amino] thiazol-4-yl] acétique (décrite dans la demande de brevet belge N° 864828) dans 200 $cm^3$ de dichlorométhane, refroidit à 0°C puis on ajoute 2,315 g de 1-(3-diméthylamino propyl) 3-éthyl carbodiimide (EDC), agite pendant 40 minutes, lave et sèche, évapore les solvants et chromatographie en éluant par le mélange dichlorométhane-éther isopropylique (9-1). On recueille 5,51 g de produit attendu. Rf = 0,3.

**Stade B : 7béta-[[oxo [2-[(triphénylméthyl) amino] thiazol-4-yl] acétyl] amino] 3-[(Z+E)-3-iodo 1-propényl] 8-oxo 5-thia 1-azabicyclo[4,2,0]oct-2-èn-2-carboxylate de 4-méthoxy** benzyle.

On dissout 5,5 g du produit préparé au stade précédent dans 110 $cm^3$ d'acétone, ajoute 3,094 g de iodure de sodium, agite pendant une heure et demi à 20°C, évapore le solvant, reprend le résidu dans 350 $cm^3$ de dichlorométhane, lave, sèche, filtre et amène à sec. La mousse obtenue est filtrée et chromatographiée en éluant par le mélange dichlorométhane-éther isopropylique (90-10) ; on recueille 3,93 g du dérivé iodé attendu. Rf = 0,3
Analyse Infra-rouge ($CHCl_3$)
1786, 1721,1701 et 1663 $cm^{-1}$ : C=O et béta-lactame
1613, 1586, 1535, 1516 $cm^{-1}$ : aromatique + système conjugué
Analyse RMN du proton ($CDCl_3$ 300 MHz en ppm)
3,57 : -S-$CH_2$-C(C=CH-)=C-
3,82 (s) : Ar-$OCH_3$
3,98 (d, J=8) : -CH=CH-$CH_2$-I
5,03 (d J=5) ppm : CO-NH-CH(C=O)-CH-(N-)-S-
5,25 (AB, J=12) : CO-O-$CH_2$-Ar
5,75 : CO-NH-CH(C=O)-CH(N-)-S-
6,14 (dt, J=16 et 8) ppm : -CH=CH-$CH_2$-I isomérie E
6,8 à 7,4 (m) : -S-CH=C(C=N-)-N=C(NH-trityl)- et Ar-H du trityle
8,19 (d) : CO-CO-NH-CH (C=O)-CH (N-)-S-

**Stade C : Iodure de 1-[3-[7béta-[[oxo [2-[(triphénylméthyl) amino] thiazol-4-yl] acétyl] amino] 2-[(4-méthoxy benzyloxy) carbonyl] 8-oxo 5-thia 1-azabicyclo[4,2,0] [oct-2-en-3-yl] 2-propènyl] thièno-[2,3-b]pyridinium**

On agite pendant une heure et demie à 20°C, 3,93 g du dérivé iodé obtenu au stade précédent mélangé à 6 g de thiéno[2,3-b]pyridine, précipite dans 400 $cm^3$ d'éther filtre et sèche sous vide ; on obtient ainsi 4,12 g du produit recherché. Rf = 0,1 [éluant : dichlorométhane-méthanol (92-8)]

| Microanalyse FM = $C_{49}H_{40}IN_5O_6S_3$ | | | | | |
|---|---|---|---|---|---|
| calculés | C% 57,81 | H% 3,96 | I% 12,46 | N% 6,87 | S% 9,44 |
| trouvés | 57,3 | 3,9 | 11,7 | 6,7 | 9,6 |

Analyse RMN du proton ($CDCl_3$, 300 MHz en ppm)
5,02 (d) : CO-NH-CH(C=O)-CH-(N-)-S-
5,27 (s) : -CO-O-$CH_2$-Ar
5,6 (dd) : -CH=CH-$CH_2$-$N^+$,
5,74 (dt) : CO-NH-CH(C=O)-CH(N-)-S-
6,56 (d) : -CH=CH-$CH_2$-$N^+$ isomérie E
7,3 : Ar-H du trityle
8,3 : -S-CH=C(C=N-)-N=C($NH_2$)-
7,89 (d) : H en position 2 du thièno[2,3-b]pyridinium

7,71 (d) : H en position 3 du thièno[2,3-b]pyridinium
8,8 (d) : H en position 4 du thièno[2,3-b]pyridinium
8,10 (m) : H en position 5 du thièno[2,3-b]pyridinium

**PREPARATION 11 : Acide [4-fluoro (2,3-bis hydroxy) phényl] hydroxy acétique**

On dissout à 20°C 51,2 g de 3-fluorocatéchol et 36,8 g d'acide glyoxylique dans 160 $cm^3$ d'eau, à cette solution refroidie à 0°C on ajoute, en 30 minutes, 34,8 g de soude en solution dans 400 $cm^3$ d'eau. On chauffe 4 heures à 46°C, puis on refroidit à 0°C, ramène le pH à 4,6 par ajout d'acide chlorhydrique concentré et extrait avec de l'éther éthylique (après évaporation on recueille 14,7 g de 3-fluorocatéchol de départ) on acidifie la phase aqueuse avec de l'acide chlorhydrique concentré jusqu'à un pH de 1,8, extrait avec de l'acétate d'éthyle et obtient, après évaporation du solvant, 47,7 g du produit recherché (mélange d'isomères 2,3 bis hydroxy 4-fluoro ; 3,4bis-hydroxy 5-fluoro ; 2-fluoro 3,4bis-hydroxy) utilisé tel quel au stade A de l'exemple 16.

**PREPARATION 12 : 3-bromo 4,5-bis [(2-méthoxyéthoxy) méthoxy] benzaldéhyde.**

En opérant comme à la préparation 4 à partir de 5-bromo vanilline, on a préparé le produit attendu utilisé à l'exemple 15.

**EXEMPLE 1 : Sel interne de [6R-[3(E), 6alpha, 7béta(Z)]] 1-[3-[7-[[(2-amino 4-thiazolyl) [[carboxy (2-chloro 3,4-dihydroxyphényl) méthoxy] imino] acétyl] amino] 2-carboxy 8-oxo 5-thia 1-azabicyclo[4,2,0]oct-2-en-3-yl] 2-propènyl] quinoléinium**

**Stade A : Acide [2-chloro 3,4-bis[(2-méthoxy éthoxy) méthoxy] phényl] hydroxy acétique.**

2,42 g de chlorure de lithium et 7,02 g de potasse sont dissous dans 30 $cm^3$ d'eau à 0°C ; on ajoute à ce mélange une solution contenant 10 g de 2-chloro 3,4-bis[(2-méthoxy éthoxy) méthoxy benzaldéhyde (synthétisé à la préparation 1), 2,8 $cm^3$ de bromoforme et 38 $cm^3$ de dioxanne, laisse sous agitation pendant 24 Heures à 0°C, puis rajoute 2,8 $cm^3$ de bromoforme et laisse pendant 16 heures. Après dilution dans 100 $cm^3$ d'eau, lavage à l'éther, on décante, refroidit à 0°C, acidifie jusqu'à pH = 2,5-3 et extrait à l'éther. La phase organique est lavée à l'eau, séchée et le solvant chassé pour recueillir 11,4 g du produit recherché sous forme d'une huile. Rf = 0,1 [éluant : chlorure de méthylène-méthanol-acide acétique (89-10-1)].
Analyse Infra-rouge ($CHCl_3$)
Absorption générale dans la région -OH (acide + OH associé)
1726 $cm^{-1}$ complexe : C=O de la fonction acide
1598-1489 $cm^{-1}$ : noyau aromatique
Analyse RMN du proton ($CDCl_3$ 250 MHz en ppm)
3,37 (s l) (6 H) : $-OC\underline{H}_3$
3,57 (m) (4 H), 3,81 (m) (2 H) et 4,01 (m) (2 H) : $O-C\underline{H}_2-C\underline{H}_2-O$
5,25 (m) (4 H) et 5,52 (s 1) (1 H) : $-O-C\underline{H}_2-O$ et Ar-C<u>H</u>-CO 7,11 (s 1) (2 H) : Ar-<u>H</u>

**Stade B : [2-chloro 3,4-bis[(2-méthoxy éthoxy) méthoxy] phényl] hydroxy acétate de diphénylméthyle.**

On dissout à température ambiante et sous azote, 11,4 g de l'acide préparé au stade A dans 120 $cm^3$ de dichlorométhane, puis ajoute à 15°C, 120 $cm^3$ d'une solution 0,3 molaire de diphényldiazométhane dans le dichlorométhane et laisse sous agitation pendant 16 heures à température ambiante. On ajoute ensuite 30 $cm^3$ d'eau, décante et acidifie par 30 $cm^3$ d'acide acétique, lave avec une solution saturée de bicarbonate de soude, sèche et évapore les solvants. Après chromatographie sur silice en éluant par le mélange acétate d'éthyle-hexane (60-40), on recueille 8,1 g du produit recherché.
Analyse Infra-rouge ($CHCl_3$)
3528 $cm^{-1}$ : -OH non phénolique
1734 $cm^{-1}$ : C=O de la fonction ester
1597, 1496, 1487 $cm^{-1}$ : noyau aromatique
Analyse RMN du proton ($CDCl_3$ 300 MHz en ppm)
3,36 (s) : $-OC\underline{H}_3$
3,55 (s) (4 H), 3,82 (s) (2 H) et 4,03 (s) (2 H) :
$O-C\underline{H}_2-C\underline{H}_2-O$
5,27 (s) et 5,29 (s) : $-O-C\underline{H}_2-O$

5,67 (d après échange) : Ar-CH(C=O)-OH
6,88 (s) : CO-O-$CHAr_2$
7,0 (masqué) et 7,08 (d) : Ar-H couplés (ortho du cycle tétrasubstitué)
7,00 (2 H), 7,18 (3 H) et 7,30 (s) (5 H) : Ar-H

**Stade C : [2-chloro 3,4-bis[(2-méthoxy éthoxy) méthoxy] phényl] phtalimidoxy acétate de diphénylméthyle.**

On ajoute 2,47 g de N-hydroxy phtalimide et 7,55 g de triphényl phosphine à une solution de 8,1 g de l'ester préparé au stade B dans 90 $cm^3$ de tétrahydrofuranne, refroidit à 10°C puis verse 3,88 $cm^3$ d'azodicarboxylate de diéthyle (DEAD) et laisse sous agitation pendant 16 heures. Après concentration des solvants et chromatographie sur silice en éluant par le mélange acétate d'éthyle-hexane (60-40), on recueille 6,27 g du produit recherché sous forme d'une huile (Rf = 0,25).
Analyse Infra-rouge ($CHCl_3$)
1739, 1755 (ep) et 1795 $cm^{-1}$ : C=O
1597, 1489 $cm^{-1}$ : noyau aromatique
Analyse RMN du proton ($CDCl_3$ 300 MHz en ppm)
3,33 et 3,37 : $-OCH_3$
3,54 (m) (4 H), 3,82 (m) (2 H) et 3,92 (m) (2 H) : O-$CH_2$-$CH_2$-O
5,22 (sys. AB ) et 5,30 (sys. AB) : -O-$CH_2$-O
6,50 : Ar-CH(C=O)-O-
6,93 (s) ppm : CO-O-CH-$Ar_2$
7,09 (d), 7,41 (d) : Ar-H couplés (ortho du cycle tétrasubstitué)
7,44 (d) (4 H) : Ar-H du phtalimido
7,05 à 7,33 ppm : Ar-H aromatiques

**Stade D : Aminoxy [2-chloro 3,4-bis[(2-méthoxy éthoxy) méthoxy] phényl] acétate de diphénylméthyle.**

On dissout sous azote 6,27 g du produit préparé au stade précédent dans 65 $cm^3$ d'éthanol, refroidit à 5°C puis ajoute 0,47 $cm^3$ d'hydrate d'hydrazine, maintient à 5°C pendant une demi-heure puis revient à température ambiante en 2 heures. Après filtration, évaporation du solvant et chromatographie sur silice en éluant par l'acétate d'éthyle, on recueille 3,64 g du produit recherché sous forme d'une huile. Rf = 0,3

| Microanalyse FM = $C_{29}H_{34}ClNO_9$ | | | |
|---|---|---|---|
| calculés | C% 60,46 | H% 5,95 | N% 2,43 |
| trouvés | 60,1 | 6,1 | 2,6 |

Analyse Infra-rouge ($CHCl_3$)
3235 $cm^{-1}$ : $-NH_2$
1738 $cm^{-1}$ : C=O de la fonction ester
1598, 1572, 1490 $cm^{-1}$ : noyau aromatique + $NH_2$

**Stade E : Acide [[[1-[2-chloro 3,4-bis[(2-méthoxy éthoxy) méthoxy] phényl] 2-oxo 2-(diphenylméthoxy) éthyl] oxy] imino] [2-[(triphénylméthyl) amino] thiazol-4-yl] acétique.**

On agite pendant 3 heures sous azote et à température ambiante 0,69 g du produit préparé au stade précédent et 0,496 g d'acide oxo [2-[(triphénylméthyl) amino] thiazol-4-yl] acétique (décrit dans la demande de brevet belge N° 864828) en présence de 7 $cm^3$ de méthanol, puis élimine le solvant et chromatographie sur silice en éluant par le mélange chlorure de méthylène-méthanol (95-5) pour recueillir 0,726 g du produit attendu. Rf = 0,3 [éluant : $CHCl_2$-MeOH (90-10)]

| Microanalyse FM = $C_{53}H_{50}ClN_3O_{11}S$ | | | | | |
|---|---|---|---|---|---|
| calculés : | C% 65,45 | H% 5,18 | N% 4,32 | S% 3,29 | Cl% 3,64 |
| trouvés : | 63,7 | 5,1 | 4,2 | 3,2 | 3,1 |

**Stade F : 7béta- [[[[[1-[2-chloro 3,4-bis[(2-méthoxy éthoxy) méthoxy] phényl] 2-oxo 2-(diphénylméthoxy) éthyl] oxy] imino] [2-[(triphénylméthyl) amino] thiazol-4-yl] acétyl] amino] 3-[(Z)-3-chloro 1-propényl] 8-oxo 5-thia 1-azhbicyclo[4,2,0]oct-2-èn-2-carboxylate de 4-méthoxy benzyle.**

On agite 0,322 g de chlorhydrate de 7béta-amino 3-[(Z)-3-chloro 1-propényl] 8-oxo 5-thia 1-azabicyclo[4,2,0] oct-2-èn-2-carboxylate de 4-méthoxy benzyle (décrit dans la demande de brevet européen N° 0333 154), 0,72 g du produit préparé au stade précédent dans 10 $cm^3$, refroidit à 5°C puis on ajoute 0,1803 g de 1-(3-diméthylamino propyl) 3-éthyl carbodiimide (EDC), agite pendant une demi-heure puis traite avec 10 $cm^3$ d'hydrogénophosphate de potassium dans 10 $cm^3$ de chlorure de méthylène, décante, lave et sèche et évapore les solvants. On recueille 0,670 g de produit attendu. Rf =0,43 [éluant : chlorure de méthylène-acétate d'éthyle (80-20)].

**Stade G : 7béta-[[[[[1-[2-chloro 3,4-bis[(2-méthoxy éthoxy) méthoxy] phényl] 2-oxo 2-(diphénylméthoxy) éthyl] oxy] imino] [2-[(triphénylméthyl) amino] thiazol-4-yl] acétyl] amino] 3-[(Z)-3-iodo 1-propényl] 8-oxo 5-thia 1-azabicyclo[4,2,0]oct-2-èn-2-carboxylate de 4-méthoxy benzyle.**

On agite pendant une heure à température ambiante 0,67 g du produit obtenu au stade précédent dans 10 $cm^3$ d'acétone, 0,223 g d'iodure de sodium en présence d'un cristal d'iode. Après élimination du solvant, on reprend le résidu dans le chlorure de méthylène, lave la phase organique, sèche, élimine le solvant et recristallise à l'éther isopropylique pour recueillir 0,579 g de produit iodé. Rf = 0,27 [éluant : chlorure de méthylène-méthanol (90-10)].

**Stade H : Iodure de 1-[3-[7béta-[[[[[1-[2-chloro 3,4-bis[(2-méthoxy éthoxy) méthoxy] phényl] 2-oxo 2-(diphénylméthoxy) éthyl] oxy] imino] [2-[(tripbénylméthyl) amino] thiazol-4-yl] acétyl] amino] 2-[(4-méthoxy benzyloxy) carbonyl] 8-oxo 5-thia 1-azabicyclo[4,2,0] [oct-2-en-3-yl] 2-propènyl] quinoléinium**

On dissout 0,572 g du produit obtenu au stade précédent dans 6 $cm^3$ de chlorure de méthylène et ajoute 0,234 g de quinoléine, agite 1 heure, évapore le solvant, reprend dans l'éther, cristallise, essore et chromatographie si silice en éluant par le mélange chlorure de méthylène-méthanol (9-1). On recueille ainsi 0,235 g de produit attendu. Rf = 0,27 [éluant: chlorure de méthylène-méthanol (90-10)].
Analyse RMN du proton ($CDCl_3$ 250 MHz ; ppm)
3,31 à 3,34 : $-OCH_3$
3,73 (s) : $Ar-O-CH_2$
3,42 à 4,01 : CH2-S et $O-CH_2-CH_2-O$
4,92 (m) : -CH(N-)-S-
5,84 (m) : -NH-CH-C-
5,20 à 5,30 : $-O-CH_2-O$ et $COO-CH_2-Ar$
5,93 à 6,15 : $=C-CH_2-N^+$
6,40 à 6,55 : $=CH-CH^2-$ isomérie E et O-CH-Ar
6,76 dédoublé : H en position 5 du cycle thiazole
6,86 à 7,42 : -CH-Ar, $COO-CHAr_2$, =C-CH=CH- isomérie E 8,05 à 8,25 (3 H), 8,37 (m) (1 H), 8,92 (d (dédoublé)) (1 H), et 10,4 (d) : Hydrogènes du cycle quinoléine
7,83 et 8,27 : -CO-NH-

**Stade I : Sel interne de [6R-[3(E), 6alpha, 7béta(Z)]] 1-[3-[7-[[(2-amino 4-thiazolyl) [[carboxy (2-chloro 3,4-dihydroxyphényl) méthoxy] imino] acétyl] amino] 2-carboxy 8-oxo 5-thia 1-azabicyclo[4,2,0]oct-2-en-3-yl] 2-propènyl] quinoléinium isomère (R) et isomère (S)**

On agite à température ambiante pendant une heure et demi un mélange contenant 0,235 g du produit préparé au stade précédent et 5 $cm^3$ d'acide trifluoroacétique contenant 10 % d'anisole Après ajout d'éther, passage aux ultrasons, filtration, lavage et sèchage pendant 16 heures sous vide à température ambiante, on isole 0,118 g du sel interne recherché.

| Microanalyse FM = $C_{32}H_{25}ClN_6O_9S_2$ + 1,5 $C_2HF_3O_2$ + 0,5 HI pour PM = 1014 | | | | | | | |
|---|---|---|---|---|---|---|---|
| | C | H | N | Cl | S | F | I |
| % calculés : | 43,81 | 2,88 | 8,28 | 3,49 | 6,32 | 8,42 | 6,25 |
| % trouvés : | 44,1 | 2,7 | 8,5 | 4,1 | 6,7 | 8,5 | 6,0 |

Analyse RMN du proton (DMSO 300 MHz en ppm)
-S-C<u>H</u>$_2$-C(C=CH-)=C- masqué par l'eau du DMSO
5,15 (d dédoublé) ppm : CO-NH-CH(C=O)-C<u>H</u>-(N-)-S- 5,72 à 5,89 (4 H) : CO-NH-C<u>H</u>(C=O)-CH(N-)-S-, Ar-C<u>H</u>(CO-O-)-O- et -CH=CH-C<u>H</u>$_2$-$N^+$,
6,38 (m) ppm : -CH=C<u>H</u>-CH$_2$-$N^+$ isomérie E
6,7 à 6,82 : -S-C<u>H</u>=C(C=N-)-N=C(NH$_2$)- et Ar-<u>H</u> du catéchol 6,98 ppm (d J=15,5) : -C<u>H</u>=CH-CH$_2$-$N^+$ isomérie E
7,30 : N<u>H</u>$^2$
8,06 (t) (1 H), 8,25 (m) (2 H), 8,54 (m) (2 H) et 9,34 (d) (1 H), : Ar-<u>H</u> de la quinoléine
9,29 (s l) et 9,94 (s 1) : <u>H</u> mobiles
9,47 (d) et 9,58 (d) : Ar-<u>H</u> et -CO-N<u>H</u>-CH(C=O)-CH(N-)-S-

**<u>EXEMPLE 2</u> : le sel interne de [6R-[3(E), 6alpha, 7béta(Z)]] 1-[3-[7-[[(2-amino 4-thiazolyl) [[carboxy (3-cyano 4,5-dihydroxy phényl) méthoxy] imino] acétyl] amino] 2-carboxy 8-oxo 5-thia 1-azabicyclo[4,2,0]oct-2-en-3-yl] 2-propènyl] quinoléinium (R) ou (S) ou d'un mélange (R+S),**

**Stade A : Acide [3-cyano 4,5-bis[(2-méthoxy éthoxy) méthoxy] phényl] hydroxy acétique.**

En opérant comme au stade A de l'exemple 1, à partir de 6,2 g du produit obtenu au stade C de la préparation 2, 4,4 g de potasse, 3,15 g de bromure de lithium, 1,7 $cm^3$ de bromoforme et 15 $cm^3$, on obtient après agitation pendant 48 heures à -5°C, 7,2 g d'acide brut qui après chromatographie sur silice en éluant par le mélange dichlorométhane-méthanol-acide acétique (90-7-3) conduit à 3,42 g du produit recherché. Rf = 0,3 [éluant : chlorure de méthylène-méthanol-acide acétique (91-07-3)]
Analyse Infra-rouge ($CHCl_3$)
Absorption générale dans la région -OH (acide + OH associé) 1721 $cm^{-1}$ max + 1750 $cm^{-1}$ ép. : C=O de la fonction acide 2235 $cm^{-1}$
1602-1586-1489 $cm^{-1}$ : noyau aromatique

**Stade B : [3-cyano 4,5-bis[(2-méthoxy éthoxy) méthoxy] phényl] hydroxy acétate de diphénylméthyle.**

On dissout à température ambiante et sous azote, 8,5 g de l'acide préparé au stade A dans 100 $cm^3$ de dichloro-méthane, puis ajoute à 15°C, 75 $cm^3$ d'une solution 0,3 molaire de diphényldiazométhane dans l'éther et laisse sous agitation pendant 16 heures à température ambiante. On ajoute ensuite 30 $cm^3$ d'eau, décante et acidifie par 30 $cm^3$ d'acide acétique, lave avec une solution saturée de bicarbonate de soude, sèche et évapore les solvants. Après chromatographie sur silice en éluant par le mélange acétate d'éthyle-hexane (50-50), on recueille 4,2 g du produit recherché. Rf = 0,3.
Analyse Infra-rouge ($CHCl_3$)
3525 $cm^{-1}$ : -OH non phénolique
2235 $cm^{-1}$ : C≡N
1732 et 1750 $cm^{-1}$ : C=O de la fonction ester
1600, 1585, 1495, 1490 $cm^{-1}$ : noyau aromatique
Analyse RMN du proton ($CDCl_3$ 300 MHz en ppm)
3,32 à 3,47 : -OC<u>H</u>$_3$
3,58 et 3,72 : O-C<u>H</u>$_2$-C<u>H</u>$_2$-O
5,15 à 5,40 : -O-C<u>H</u>$_2$-O et Ar-C<u>H</u>(C=O)-OH
6,89 (s) : CO-O-C<u>H</u>Ar$_2$
7,24 à 7,50 : Ar-<u>H</u>

**Stade C : [3-cyano 4,5-bis[(2-méthoxy éthoxy) méthoxy] phényl] phtalimidoxy acétate de diphénylméthyle.**

On ajoute 0,134 g de N-hydroxy phtalimide et 0,409 g de triphényl phosphine à une solution de 0,430 g de l'ester préparé au stade B dans 10 $cm^3$ de tétrahydrofuranne, refroidit à -10°C puis verse 0,21 $cm^3$ d'azodicarboxylate de diéthyle (DEAD) et laisse sous agitation pendant 16 heures. Après concentration des solvants et chromatographie sur silice en éluant par le mélange dichlorométhane-acétone (95-5), on recueille 0,2 g du produit recherché sous forme d'une huile.
Rf = 0,25 [éluant : diclorométhane-acétone (97-3)].
Analyse Infra-rouge ($CHCl_3$)
1792 (m), 1775 (épaul) 1760 (épaul) et 1738 (max) $cm^{-1}$ : C=O 2230 $cm^{-1}$ : C≡N
1604, 1586, 1490 $cm^{-1}$ : noyau aromatique

**Stade D : Aminoxy [3-cyano 4,5-bis[(2-méthoxy éthoxy) méthoxy] phényl] acétate de diphénylméthyle.**

On dissout sous azote 2,7 g du produit préparé au stade précédent dans 80 $cm^3$ d'éthanol, refroidit à -2°C puis ajoute 0,20 $cm^3$ d'hydrate d'hydrazine, maintient à -2°C pendant une demi-heure puis revient à température ambiante en 1 heure. Après filtration, évaporation du solvant et chromatographie sur silice en éluant par le mélange acétate d'éthyle-hexane (7-3), on recueille 1,8 g du produit recherché sous forme d'une huile. Rf = 0,25

| Microanalyse FM = $C_{30}H_{34}N_2O_9$ | | | |
|---|---|---|---|
| calculés : | C% 63,53 | H% 6,04 | N% 4,94 |
| trouvés : | 63,3 | 6,4 | 4,6 |

Analyse Infra-rouge ($CHCl_3$)
3335 $cm^{-1}$ : O-$NH_2$
2230 $cm^{-1}$ : C≡N
1748 $cm^{-1}$ : C=O de la fonction ester
1600, 1588, 1488 $cm^{-1}$ : noyau aromatique + $NH_2$

**Stade E : Acide [([1-[3-cyano 4,5-bis[(2-méthoxy éthoxy) méthoxy] phényl] 2-oxo 2-(diphénylméthoxy) éthyl] oxy] imino] [2-[(triphénylméthyl) amino] thiazol-4-yl] acétique.**

On agite pendant 2 heures sous azote et à température ambiante 0,650 g du produit préparé au stade précédent et 0,428 g d'acide oxo [2-[(triphénylméthyl) amino] thiazol-4-yl] acétique (décrit dans la demande de brevet belge N° 864828) en présence de 6 $cm^3$ de méthanol, puis élimine le solvant, reprend le résidu dans l'éther et filtre ; le filtrat est chromatographié sur silice en éluant par le mélange chlorure de méthylène-méthanol (95-5) pour recueillir au total 0,841 g du produit attendu. PF = 158°C
Analyse Infra-rouge ($CHCl_3$)
3402 $cm^{-1}$ : =C-NH
Absorption générale OH/NH
2235 $cm^{-1}$ : C≡N
1755 (épaul), 1740 (Max) et 1717 (épaul) $cm^{-1}$ : C=O 1587, 1531, 1509 et 1490 $cm^{-1}$ : aromatique, hétérocycle, système conjugué
Analyse RMN du proton (en ppm)
3,36 (s) et 3,43 (s) : -$OC\underline{H}_3$
3,55 à 3,90 (m) (6 H) et 4,05 (m) (2 H) : O-$C\underline{H}_2$-$C\underline{H}_2$-O 5,03 (d), 5,12 (d) et 5,33 (d) : -O-$C\underline{H}_2$-O
5,85 (s) : Ar-C$\underline{H}$(C=O)-O-
6,68 (s) : CO-O-C$\underline{H}$$Ar_2$,
6,87 (s) : -S-C$\underline{H}$=C(C=N)-N
7,10 à 7,36 (massif) (27 H), 7,67 (d) (1 H) : Ar-$\underline{H}$

**Stade F : 7béta-[[[[[1-[3-cyano 4,5-bis[(2-méthoxy éthoxy) méthoxy] phényl] 2-oxo 2-(diphénylméthoxy) éthyl] oxy] imino] [2-[(triphénylmethyl) amino] thiazol-4-yl] acétyl] amino] 3-[(Z)-3-chloro 1-propényl] 8-oxo 5-thia 1-azabicyclo [4,2,0]oct-2-èn-2-carboxylate de 4-méthoxy benzyle.**

On agite 0,160 g de chlorhydrate de 7béta-amino 3-[(Z)-3-chloro 1-propényl] 8-oxo 5-thia 1-azabicyclo[4,2,0] oct-2-èn-2-carboxylate de 4-méthoxy benzyle (décrit dans la demande de brevet européen N° 0333 154), 0,36 g du produit préparé au stade précédent dans 5 $cm^3$ de dichlorométhane, refroidit à 5°C puis on ajoute 0,0893 g d'EDC, agite pendant une un quart d'heure puis traite avec 15 $cm^3$ d'hydrogénophosphate de potassium dans 8 $cm^3$ de dichlorométhane, décante, lave et sèche et évapore les solvants. Après chromatographie sur silice en éluant par le mélange dichlorométhane-acétate d'éthyle (8-2), on recueille 0,349 g de produit attendu. Rf =0,4 [éluant : dichlorométhane-acétate d'éthyle (8-2)]

**Stade G : 7béta-[[[[[1-[3-cyano 4,5-bis[(2-méthoxy éthoxy) méthoxy] phényl] 2-oxo 2-(diphénylméthoxy) éthyl] oxy] imino] [2-[(triphénylméthyl) amino] thiazol-4-yl] acétyl] amino] 3-[(Z)-3-iodo 1-propényl] 8-oxo 5-thia 1-azabicyclo[4,2,0]oct-2-èn-2-carboxylate de 4-méthoxy benzyle.**

On agite pendant une heure à température ambiante 0,573 g du produit obtenu au stade précédent dans 7 $cm^3$ d'acétone, 0,192 g d'iodure de sodium en présence d'un cristal d'iode. Après élimination du solvant, on reprend

le résidu dans le dichlorométhane, lave la phase organique, sèche, élimine le solvant et recristallise à l'éther isopropylique pour recueillir 0,463 g de produit iodé. Rf = 0,30 [éluant : dichlorométhane-méthanol (9-2)]

**Stade H : Iodure de 1-[3-[7béta-[[[[1-[3-cyano 4,5-bis[(2-méthoxy éthoxy) méthoxy] phényl] 2-oxo 2-(diphénylméthoxy) éthyl] oxy] imino] [2-[(triphénylméthyl) amino] thiazol-4-yl] acétyl] amino] 2-[(4-méthoxy benzyloxy) carbonyl] 8-oxo 5-thia 1-azabicyclo[4,2,0] [oct-2-en-3-yl] 2-propènyl] quinoléinium**

On dissout 0,463 g du produit obtenu au stade précédent dans 4 $cm^3$ de dichlorométhane et ajoute 0,191 g de quinoléine, agite 1 heure à température ambiante, évapore le solvant, reprend dans l'éther, cristallise, essore et chroma-tographie sur silice en éluant par le mélange dichlorométhane-méthanol (97-3). On recueille ainsi 0,241 g de produit attendu.
Rf = 0,27
Analyse RMN du proton ($CDCl_3$ 400 MHz ; ppm)
3,25 (s), 3,27 (s) et 3,34 : -OC<u>H</u>$_3$
3,26 à 4,04 : C<u>H</u>$_2$-S et O-C<u>H</u>$_2$-C<u>H</u>$_2$-O
3,98 (s) : Ar-O-C<u>H</u>$_3$
4,90 à 5,08 et 5,15 à 5,40 : -C<u>H</u>(N-)-S-, =C-C<u>H</u>$_2$-$N^+$, -O-C<u>H</u>$_2$-O et COO-C<u>H</u>$_2$-Ar
5,75 à 6,05 : N-C<u>H</u>(C=O)-CH(N-)-S et -O-C<u>H</u>(C=O)-Ar 6,38 (m) et 6,56 (m) : =C<u>H</u>-CH- isomérie E
6,73 (m) : -S-C<u>H</u>-C(C=N)-N
6,80 à 7,50 : Trityles, COO-C<u>H</u>Ar$_2$,
7,9 à 8,56 (5 H), 8,56 (d) et 8,87 (d) dédoublé (1 H), 10,27 (d) et 10,83 (d) : Hydrogènes du cycle quinoléine et -N<u>H</u>-

**Stade I : Sel interne de [6R-[3(E), 6alpha, 7béta(Z)]] 1-[3-[7-[[(2-amino 4-thiazolyl) [[carboxy (3-cyano 4,5-dihydroxy phényl) méthoxy] imino] acétyl] amino] 2-carboxy 8-oxo 5-thia 1-azabicyclo[4,2,0]oct-2-en-3-yl] 2-propènyl] quinoléinium isomère (R) et isomère (S)**

On agite à température ambiante pendant une heure et demi un mélange contenant 0,241 g du produit préparé au stade précédent et 5 $cm^3$ d'acide trifluoroacétique contenant 10 % d'anisole. Après ajout d'éther, cristallisation, filtration, lavage et séchage pendant 16 heures sous vide à température ambiante, on isole 0,128 g du sel interne recherché.

| Microanalyse FM = $C_{33}H_{25}N_7O_9S_2$ + 1,4 $C_2HF_3O_2$ + 0,4 HI | | | | | | |
|---|---|---|---|---|---|---|
| | C | H | N | S | F | I |
| % calculés : | 45,8 | 2,87 | 10,43 | 6,83 | 8,5 | 5,4 |
| % trouvés : | 44,1 | 2,7 | 8,5 | 7,0 | 8,4 | 4,1 |

Analyse RMN du proton (DMSO 300 MHz en ppm)
3,53 (d) et 3,74 (d) : -S-C<u>H</u>$_2$-C(C=CH-)=C-
5,14 (d dédoublé) ppm : CO-NH-CH(C=O)-C<u>H</u>-(N-)-S-
5,40 (s) : Ar-C<u>H</u>(CO-O-)-O-
5,76 (m) : CO-NH-C<u>H</u>(C=O)-CH(N-)-S-
à 5,89 (m) : -CH=CH-C<u>H</u>$_2$-$N^+$,
6,34 (m) ppm : -CH=C<u>H</u>-CH$_2$-$N^+$ isomérie E
6,76 (s) et 6,79 (s) : -S-C<u>H</u>=C(C=N-)-N=C(NH$_2$)-
6,98 ppm (d J=16) : -C<u>H</u>=CH-CH$_2$-$N^+$ isomérie E
7,11 à 7,15 (m) (2 H) : Ar-<u>H</u>
7,30 (m) et 10,38 (m) : <u>H</u> mobiles
8,06 (t) (1 H), 8,26 (m) (2 H), 8,53 (m) (2 H), 9,33 (d) et 9,58 (d) : Ar-<u>H</u>
9,60 (d) et 9,65 (d) : -CO-N<u>H</u>-CH(C=O)-CH(N-)-S-

**EXEMPLE 3 : le sel interne de [6R-[3(E), 6alpha, 7béta(Z)]] 1-[3-[7-[[(2-amino 4-thiazolyl) [[carboxy (3-fluoro 4,5-dihydroxy phényl) méthoxy] imino] acétyl] amino] 2-carboxy 8-oxo 5-thia 1-azabicyclo[4,2,0]oct-2-en-3-yl] 2-propènyl] quinoléinium (R) ou (S) ou d'un mélange (R+S).**

**Stade A : Acide [3-fluoro 4,5-bis[(2-méthoxy éthoxy) méthoxy] phényl] hydroxy acétique.**

0,662 g de bromure de lithium et 3,6 g de potasse sont dissous dans 13 $cm^3$ d'eau à 0°C ; on ajoute à ce mélange une solution contenant 5,5 g de 3-fluoro 4,5-bis[(2-méthoxy éthoxy) méthoxy benzaldéhyde (synthétisé à la préparation 3), 2,85 $cm^3$ de bromoforme et 13 $cm^3$ de dioxanne, laisse sous agitation pendant 24 Heures à 0°C, puis rajoute 2,85 $cm^3$ de bromoforme et laisse pendant 16 heures. Après dilution dans l'eau, lavage à l'éther, on décante, refroidit à 0°C, acidifie jusqu'à pH = 2,5-3 et extrait à l'éther. La phase organique est lavée à l'eau, séchée et le solvant chassé pour recueillir 5,3 g du produit recherché sous forme d'une huile. Analyse Infra-rouge ($CHCl_3$)
3600 $cm^{-1}$ : région -OH (acide + OH associé)
1715 $cm^{-1}$ complexe : C=O de la fonction acide
1616, 1595 et 1510 $cm^{-1}$ : noyau aromatique

**Stade B : [3-fluoro 4,5-bis[(2-méthoxy éthoxy) méthoxy] phényl] hydroxy acétate de diphénylméthyle.**

On dissout à température ambiante et sous azote, 5,3 g de l'acide préparé au stade A dans 66 $cm^3$ de dichlorométhane, puis ajoute à 15°C, 46 $cm^3$ d'une solution de diphényldiazométhane à 6,5 $g/dm^3$ dans l'éther et laisse sous agitation pendant 16 heures à température ambiante. On ajoute ensuite de l'eau, décante et acidifie par l'acide acétique, lave avec une solution saturée de bicarbonate de soude, sèche et évapore les solvants. Après chromatographie sur silice en éluant par le mélange acétate d'éthyle-hexane (50-50), on recueille 4,0 g du produit recherché. Rf = 0,17
Analyse Infra-rouge ($CHCl_3$)
3520 $cm^{-1}$ : -OH non phénolique
1735 $cm^{-1}$ : C=O de la fonction ester
1615, 1595 et 1509 $cm^{-1}$ : noyau aromatique
Analyse RMN du proton ($CDCl_3$ 250 MHz en ppm)
3,33 (s) et 3,37 (s) : $-OC\underline{H}_3$
3,46 (m), 3,57 (m), 3,78 (m) et 3,98 (m) : $O-C\underline{H}_2-C\underline{H}_2-O$ 5,19 à 5,30 : $-O-C\underline{H}_2-O$ et $Ar-C\underline{H}(C=O-O)-O-$
6,87 (s) : $CO-O-C\underline{H}Ar_2$
6,87 (m) (1 H), 6,97 (m) (2 H), 7,07 (m) (1 H), 7,21 (m) (3 H) et 7,32 (m) (5 H) : $Ar-\underline{H}$

**Stade C : [3-fluoro 4,5-bis[(2-méthoxy éthoxy) méthoxy] phé nyl] phtalimidoxy acétate de diphénylméthyle.**

On ajoute 0,77 g de N-hydroxy phtalimide et 2,42 g de triphényl phosphine à une solution de 2,6 g de l'ester préparé au stade B dans 28 $cm^3$ de tétrahydrofuranne, refroidit à -10°C puis ajoute goutte à goutte 1,4 $cm^3$ d'azodicarboxylate de diéthyle (DEAD) et laisse sous agitation à 0°C pendant une heure et demie. Après concentration des solvants et chroma-tographie sur silice en éluant par le mélange dichlorométhane-acétone (95-5), on recueille 2,56 g du produit recherché sous forme d'une huile. Rf = 0,6 [éluant dichlorométhane-acétone (85-15)].
Analyse Infra-rouge ($CHCl_3$)
1794, 1752 et 1738 (max) $cm^{-1}$ : C=O
Analyse RMN du proton ($CDCl_3$ 250 MHz en ppm)
3,34 (s) et 3,33 (s) : $-OC\underline{H}_3$
3,48 (m) (2 H), 3,56 (m) (2 H), 3,77 (m) (2 H) et 3,96 (m) (2 H) : $O-C\underline{H}_2-C\underline{H}_2-O$
5,22 (m) (4 H) : $-O-C\underline{H}_2-O$
5,92 (s) : $Ar-C\underline{H}(C=O-O)-O-$
6,92 (s) : $CO-O-C\underline{H}Ar_2$
7,00 à 7,35 (m) : $Ar-\underline{H}$
7,75 (m) (4 H) : $Ar-\underline{H}$ du phtalimide

**Stade D : Aminoxy [3-fluoro 4,5-bis[(2-méthoxy éthoxy) méthoxy] phényl] acétate de diphénylméthyle.**

On dissout sous azote 2,4 g du produit préparé au stade précédent dans 25 $cm^3$ d'éthanol, refroidit à -5°C puis ajoute 0,38 $cm^3$ d'hydrate d'hydrazine, maintient à -5°C pendant deux heures. Après filtration, évaporation du solvant et chromatographie sur silice en éluant par le mélange acétate d'éthyle-hexane (7-3), on recueille 1,67 g du produit recherché sous forme d'une huile. Rf = 0,3

Analyse Infra-rouge ($CHCl_3$)
3340 $cm^{-1}$ : O-$NH_2$
1747 $cm^{-1}$ : C=O de la fonction ester
1616, 1596, 1581, 1508 et 1497 $cm^{-1}$ : noyau aromatique + $NH_2$ Analyse RMN du proton ($CDCl_3$ 300 MHz en ppm)
3,34 et 3,36 : -OC<u>H</u>$_3$
3,48, 3,56, 3,77 et 3,97 : O-C<u>H</u>$_2$-C<u>H</u>$_2$-O
5,17 (s) : Ar-C<u>H</u>(C=O-O)-O-
5,20 et 5,21 : -O-C<u>H</u>$_2$-O
5,79 (s) : O-$NH_2$
6,82 (dd) : Ar-<u>H</u> en ortho de F
6,91 (s) : CO-O-C<u>H</u>Ar$_2$
7,02 : Ar-<u>H</u> en para de F
7,05 à 7,40 : Ar-<u>H</u>

**Stade E : Acide [[[1-[3-fluoro 4,5-bis[(2-méthoxy éthoxy) méthoxy] phényl] 2-oxo 2-(diphénylméthoxy) éthyl] oxy] imino] [2-[(triphénylméthyl) amino] thiazol-4-yl] acétique.**

On agite pendant 4 heures et demi sous azote et à température ambiante 0,640 g du produit préparé au stade précédent et 0,474 g d'acide oxo [2-[(triphénylméthyl) amino] thiazol-4-yl] acétique (décrit dans la demande de brevet belge N° 864828) en présence de 20 $cm^3$ de méthanol, puis élimine le solvant, et chromatographie sur silice en éluant par le mélange dichlorométhane-méthanol (95-5) pour recueillir au total 0,612 g du produit attendu. Rf = 0,35 [éluant : dichlorométhane-méthanol (9-1)].
Analyse Infra-rouge ($CHCl_3$)
3400$cm^{-1}$ : =C-NH
1755 (épaul), 1736 (Max) $cm^{-1}$ : C=O
1695, 1527, 1509 et 1496 $cm^{-1}$ : aromatique, hétérocycle, 1635 $cm^{-1}$ : C=O
Analyse RMN du proton ($CDCl_3$ en ppm)
3,09 (s) et 3,22 (s) : -OC<u>H</u>$_3$
3,30 (m), 3,55 (m), 3,60 (m), 3,92 (m), : O-C<u>H</u>$_2$-C<u>H</u>$_2$-O 5,15 (s), 5,10 (d) et 5,20 (d) : -O-C<u>H</u>$_2$-O
5,81 (s) : Ar-C<u>H</u>(C=O)-O-
6,56 (s) : CO-O-C<u>H</u>Ar$_2$,
6,74 (s) : -S-C<u>H</u>=C(C=N)-N
7,10 à 7,33 (m) : Ar-<u>H</u>

**Stade F : 7béta-[[[[[1-[3-fluoro 4,5-bis[(2-méthoxy éthoxy) méthoxy] phényl] 2-oxo 2-(diphénylméthoxy) éthyl] oxy] imino] [2-[(triphénylméthyl) amino] thiazol-4-yl] acétyl] amino] 3-[(Z)-3-chloro 1-propényl] 8-oxo 5-thia 1-azabicyclo[4,2,0]oct-2-èn-2-carboxylate de 4-méthoxy benzyle.**

On agite 0,289 g de chlorhydrate de 7béta-amino 3-[(Z)-3-chloro 1-propényl] 8-oxo 5-thia 1-azabicyclo[4,2,0] oct-2-èn-2-carboxylate de 4-méthoxy benzyle (décrit dans la demande de brevet européen N° 0333 154), 0,641 g du produit préparé au stade précédent dans 6 $cm^3$ de dichlorométhane, refroidit à 5°C puis on ajoute 0,160 g d'EDC, agite pendant une demi heure puis traite à l'hydrogénophosphate de potassium dans du dichlorométhane, décante, lave, sèche et évapore les solvants. Après chromatographie sur silice en éluant par le mélange dichlorométhane-acétate d'éthyle (8-2), on recueille 0,678 g de produit attendu. Rf = 0,4 [éluant : dichlorométhane-acétate d'éthyle (8-2)]

**Stade G : 7béta-[[[[[1-[3-fluoro 4,5-bis[(2-méthoxy éthoxy) méthoxy] phényl] 2-oxo 2-(diphénylméthoxy) éthyl] oxy] imino] [2-[(triphénylméthyl) amino] thiazol-4-yl] acétyl] amino] 3-[(Z)-3-iodo 1-propényl] 8-oxo 5-thia 1-azabicyclo[4,2,0]oct-2-èn-2-carboxylate de 4-méthoxy benzyle.**

On agite pendant une heure à température ambiante 0,678 g du produit obtenu au stade précédent dans 7 $cm^3$ d'acétone, 0,228 g d'iodure de sodium en présence d'un cristal d'iode. Après élimination du solvant, on reprend le résidu dans le dichlorométhane, lave la phase organique, sèche, élimine le solvant et essore le résidu obtenu pour recueillir 0,501 g de produit iodé. Rf = 0,2 [éluant : dichlorométhane-méthanol (97-3)].

**Stade H : Iodure de 1-[3-[7béta-[[[[(1-[3-fluoro 4,5-bis[(2-méthoxy éthoxy) méthoxy] phényl] 2-oxo 2-(diphénylméthoxy) éthyl] oxy] imino] [2-[(triphénylméthyl) amino] thiazol-4-yl] acétyl] amino] 2-[(4-méthoxy benzyloxy) carbonyl] 8-oxo 5-thia 1-azabicyclo[4,2,0] [oct-2-en-3-yl] 2-propènyl] quinoléinium**

On dissout 0,500 g du produit obtenu au stade précédent dans 2 $cm^3$ de dichlorométhane et ajoute 0,226 $cm^3$ de quinoléine, concentre le solvant, agite pendant une heure à température ambiante, rajoute 2 $cm^3$ de dichlorométhane, puis précipite par de l'éther et chromatographie sur silice en éluant par le mélange dichlorométhane-méthanol (97-3). On recueille ainsi 0,220 g de produit attendu. Rf = 0,20

**Stade I : Sel interne de [6R-[3(E), 6alpha, 7béta(Z)]] 1-[3-[7-[[(2-amino 4-thiazolyl) [[carboxy (3-fluoro 4,5-dihydroxy phényl) méthoxy] imino] acétyl] amino] 2-carboxy 8-oxo 5-thia 1-azabicyclo[4,2,0]oct-2-en-3-yl] 2-propènyl] quinoléinium isomère (R) et isomère (S)**

On agite à température ambiante pendant une heure et demie un mélange contenant 0,220 g du produit préparé au stade précédent et 5 $cm^3$ d'acide trifluoroacétique contenant 10 % d'anisole. Après ajout de 15 $cm^3$ d'éther, le précipité formé est lavé et séché, et conduit à 0,0994 g du sel interne recherché.

| Microanalyse FM = $C_{32}H_{25}FN_6O_9S_2$ + 1,2 $C_2HF_3O_2$ + 0,5 HI | | | | | | |
|---|---|---|---|---|---|---|
| | C | H | N | S | F | I |
| % calculés : | 44,8 | 2,89 | 9,12 | 6,95 | 9,48 | 6,88 |
| % trouvés : | 44,7 | 2,8 | 8,8 | 6,6 | 9,1 | 6,6 |

Analyse RMN du proton (DMSO 300 MHz en ppm)
3,5 et 3,8 : (masqué par l'eau du solvant)
5,14 (d dédoublé) ppm : CO-NH-CH(C=O)-C<u>H</u>-(N-)-S-
5,34 (s dédoublé) : Ar-C<u>H</u>(CO-O-)-O-
5,77 (dd dédoublé d dédoublé) : CO-NH-C<u>H</u> (C=O)-CH(N-)-S- 5,80 à 6,0 : -CH=CH-C<u>H</u>$_2$-N$^+$,
6,37 (m) : -CH=C<u>H</u>-CH$_2$-N$^+$ isomérie E
6,7 à 6,8 : -S-C<u>H</u>=C(C=N-)-N=C(NH$_2$)- et Ar-<u>H</u> en ortho et para du F
6,98 ppm (d dédoublé) : -C<u>H</u>=CH-CH$_2$-N$^+$ isomérie E
8,07 (t), 8,29 (t) : <u>H</u> en positions 6 et 7 du quinoléinium
8,24 (dd) : <u>H</u> en position 3 du quinoléinium
8,53 : <u>H</u> en positions 5 et 8 du quinoléinium
9,34 (d) : <u>H</u> en position 4 du quinoléinium
9,58 (d) : <u>H</u> en position 2 du quinoléinium
7,31-9,19 et 9,5 à 9,7 : <u>H</u> mobiles

**<u>EXEMPLE 4</u> : le sel interne de [(±) (cis) (Z)] 7-[3-[7-[[(2-amino 4-thiazolyl) [[carboxy (3-chloro 4,5-dihydroxy phényl) méthoxy] imino] acétyl] amino] 2-carboxy 8-oxo 5-thia 1-aza-bicyclo[4,2,0]oct-2-en-3-yl] 2(E)-propènyl] thièno[2,3-b]pyridinium (R) ou (S) ou d'un mélange (R+S)**

**Stade A : Acide [3-chloro 4,5-bis[(2-méthoxy éthoxy) méthoxy] phényl] hydroxy acétique.**

3,34 g de chlorure de lithium et 9,64 g de potasse sont dissous dans 36 $cm^3$ d'eau à 0°C ; on ajoute à ce mélange une solution contenant 13,77 g de 3-chloro 4,5-bis[(2-méthoxy éthoxy) méthoxy benzaldéhyde (synthétisé à la préparation 4), 3,74 $cm^3$ de bromoforme et 34 $cm^3$ de dioxanne, laisse sous agitation pendant 24 heures à 0°C, puis rajoute 3,74 $cm^3$ de bromoforme et laisse pendant 16 heures. Après dilution dans 100 $cm^3$ d'eau, lavage à l'éther, on décante, refroidit à 0°C, acidifie jusqu'à pH = 2,5-3 et extrait à l'éther. La phase organique est lavée à l'eau, séchée et le solvant chassé pour recueillir 13,95 g du produit recherché sous forme d'une huile jaune.
Analyse Infra-rouge ($CHCl_3$)
1700 (max) et 1730 ( épaul) $cm^{-1}$ : C=O
1599, 1578, 1489 $cm^{-1}$ : noyau aromatique
Analyse RMN du proton ($CDCl_3$ 250 MHz en ppm)
3,38 (m) (6 H) : -OC<u>H</u>$_3$
5,09 à 5,35 (m) : -O-C<u>H</u>$_2$-O et Ar-C<u>H</u>(C=O)-O
7,05 à 7,78 ppm (m) (2 H) : Ar-<u>H</u>

**Stade B : [3-chloro 4,5-bis[(2-méthoxy éthoxy) méthoxy] phényl] hydroxy acétate de diphénylméthyle.**

On dissout à température ambiante et sous azote, 13,95 g de l'acide préparé au stade A dans 150 $cm^3$ d'éther, puis ajoute à 15°C 150 $cm^3$ d'une solution 0,3 molaire de diphény-diazométhane dans le dichlorométhane et laisse sous agitation pendant 16 heures à température ambiante. On ajoute ensuite 30 $cm^3$ d'eau, décante et acidifie par 30 $cm^3$ d'acide acétique, lave avec une solution saturée de bicarbonate de soude, sèche et évapore les solvants. Après chromatographie sur silice en éluant par le mélange dichlorométhane-méthanol (99-1), on recueille 10 g du produit recherché.
Analyse Infra-rouge ($CHCl_3$)
3535 $cm^{-1}$ **:** -OH non phénolique
1738 $cm^{-1}$ : C=O de la fonction ester
1600, 1580, 1489 $cm^{-1}$ : noyau aromatique
Analyse RMN du proton ($CDCl_3$ 250 MHz en ppm)
3,59 (m) et 3,47 (m) (4 H), 3,75 (m) (2 H) et 4,04 (m) (2 H) : O-C$\underline{H}_2$-C$\underline{H}_2$-O
3,38, 3,33 et 3,25 (m) (6 H) : -OC$\underline{H}_3$
5,02 à 5,30 (m) : O-C$\underline{H}_2$-O et Ar-C$\underline{H}$(C=O)-O
6,8 à 7,35 (m) : Ar-$\underline{H}$

**Stade C : [3-chloro 4,5-bis[(2-méthoxy éthoxy) méthoxy] phényl] phtalimidoxy acétate de diphénylméthyle.**

On ajoute 1 g de N-hydroxy phtalimide et 1,54 g de triphényl phosphine à une solution de 1 g de l'ester préparé au stade B dans 35 $cm^3$ de tétrahydrofuranne, refroidit à 10°C puis ajoute goutte à goutte 1,58 g d'azodicarboxylate de diéthyle (DEAD) et laisse sous agitation pendant 16 heures.
Après concentration des solvants et chromatographie sur silice en éluant par le mélange dichlorométhane-méthanol (99-1), on recueille 3,09 g du produit recherché sous forme d'une huile incolore (Rf =0,3).
Analyse Infra-rouge ($CHCl_3$)
1738, 1755 (ep) et 1795 $cm^{-1}$ : C=O
1600, 1578, 1488 $cm^{-1}$ : noyau aromatique
Analyse RMN du proton ($CDCl_3$ 250 MHz en ppm)
3,34 (s), et 3,36 (s) : -OC$\underline{H}_3$
3,49 (m) (2 H), 3,57 (m) (2 H), 3,77 (m) (2 H) et
4,02 (m) (2 H) ppm : O-C$\underline{H}_2$-C$\underline{H}_2$-O
5,22 (s) et 5,27 (s) ppm : -O-C$\underline{H}_2$-O
5,91 (s) et 5,92 (s) ppm : Ar-C$\underline{H}$(C=O)-O
6,92 (s) ppm : CO-O-C$\underline{H}$-$Ar_2$
7,75 ppm (m) (4 H) : Ar-$\underline{H}$ du phtalimido
7,11(m) et 7,21 à 7,33 ppm : les autres $\underline{H}$ aromatiques

**Stade D : Aminoxy [3-chloro 4,5-bis[(2-méthoxy éthoxy) méthoxy] phényl] acétate de diphénylméthyle.**

On dissout sous azote 3 g du produit préparé au stade précédent dans 40 $cm^3$ d'éthanol, refroidit à 5°C puis ajoute 0,224 $cm^3$ d'hydrate d'hydrazine, maintient à 5°C pendant une demi-heure puis revient à température ambiante pendant 2 heures. Après filtration et évaporation du solvant, on recueille le produit recherché sous forme d'une huile.
Rf = 0,3 [éluant : acétone-cyclohexane (3-7)]
Analyse Infra-rouge ($CHCl_3$)
3330 $cm^3$ : -$NH_2$
1745 $cm^{-1}$ : C=O
1600, 1578, 1488 $cm^{-1}$ : noyau aromatique + $NH_2$
Analyse RMN du proton ($CDCl_3$ 250 MHz en ppm)
3,34 (s) et 3,38 (s) : -OC$\underline{H}_3$
3,48 (m), 3,58 (m), 3,76 (m) et 4,02 (m) : O-C$\underline{H}_2$-C$\underline{H}_2$-O 5,17 : Ar-C$\underline{H}$(C=O)-O
5,19 (s) et 5,25 (s) : -O-C$\underline{H}_2$-O
5,80 : $NH_2$ mobile
6,91 (s) : CO-O-C$\underline{H}$-$Ar_2$
7,10 (m) (4 H) et 7,21 à 7,33 (8 H) : Ar-$\underline{H}$

**Stade E : Iodure de 7-[3-[7béta-[[[[[3-chloro 4,5-bis[(2-méthoxy éthoxy) méthoxy] phényl] 2-oxo 2-(diphénylméthoxy) éthyl] oxy] imino] [2-[(triphénylméthyl) amino] thiazol-4-yl] acétyl] amino] 2-[(4-méthoxy benzyloxy) carbonyl] 8-oxo 5-thia 1-azabicyclo[4,2,0] [oct-2-en-3-yl] 2-propènyl] thièno-[2,3-b]pyridinium**

On mélange pendant 5 minutes sous azote 0,441 g du produit préparé au stade précédent, 24 $cm^3$ de méthanol et 145 mg d'acide para-toluènesulfonique et on ajoute une solution de 0,65 mg d'iodure de 1-[3-[7béta-[[oxo [2-[(triphénylméthyl) amino] thiazol-4-yl] acétyl] amino] 2-[(4-méthoxy benzyloxy) carbonyl] 8-oxo 5-thia 1-azabicyclo [4,2,0] [oct-2-en-3-yl] 2-propènyl] thièno-[2,3-b]pyridinium (synthétisé à la préparation 10) puis laisse sous agitation pendant 16 heures. Après évaporation du solvant, on reprend le résidu dans l'éther, filtre le précipité, lave et sèche et récupère ainsi 0,730 g du produit recherché. Point de fusion (PF) =135°C (gomme) ; Rf = 0,3 [éluant dichlorométhane-méthanol (9-1)]

**Stade F : le sel interne de [(±)(cis)(Z)] 7-[3-[7-[[(2-amino 4-thiazolyl) [[carboxy (3-chloro 4,5-dihydroxy phényl) méthoxy] imino] acétyl] amino] 2-carboxy 8-oxo 5-thia 1-azabicyclo [4,2,0]oct-2-en-3-yl] 2(E)-propènyl] thièno [2,3-b]pyridinium (R) ou (S) ou d'un mélange (R+S),**

On verse une solution à 0°C de 31 $cm^3$ d'acide trifluoroacétique dans 8,5 $cm^3$ de dichlorométhane, dans un mélange à 0°C contenant 0,69 mg du produit préparé au stade précédent, 17 $cm^3$ de dichlorométhylène et 3 $cm^3$ d'anisole, agite à cette température pendant une heure, élimine les solvants, cristallise, filtre, lave, sèche, chromatographie en éluant par le mélange acétonitrile-eau (1-1) et isole ainsi 0,22 g du produit recherché.
Analyse RMN du proton (DMSO 300 MHz en ppm)
3,19 : C=C-$CH_2$-S-
5,01 (d) et 5,07(d) : CO-NH-CH(C=O)-CH-(N-)-S-
5,12 (s) et 5,17 (s) : Ar-CH(CO-O-)-O-
5,52 (1) : -CH=CH-$CH_2$-$N^+$,
5,60 : CO-NH-CH(C=O)-CH(N-)-S- (cis/H en 6)
5,87 (m) : -CH=CH-$CH_2$-$N^+$ isomérie E
6,78 à 6,86 : -S-CH=C(C=N-)-N=C($NH_2$)- et Ar-H en ortho et para du Cl
7,21 et 7,41 : -$NH_2$
7,87 (d) : H en position 3 du thièno[2,3-b]pyridinium
8,10 (l) : H en position 5 du thièno[2,3-b]pyridinium
8,26 (d) : H en position 2 du thièno[2,3-b]pyridinium
9,03 (l) : H en position 4 du thièno[2,3-b]pyridinium
9,23 (l) : H en position 6 du thièno[2,3-b]pyridinium
9,74 (d) : CO-NH-CH(C=O)-CH(N-)-S

En opérant de manière analogue à celle de l'exemple 4 et en utilisant les produits synthétisés aux préparations 1 à 12, on a obtenu les composés suivants :

**EXEMPLE 5 : le sel interne de [[6R-[3(E), 6alpha, 7béta(Z)]] 7-[3-[7-[[(2-amino 4-thiazolyl) [[carboxy (3,4-dihydroxy 5-nitro phényl) méthoxy] imino] acétyl] amino] 2-carboxy 8-oxo 5-thia 1-azabicyclo[4,2,0]oct-2-en-3-yl] 2-propènyl] thièno-[2,3-b]pyridinium (R) ou (S) ou d'un mélange (R+S),**

Analyse RMN du proton (DMSO 300 MHz en ppm)
3,49 à 3,9 (m) : C=C-$CH_2$-S-
3,14 (d) et 5,18 (d) : CO-NH-CH(C=O)-CH-(N-)-S-
5,47 (s),5,50 (s), 5,60 (s), 5,63 (s) : Ar-CH(CO-O-)-O- 5,67 (d) : -CH=CH-$CH_2$-$N^+$,
5,77 (m) : CO-NH-CH(C=O)-CH(N-)-S-
6,29 (m) : -CH=CH-$CH_2$-$N^+$
6,80 (s), 6,83 (s),
7,64 (s) et 7,66 (s) : -S-CH=C(C=N-)-N=C($NH_2$)-
7,42 (d) et 7,47 (d) : Ar-H en ortho du nitro
7,1 à 7,2 (m) : -CH=CH-$CH_2$-$N^+$ et Ar-H en para du nitro
7,89 (d) : H en position 3 du thièno[2,3-b]pyridinium
8,15 (dd) : H en position 5 du thièno[2,3-b]pyridinium
8,29 (d) : H en position 2 du thièno[2,3-b]pyridinium
9,09 (d) : H en position 4 du thièno[2,3-b]pyridinium
9,23 (d) : H en position 6 du thièno[2,3-b]pyridinium
9,54 (d déd.), 9,65 (d) et 9,69 (d) : CO-NH-CH(C=O)-CH(N-)-S 10,42 (m) : H mobiles

**EXEMPLE 6 : le sel interne de [(±)(cis)(Z)] 7-[3-[7-[[(2-amino 4-thiazolyl) [[carboxy (3,4-dihydroxy 5-iodo phényl) méthoxy] imino] acétyl] amino] 2-carboxy 8-oxo 5-thia 1-azabicyclo[4,2,0]oct-2-en-3-yl] 2(E)-propènyl] thièno [2,3-b]pyridinium (R) ou (S) ou d'un mélange (R+S),**

| Microanalyse FM = $C_{30}H_{23}IN_6O_9S_3$ + 1 $C_2HF_3O_2$ + 1 APTS | | | | | |
|---|---|---|---|---|---|
| | C | H | N | S | I |
| % calculés : | 41,78 | 2,85 | 7,5 | 11,43 | 11,3 |
| % trouvés : | 46,6 | 2,8 | 7,8 | 12,0 | 10,9 |

Analyse RMN du proton (DMSO 300 MHz en ppm)
3,70 : C=C-$CH_2$-S-
5,11 (s) et 5,17(s) (1 H) : CO-NH-CH(C=O)-CH-(N-)-S-
5,29 (s) et 5,32 (s) : Ar-CH(CO-O-)-O-
5,67 (d) : -CH=CH-$CH_2$-$N^+$,
5,75 (m) : CO-NH-CH(C=O)-CH(N-)-S-
6,29 : -CH=CH-$CH_2$-$N^+$ isomérie E
6,77 (dédoubl.) (1 H) : -S-CH=C(C=N-)-N=C($NH_2$)-
6,90 (2 H) : Ar-H en ortho et para du I
7,89 (d) : H en position 3 du thièno[2,3-b]pyridinium
8,15 (dd) : H en position 5 du thièno[2,3-b]pyridinium
8,29 (d) : H en position 2 du thièno[2,3-b]pyridinium
9,08 (d) : H en position 4 du thièno[2,3-b]pyridinium
9,13 (d) : H en position 6 du thièno[2,3-b]pyridinium
9,37 (sl) : CO-NH-CH(C=O)-CH(N-)-S

**EXEMPLE 7 : le sel interne de [(±)(cis)(Z)] 7-[3-[7-[[(2-amino 4-thiazolyl) [[carboxy (3,4,5-trihydroxy phényl) méthoxy] imino] acétyl] amino] 2-carboxy 8-oxo 5-thia 1-azabicyclo[4,2,0]oct-2-en-3-yl] 2(E)-propènyl] thièno [2,3-b]pyridinium (R) ou (S) ou d'un mélange (R+S),**

Analyse RMN du proton (DMSO 300 MHz en ppm)
5,15 (d) et 5,17(d) (1 H) : CO-NH-CH(C=O)-CH-(N-)-S- [2/5<(R/S)<3/5]
5,22 et 5,34 (2 H) : Ar-CH(CO-O-)-O-
5,67 (d) : -CH=CH-$CH_2$-$N^+$,
5,76 (m), 6,20 à 6,40 (m) : CO-NH-CH(C=O)-CH(N-)-S-, -CH=CH-$CH_2$-$N^+$ et Ar-H du triphénol
6,75, 6,77, 6,87 et 6,90 (1 H) : -S-CH=C(C=N-)-N=C($NH_2$)-
7,74 (d) : H en position 5 du thièno[2,3-b]pyridinium
7,89 (d) et 8,28 (d) : H en positions 4 et 3 du thièno[2,3-b]pyridinium
8,10 à 8,27, 9,09 et 9,23 : H en positions 2 et 6 du thièno[2,3-b]pyridinium
9,6 à 9,85 : CO-NH-CH(C=O)-CH(N-)-S

**EXEMPLE 8 : le sel interne de [(±)(cis)(Z)] 7-[3-[7-[[(2-amino 4-thiazolyl) [[carboxy (2-chloro 3,4-dihydroxy phényl) méthoxy] imino] acétyl] amino] 2-carboxy 8-oxo 5-thia 1-aza-bicyclo[4,2,0]oct-2-en-3-yl] 2 (E)-propènyl] thièno[2,3-b]pyridinium (R) ou (S) ou d'un mélange (R+S),**

| Microanalyse FM = $C_{30}H_{23}IN_6O_9S_3$ + 2 APTS | | | | | |
|---|---|---|---|---|---|
| | C | H | N | S | Cl |
| % trouvés : | 46,8 | 2,55 | 8,53 | 9,76 | 3,59 |

Analyse RMN du proton (DMSO 300 MHz en ppm)
3,5 à 3,9 (m) : C=C-$CH_2$-S-
5,17(m) (1 H) : CO-NH-CH(C=O)-CH-(N-)-S-

5,67 (dl) : $-CH=CH-C\underline{H}_2-N^+$,
5,70 à 5,90 (2 H) : CO-NH-C<u>H</u>(C=O)-CH(N-)-S- + autre <u>H</u>
6,33 (m) : $-C\underline{H}=CH-CH_2-N^+$ isomérie E
6,79 (m) et 6,96 (m) (4 H) : 4 <u>H</u>
7,89 (d) et 8,29 (d) : <u>H</u> en positions 2 et 3 du thièno[2,3-b]pyridinium
8,14 (m) : <u>H</u> en position 5 du thièno[2,3-b]pyridinium
9,08 (d) : <u>H</u> en position 4 du thièno[2,3-b]pyridinium
9,26 (d) : <u>H</u> en position 6 du thièno[2,3-b]pyridinium

**<u>EXEMPLE 9</u> : le sel interne de [[6R-[3(E), 6alpha, 7béta(Z)]] 7-[3-[7-[[(2-amino 4-thiazolyl) [[carboxy (2,5-dichloro 3,4-dihydroxy phényl) méthoxy] imino] acétyl] amino] 2-carboxy 8-oxo 5-thia 1-azabicyclo[4,2,0]oct-2-en-3-yl] 2-propènyl] thièno[2,3-b]pyridinium (R) ou (S) ou d'un mélange (R+S),**

Analyse RMN du proton (DMSO 300 MHz en ppm)
5,20 : Ar-<u>H</u> en ortho du Cl
5,79 : Ar-C<u>H</u>(CO-O-)-O-
5,78 (m) : $-CH=CH-C\underline{H}_2-N^+$,
6,31 : $-CH=C\underline{H}-CH_2-N^+$
7,88 : <u>H</u> en position 3 du thièno[2,3-b]pyridinium 8,15 (m) : <u>H</u> en position 5 du thièno[2,3-b]pyridinium
8,98 (m) : <u>H</u> en position 2 du thièno[2,3-b]pyridinium
9,08 (d) : <u>H</u> en position 4 du thièno[2,3-b]pyridinium
9,13 (d) : <u>H</u> en position 6 du thièno[2,3-b]pyridinium

**<u>EXEMPLE 10</u> : le sel interne de [[6R-[3(E), 6alpha, 7béta(Z)]] 7-[3-[7-[[(2-amino 4-thiazolyl) [[carboxy (3-cyano 4,5-dihydroxy phényl) méthoxy] imino] acétyl] amino] 2-carboxy 8-oxo 5-thia 1-azabicyclo[4,2,0]oct-2-en-3-yl] 2-propènyl] thièno-[2,3-b]pyridinium (R) ou (S) ou d'un mélange (R+S),**

Analyse RMN du proton (DMSO 300 MHz en ppm)
3,19 : $C=C-CH_2-S-$
5,15 (d) et 5,20 (d) : CO-NH-CH(C=O)-C<u>H</u>-(N-)-S-
5,40 (s) : Ar-C<u>H</u>(CO-O-)-O-
5,67 (d) : $-CH=CH-C\underline{H}_2-N^+$,
5,81 (m) : CO-NH-C<u>H</u>(C=O)-CH(N-)-S-
6,67 (s) : $-S-C\underline{H}=C(C=N-)-N=C(NH_2)-$
7,10 à 7,20 (m) (3 H) : Ar-<u>H</u> et C<u>H</u>=
7,34 (m), 10,27 à 10,42 (m) : <u>H</u> mobiles
7,89 (d) : <u>H</u> en position 3 du thièno[2,3-b]pyridinium
8,15 (dd) : <u>H</u> en position 5 du thièno[2,3-b]pyridinium
8,28 (d) : <u>H</u> en position 2 du thièno[2,3-b]pyridinium
9,09 (d) : <u>H</u> en position 4 du thièno[2,3-b]pyridinium
9,23 (d) : <u>H</u> en position 6 du thièno[2,3-b]pyridinium
9,52 (d) et 9,62 (d) : CO-N<u>H</u>-CH(C=O)-CH(N-)-S

**<u>EXEMPLE 11</u> : le sel interne de [[6R-[3(E), 6alpha, 7béta(Z)]] 7-[3-[7-[[(2-amino 4-thiazolyl) [[carboxy (3,4-dihydroxy 5-méthoxy phényl) méthoxy] imino] acétyl] amino] 2-carboxy 8-oxo 5-thia 1-azabicyclo(4,2,0]oct-2-en-3-yl] 2-propènyl] thièno[2,3-b]pyridinium (R) ou (S) ou d'un mélange (R+S),**

Les isomères (R) et (S) sont séparés par HPLC (colonne Microbondapack C18), solvant : Eau/acétonitrile (85/15)

**Isomère (S)**

Analyse RMN du proton (DMSO 300 MHz en ppm)
3,71 (s) : $Ar-OC\underline{H}_3$
5,16 (d J=5) : CO-NH-CH(C=O)-C<u>H</u>-(N-)-S-
5,32 (s) : Ar-C<u>H</u>(CO-O-)-O-
5,68 (d) : $-CH=CH-C\underline{H}_2-N^+$,
5,79 (dd J=5 et 7,5) : CO-NH-C<u>H</u>(C=O)-CH(N-)-S-

6,28 (m) : -CH=CH-$CH_2$-$N^+$,
6,55 (s) (2 H) : Ar-H en ortho et para du -OMe
6,78 (s) : -S-CH=C(C=N-)-N=C($NH_2$)-
7,11 (d) : -CH=CH-$CH_2$-$N^+$,
7,29 (m), 8,38 (m), 8,99 (m) : H mobiles
7,88 (d) : H en position 3 du thièno[2,3-b]pyridinium
8,15 (dd) : H en position 5 du thièno[2,3-b]pyridinium
8,29 (d) : H en position 2 du thièno[2,3-b]pyridinium
9,08 (d) : H en position 4 du thièno[2,3-b]pyridinium
9,22 (d) : H en position 6 du thièno[2,3-b]pyridinium
9,55 (d) : CO-NH-CH(C=O)-CH(N-)-S

**Isomère (R)**

Analyse RMN du proton (DMSO 300 MHz en ppm)
3,50 (s) (masqué en partie) : C=C-$CH_2$-S-
3,72 (s) : Ar-$OCH_3$
5,19 (d J=5) : CO-NH-CH(C=O)-CH-(N-)-S-
5,32 (s) : Ar-CH(CO-O-)-O-
5,68 (d J=6) : -CH=CH-$CH_2$-$N^+$,
5,76 (dd J=5 et 7,5) : CO-NH-CH(C=O)-CH(N-)-S-
6,29 (m) : -CH=CH-$CH_2$-$N^+$,
6,55 (s) et 6,57 (s) (2 H) : Ar-H en ortho et para du -OMe 6,74 (s) : -S-CH=C(C=N-)-N=C($NH_2$)-
7,15 (d) : -CH=CH-$CH_2$-$N^+$,
7,30 (m) (2 H), 8,39 (s) (1 H), 8,96 (s) (1 H) : H mobiles
7,89 (d) : H en position 3 du thièno[2,3-b]pyridinium
8,15 (dd) : H en position 5 du thièno[2,3-b]pyridinium
8,29 (d) : H en position 2 du thièno[2,3-b]pyridinium
9,08 (d) : H en position 4 du thièno[2,3-b]pyridinium
9,23 (d) : H en position 6 du thièno[2,3-b]pyridinium
9,62 (d) : CO-NH-CH(C=O)-CH(N-)-S

**EXEMPLE 12 : le sel interne de [[6R-[3(E), 6alpha, 7béta(Z)]] 1-[3-[7-[[(2-amino 4-thiazolyl) [[carboxy (2,5-dichloro 3,4-dihydroxy phényl) méthoxy] imino] acétyl] amino] 2-carboxy 8-oxo 5-thia 1-azabicyclo[4,2,0]oct-2-en-3-yl] 2-propènyl] quinoléinium (R) ou (S) ou d'un mélange (R+S),**

**EXEMPLE 13 : le sel interne de [[6R-[3(E), 6alpha, 7béta(Z)]] 2-[3-[7-[[(2-amino 4-thiazolyl) [[carboxy (2,5-dichloro 3,4-dihydroxy phényl) méthoxy] imino] acétyl] amino] 2-carboxy 8-oxo 5-thia 1-azabicyclo[4,2,0]oct-2-en-3-yl] 2-propènyl] isoquinoléinium (R) ou (S) ou d'un mélange (R+S),**

Rf = 0,6 (éluant : acétone-eau (8-2))

**EXEMPLE 14 : le sel interne de [[6R-[3(E), 6alpha, 7béta(Z)]] 1-[3-[7-[[(2-amino 4-thiazolyl) [[carboxy (2,5-dichloro 3,4-dihydroxy phényl) méthoxy] imino] acétyl] amino] 2-carboxy 8-oxo 5-thia 1-azabicyclo[4,2,0]oct-2-en-3-yl] 2-propènyl] 4-(méthylthio) pyridinium (R) ou (S) ou d'un mélange (R+S),**

Rf = 0,6 (éluant : acétone-eau (8-2)).

**<u>EXEMPLE 15</u> : le sel interne de [(±)(cis)(Z)] 7-[3-[7-[[(2-amino 4-thiazolyl) [[carboxy (3-bromo 4,5-dihydroxy phényl) méthoxy] imino] acétyl] amino] 2-carboxy 8-oxo 5-thia 1-azabicyclo[4,2,0]oct-2-en-3-yl] 2(E)-prapènyl] thièno[2,3-b]pyridinium (R) ou (S) ou d'un mélange (R+S)**

**<u>EXEMPLE 16</u> : sel interne de (6R(3(E), 6alpha, 7béta (Z))) 1-(3-(7-(((2-amino 4-thiazolyl) ((carboxy (2,3-dihydroxy 4-fluorophényl) méthoxy) imino) acétyl) amino) 2-carboxy 8-oxo 5-thia 1-azabicyclo(4.2.0)oct-2-en-3-yl) 2-propènyl) quinoléinium**

**Stade A : [4-fluoro-(2,3bis-hydroxy) phényl] hydroxy acétate de diphénylméthyle**

A une solution de 47,7 g de produit obtenu à la préparation 11 dans 500 $cm^3$ d'éther éthylique refroidie à -10°C, on ajoute en 2 heures 480 $cm^3$ d'une solution 0,3 M de diphényl diazométhane. On ramène la température à -5°C et ajoute 10 $cm^3$ d'acide acétique. Cette solution est utilisée telle quelle pour le stade suivant.

**Stade B : [4-fluoro [2,3bis[(2-méthoxyéthoxy) méthoxy] phényl] hydroxy acétate de diphénylméthyle**

A la solution obtenue au stade précédent, on ajoute 238 $cm^3$ de diisopropyléthylamine. On remplace l'éther par 500 $cm^3$ de chlorure de méthylène, on refroidit à 6/10°C et ajoute en 75 minutes 53 $cm^3$ de chlorure de méthoxy éthoxy méthyle et agite pendant encore 75 minutes.

On ajoute 500 $cm^3$ d'eau, décante, lave la phase organique avec de l'acide chlorhydrique N puis avec de la soude N et à l'eau, sèche et évapore à sec. On chromatographie le résidu sur silice en éluant avec le mélange acétate d'éthyle-hexane (1-1), on obtient 26,1 g de produit brut que l'on chromatographie à nouveau sur silice, éluant chlorure de méthylène-acétone (97-3), pour obtenir 14,58 g de produit recherché.
<u>Spectre RMN</u> : $CDCl_3$ 300 MHz ppm
3,34-3,36 : les C-O-Me ; 3,52-3,80-3,91 : les O-$CH_2$-$CH_2$-O ; 4,06 (dJ = 6,5) : H mobile ; 5,17 à 5,27 : les O-$CH_2$-O ; 5,49 (dJ = 7,5) : les $C_6H_5$-CH-O ; 6,84 (dd : J = 9 et 10) : $H_6$ ; 6,96 (dd J = 9 et 6) : $H_5$ ; 7,20 à 7,40 et 6,94 (sl) : les aromatiques.

**Stade C : [4-fluoro 2,3bis-[(2-méthoxyéthoxy) méthoxy] phényl] phtalimidoxy acétate de diphénylméthyle**

En opérant comme au stade C de l'exemple 4 à partir de 14,42 g du produit obtenu au stade B ci-dessus, après chroma-tographie sur silice, éluant chlorure de méthylène-méthanol (99-1) on obtient 11,89 g du produit recherché.
<u>Spectre IR</u> : $CHCl_3$
1792-1756-1734 $cm^{-1}$ : C=O ; 1600-1492 $cm^{-1}$ : aromatique.
<u>Spectre RMN</u> : $CDCl_3$ 250 MHz : pour les 2 groupements OMEM : 3,30 (s), 3,34 (s) : $OCH_3$ ; 3,47 (m)-3,84 (m) : O-$CH_2$-$CH_2$-O ; 5,10-5,27 : O-$CH_2$O ; puis 6,30 (s) : Ar-C<u>H</u>(ON=)-$CO_2^-$ ; 6,95 (s) $CO_2$C<u>H</u>$(C_6H_5)_2$ ; 6,84 (t) : $H_5$ ; 7,15 (2H)-7,19-7,32 (9H)-7,74(4H) : H aromatiques.

**Stade D : Aminoxy [4-fluoro 2,3bis[(2-méthoxy éthoxy) méthoxy] phényl] acétate de diphénylméthyl**

A une solution de 11,27 g du produit obtenu au stade C ci-dessus, dans 112,7 $cm^3$ de tétrahydrofuranne, on ajoute en 1 heure trente à -10°C, 0,88 $cm^3$ d'hydrate d'hydrazine. On agite 1 heure trente à -10°C, filtre l'insoluble et concentre le filtrat à sec. On chromatographie le résidu sur silice (éluant chlorure de méthylène-acétate d'éthyle (80-20) on obtient ainsi 4,72 g de produit recherché.
<u>Spectre RMN</u> : $CDCl_3$ 200 MHz
3,35 (s)-3,36 (s) : $OCH_3$ ; 3,54 (m) 4H-3,93 (m) 4H : O-$CH_2$-$CH_2$O ; 5,20 (s)-5,27 (s) : $OCH_2$O ; 6,78 (dd, J = 9,5 et 9) : $H_5$ ; 6,89 (m) : $H_6$ ; 5,73 (s) : Ar-C<u>H</u>($ONH_2$)-$CO_2$ ; 6,94 (s) : $CO_2$-C<u>H</u>-$(C_6H_5)_2$ ; 7,10 (m) 2H-7,21 (m) 5H : 10 H aromatiques $(C_6H_5)_2$.

**Stade E : Acide [[[1-[4-fluoro 2,3bis-[(2-méthoxy éthoxy) méthoxy] phényl] 2-oxo 2-(diphenylméthoxy) éthyl] oxy] imino] [2-[(triphénylméthyl) amino] thiazol 4-yl] acétique**

On opère comme au stade E de l'exemple 1 à partir de 4,65 g du produit obtenu au stade D, ci-dessus, en utilisant 3,45 g d'acide oxo [2-[(triphénylméthyl) amino] thiazol 4-yl] acétique (décrit dans la demande de brevet Belge n° 864828) on obtient après chromatographie sur silice (éluant chlorure de méthylène-méthanol (96-4)) 6,5 g du produit recherché.
<u>Spectre IR</u> : $CHCl_3$

3410 $cm^{-1}$ : =C-NH- ; 1781 $cm^{-1}$ : C=O ; 1618, 1607, 1528, 1496 $cm^{-1}$ : $CO_2$, aromatique et hétéroaromatique.
Spectre RMN : DMSO 300 MHz
3,18 (s)-3,21 (s) : $OCH_3$ ; 3,43 (m) 4H-3,81 (m) 4H : O-$CH_2$-$CH_2$-O ; 5,17 (s) 2H-5,21 (s) 2H : O-$CH_2$-O ; 5,87 (s) <3/4H : $ArC\underline{H}(O\text{-}NH_2)\text{-}CO_2^-$ ; 6,68 (s) <3/4 H : $H_5$ thiazole ; 6,84 (s) <3/4H : $CO_2C\underline{H}(C_6H_5)_2$ ; ~6,95-7,10 : $H_5$,$H_6$ ; ~7,18-7,50 : autres aromatiques ; 8,65 (s,l) : =C-NH.

**Stade F : 7béta [[[[[1-[4-fluoro 2,3bis[(2-méthoxy éthoxy) méthoxy] phényl] 2-oxo 2-(diphényl méthoxy) éthyl] oxy] imino] [2-[(triphényl méthyl) amino] thiazoL 4-yl] acétyl] amino] 3-[(Z) 3-chloro 1-propényl] 8-oxo 5-thia 1-azabicyclo[4,2,0] oct-2-èn-2-carboxylate de 4-méthoxy benzyle**

On opère comme au stade F de l'exemple 1 mais à une température de -10°C, à partir de 6,44 g de produit obtenu au stade E ci-dessus. Après chromatographie sur silice, éluant chlorure de méthylène-acétate d'éthyle (90-10), on obtient 5,35 g de produit recherché.
Spectre RMN : $CDCl_3$ 400 MHz
3,05 (d,1H)-3,20 (d,lH) : $CH_2S$ ; [3,24 (s), 3,26 (s)] 3H-3,35 (s,3H) : $OCH_3$ ; 3,43 (m,2H)-3,55 (m, 2H)-3,70-4,15 : $OCH_2CH_2O$ et =CH-$C\underline{H}_2Cl$ ; 3,81 (s) : $C\underline{H}_3OAr$ ; 4,96 (d)-4,97 (d) : $H_6$ céphalo ; 5,12-5,31 (6-7H) : $OC\underline{H}_2O$, $CO_2C\underline{H}_2Ar$, $ArC\underline{H}(ONH_2)\text{-}CO_2^-$ ; 5,81 (m) : $H_7$ céphalo ; 5,75 (m) : CH=C$\underline{H}$-$CH_2Cl$ (ΔZ) ; 6,26 (d,J = 11)-6,29 (d,J = 11) : -C$\underline{H}$=CH-$CH_2$-Cl (ΔZ) ; ~ 6,77 : $H_5$ thiazole ; ~ 6,85-7,45 : H aromatiques + $CO_2C\underline{H}$ $(C_6H_5)_2$ ; 7,85-8,20 : NH.

**Stade G : 7béta [[[[[1-[4-fluoro 2,3bis[(2-méthoxy éthoxy) méthoxy] phényl] 2-oxo 2-(diphényl méthoxy) éthyl] oxy] imino] [2-[(triphényl méthyl) amino] thiazoL 4-yl] acétyl] amino] 3-[(Z) 3-iodo 1-propényl] 8-oxo 5-thia 1-azabicyclo[4,2,0] oct-2-èn-2-carbaxylate de 4-méthoxy benzyle**

On opère comme au stade G de l'exemple 1 à partir de 1,2 g de produit obtenu au stade précédent. On obtient ainsi 1,05 g de produit recherché.
Spectre RMN : 3,23 (s)-3,26 (s)-3,35 (s) : $CH_3O$ ; ~3,50 à 3,95 : O-$CH_2$-$CH_2O$ dont 3,81 : $CH_3OAr$ ; ~4,0 : =CH-$C\underline{H}_2$-I ; 4,92 (m) : $H_6$ céphalo ; 5,16-5,30 : $OC\underline{H}_2O$, $CO_2C\underline{H}_2Ar$ ; 5,50 (m) : $H_7$ céphalo ; 6,12 (m) : =C$\underline{H}$-$CH_2I$ (ΔE) ; 6,42 (s)-6,44 (s) : Ar-$C\underline{H}(ONH_2)CO_2^-$ ; 6,66 (t,J = 9, ~ 0,5H) : H aromatique en ortho de F ; 6,76 (s dédoublé) : $H_5$ thiazole ; 6,77-7,45 : H aromatiques ; 7,95 (d)-8,25 (d) : NH.

**Stade H : Iodure de 1-[3-[7béta [[[[[1-[4-fluoro 2,3bis[(2-méthoxy éthoxy) méthoxy] phényl] 2-oxo 2-(diphénylméthoxy) éthyl] oxy] imino] [2-[(triphénylméthyl) amino] thiazol 4-yl] acétyl] amino] 2-[(4-méthoxy benzyloxy) carbonyl] 8-oxo 5-thia 1-azabicyclo[4,2,0] [oct-2-èn-3-yl] 2-propényl] quinoléinium**

On opère comme au stade H de l'exemple 1 à partir de 1,02 g du produit obtenu au stade G ci-dessus en utilisant 0,46 g de quinoléïne. Après chromatographie sur silice, éluant chlorure de méthylène-méthanol (97-3), on recueille 347 mg du produit recherché (mélange 50/50 des deux isomères R et S de structure attendue).
Spectre RMN : $CDCl_3$ 400 MHz
3,20 (s)-3,26 (s)-3,34 (s)-3,35 (s) : $CH_3O$ ; 3,79 (s)-3,80 (s) : $CH_3OAr$ ; 3,42 (m)-3,54 (m)-3,79 (m)-3,90 (m) : $OCH_2CH_2O$ ; 4,89 (d) : $H_6$ céphalo ; 5,12-5,28 : $OCH_2O$, $CO_2C\underline{H}_2Ar$ ; 5,77 (dd dédoublé) $H_7$ céphalo ; 6,00 (dd)-6,13 (dd) : =CH-$C\underline{H}_2$-N ≡ ; 6,38 (s)-6,44 (s) : Ar-C$\underline{H}$-(O-)$CO_2$- ; 6,51 (m) : =C$\underline{H}$-$CH_2$ (ΔE) ; 6,72 (dédoublé) : $H_5$ thiazole ; 6,70 à 7,15 : $H_5$-$H_6$ fluorophényl, C$\underline{H}$=CH-$CH_2$, $CO_2C\underline{H}(C_6H_5)_2$ ; ~7,30 : H aromatiques $(C_6H_5)_3$-C.

**Stade I : sel interne de (6R(3(E), 6alpha, 7béta (Z))) 1-(3-(7-(((2-amino 4-thiazolyl) ((carboxy (2,3-dihydroxy 4-fluorophényl) méthoxy) imino) acétyl) amino) 2-carboxy 8-oxo 5-thia 1-azabicyclo(4.2.0)oct-2-en-3-yl) 2-propènyl) quinoléinium**

On opère comme au stade I de l'exemple 1 à partir de 339 mg du produit obtenu au stade H ci-dessus en utilisant 5 $cm^3$ d'une solution d'acide trifluoroacétique à 10 % d'anisole. On obtient 180 mg du produit recherché.
Spectre RMN : DMSO 300 MHz
5,15 (d dédoublé) : $H_6$ céphalo ; 5,75 (dd) ~ 5,81-5,95 : $H_7$
céphalo, =CH-$C\underline{H}_2N$ ≡, Ar-C$\underline{H}$(-O-)$CO_2$ ; 6,38 (m) : CH=C$\underline{H}$-$CH_2$(ΔE) ; 6,99 (d dédoublé J = 16) : C$\underline{H}$=CH-$CH_2$(ΔE) ; 6,59 (d dédoublé J = 10) : $H_5$ fluorophényle ; 6,75-6,81 : $H_6$ et $H_5$ thiazole ; 7,35 (large) : $NH_2$ et/ou $C_6H_5$ ; 8,07 (t,1H) 8,26 (m,2H), 8,52 (d,1H) - 8,56 (dd,1H) - 9,30 (d,1H) 9,58 (sl,1H) : quinoléïne ; ~ 10,50 : OH ; 9,46 (d) : CON$\underline{H}$.

**EXEMPLE 17 : sel interne de (6R(3(E), 6alpha, 7béta (Z))) 7-(3-(7-(((2-amino 4-thiazolyl) ((carboxy (2,3-dihydroxy 4-fluorophényl) méthoxy) imino) acétyl) amino) 2-carboxy 8-oxo 5-thia 1-azabicyclo(4.2.0)oct-2-en-3-yl) 2-propènyl) thiéno(2,3-b) pyridinium**

**Stade A : Iodure de 1-[3-[7béta-[[[[[1-[4-fluoro 2,3bis[(2-méthoxy éthoxy) méthoxy] phényl] 2-oxo 2-(diphényl méthoxy) éthyl] oxy] imino] [2-(triphénylméthyl) amino] thiazol-4-yl] acétyl amino] 2-[(4-méthoxy benzyloxy carbonyl] 8-oxo 5-thia 1-azabicyclo[4,2,0] [oct-2-en-3-yl] 2-propényl thiéno (2,3-b) pyridinium.**

En opérant comme au stade H de l'exemple 16 à partir de 944 mg du produit obtenu au stade G de l'exemple 16 et en utilisant 0,45 g de thiéno[2,3-b] pyridine on obtient, après chromatographie sur silice (éluant chlorure de méthylène-méthanol (95-5)) 339 mg du produit recherché.
Spectre RMN : $CDCl_3$ 300 MHz
3,20 (s)-3,27 (s)-3,34 (s)-3,35 (s) : $OCH_3$ ; 3,40-3,60 et 3,77-4,00 : $OCH_2CH_2O$, $CH_2S$ ; 3,79 (s)-3,80 (s) : $ArOCH_3$ ; 4,92 (d,J = 5)-4,99 (d,J = 5) : $H_6$ céphalo ; ~ 5,18-5,30 : $OCH_2O$, $CO_2CH_2Ar$ ; ~ 5,77 (m) : $H_7$ céphalo ; 5,68 (m)-5,96 (m) : $=CH-CH_2\overset{+}{N}\equiv$ ; 6,39 (s)-6,45 (s) : $ArC\underline{H}(-O-)CO_2-$ ; 6,73 (s)-6,74 (s) : $H_5$ thiazole ; - 6,77 (t, dédoublé) : $H_5$ fluorophényl ; 6,90 à 7,40 : H benzéniques, $CO_2C\underline{H}(C_6H_5)_2$, $C\underline{H}=CH-CH_2(\Delta E)$ ; 6,92 (m) : $CH=C\underline{H}-CH_2(\Delta E)$ ; 7,54 (d)-7,66 (d) : $H_3'$ et 7,83-7,87 : $H_2'$ et 8,06 (m) : $H_5'$ et 8,80 (d) : $H_4'$ et 10,05 (m) : $H_6'$ et 8,23 (d)-8,34 (d) : CONH du thiéno[2,3-b]pyridine.

**Stade B : sel interne de (6R(3(E), 6alpha, 7béta (Z))) 7-(3-(7-(((2-amino 4-thiazolyl) ((carboxy (2,3-dihydroxy 4-fluorophényl) méthoxy) imino) acétyl) amino) 2-carboxy 8-oxo 5-thia 1-azabicyclo(4.2.0)oct-2-en-3-yl) 2-propènyl) thiéno(2,3-b) pyridinium**

En opérant comme au stade I de l'exemple 16, à partir de 332 mg de produit obtenu ci-dessus, on obtient 164 mg du produit recherché.
Spectre RMN DMSO 300 MHz
5,18 (d) dédoublé : $H_6$ céphalo ; 5,67 (d) $=CH-C\underline{H}_2-\overset{+}{N}\equiv$ ; 5,77-5,90 : $H_7$ céphalo, $Ar-C\underline{H}(-O-)CO_2-$ ; 6,31 (m) : $CH=C\underline{H}-CH_2(\Delta E)$ ; 7,14 (d,J = 15) : $C\underline{H}=CH-CH_2(\Delta E)$ ; ~ 6,80 (m) : $H_5$ thiazole, $H_5$ fluorophényle ; 7,36 (large) : $NH_2$ : 6,59 (t, J = 9) : $H_6$ ; 7,89 (d,J = 6)-8,15 (dd)-8,28 (d,J = 6)-9,08 (d)-9,23 (d) : $H_3'$-$H_5'$-$H_2'$-$H_4'$-$H_6'$ du thiéno[2,3-b]pyridine.

**EXEMPLE 18 : sel interne de (6R(3(E), 6alpha, 7béta (Z))) 1-(3-(7-(((2-amino 4-thiazolyl) ((carboxy (2,3-dihydroxy 4-fluorophényl) méthoxy) imino) acétyl) amino) 2-carboxy 8-oxo 5-thia 1-azabicyclo(4.2.0)oct-2-èn-3-yl) 2-propènyl) 4-(méthylthio) pyridinium**

**Stade A : Iodure de 1-[3-[7béta-[[[[[1-[4-fluoro 2,3bis[(2-méthoxy éthoxy) méthoxy] phényl] 2-oxo 2-(diphényl méthoxy) éthyl] oxy] imino] [2-(triphénylméthyl) amino] thiazol-4-yl] acétyl amino] 2-[(4-méthoxy benzyloxy carbonyl] 8-oxo 5-thia 1-azabicyclo[4,2,0] [oct-2-èn-3-yl] 2-propényl 4-(méthylthio) pyridinium**

En opérant comme au stade H de l'exemple 16, à partir de 1,052 g de produit obtenu au stade G de l'exemple 16 et en utilisant 462 mg de 4-thiométhyl pyridine, on obtient, après chromatographie sur silice (éluant chlorure de méthylène-méthanol (95-5)) 536 mg du produit recherché.
Spectre RMN : $CDCl_3$ 400 MHz
2,61 (s) : $CH_3S$ ; 3,21 (s)-3,26 (s)-3,34 (s)-3,35 (s) : $OCH_3$ ; 3,79 (s)-3,88 (s) : $CH_3OAr$ ; ~ 3,43 (m)-3,54 (m)-3,80-4,00 : $CH_2S$, $OCH_2CH_2O$ ; 4,92 (d) : $H_6$ céphalo ; 5,70 (dd)-5,77 (dd) $H_7$ céphalo ; 5,33 (m)-5,54 (m) : $CH_2\overset{+}{N}\equiv$ ; 5,17-5,30 : $OCH_2O$, $CO_2CH_2Ar$ ; 6,33 (m) : $CH=C\underline{H}-CH_2(\Delta E)$ ; 6,35 (s) : $ArC\underline{H}(-O-)CO_2-$ ; 6,74 (s)-6,75 (s) : $H_5$ thiazole ; 6,72 à 7,18 : $H_5$, $H_6$ fluorophényle, $CO_2C\underline{H}C_6H_5$, 4H aromatique, $C\underline{H}=CH-CH_2(\Delta E)$ ; 7,20 à 7,38 : H aromatique $C-(C_6H_5)_3$, $(C_6H_5)_2-CH-CO_2$ ; 7,61 (d,2H)-8,8 (m,2H) : $H_3'$, $H_5'$ et $H_2'$, $H_6'$ du pyridinium ; 7,83 (d), 8,15 (d) : -CONH.

**Stade B : sel interne de (6R(3(E), 6alpha, 7béta (Z))) 1-(3-(7-(((2-amino 4-thiazolyl) ((carboxy (2,3-dihydroxy 4-fluoro- phényl) méthoxy) imino) acétyl) amino) 2-carboxy 8-oxo 5-thia 1-azabicyclo(4.2.0)oct-2-èn-3-yl) 2-propènyl) 4-(méthylthio) pyridinium**

En opérant comme au stade I de l'exemple 16 à partir de 500 mg du produit obtenu au stade A ci-dessus, on obtient 251 mg du produit recherché.
Spectre RMN : DMSO 300 MHz
3,50-3,70 : $CH_2S$ ; 2,72 (s) : $CH_3S$ ; 5,18 (m) : $H_6$ céphalo ;
~ 5,77 (m) : $H_7$ céphalo ; 5,24 (m) : $=CH-C\underline{H}_2-\overset{+}{N}\equiv$ ; 5,83 (s), 5,87 (s) : $OCH_2O$, $ArCO_2CH_2$ ; 6,28 (m) : $CH=C\underline{H}-CH_2$

($\Delta$E) ; 6,98 (d,J = 16) : C<u>H</u>=CH-$CH_2$ ; 6,61 (t, dédoublé) : $H_6$ fluoro-phényle ; 6,77-6,82 : $H_5$ et $H_6$ thiazole ; 7,32 : $NH_2$ ; 7,96 (d)-8,71 (d) : $H_3$'-$H_5$' et $H_2$'-$H_6$' du pyridinium.

**EXEMPLE 19 : sel interne de (6R(3(E), 6alpha, 7béta (Z))) 2-(3-(7-(((2-amino 4-thiazolyl) ((carboxy (2,3-dihydroxy 4-fluorophényl) méthoxy) imino) acétyl) amino) 2-carboxy 8-oxo 5-thia 1-azabicyclo(4.2.0)oct-2-èn-3-yl) 2-propènyl) isoquinoléinium**

**Stade A : Iodure de 1-[3-[7béta-[[[[[1-[4-fluoro 2,3bis[(2-méthoxy éthoxy) méthoxy] phényl] 2-oxo 2-(diphényl méthoxy) éthyl] oxy] imino] [2-(triphénylméthyl) amino] thiazol-4-yl] acétyl amino] 2-[(4-méthoxy benzyloxy carbonyl] 8-oxo 5-thia 1-azabicyclo[4,2,0] [oct-2-en-3-yl] 2-propényl isoquinoléinium**

En opérant comme au stade H de l'exemple 16, à partir de 917 mg du produit obtenu au stade G de l'exemple 16 et en utilisant 416 mg d'isoquinoléine, on obtient, après chromatographie sur silice (éluant : chlorure de méthylène-méthanol (96-4)) 473 mg du produit recherché.
<u>Spectre RMN</u> : $CDCl_3$ 300 MHz
3,20 (s)-3,26 (s)-3,34 (s)-3,35 (s) : $CH_3O$ ; 3,76 (s)-3,78 (s) : $ArOCH_3$ ; 3,40-4,00 : $OCH_2$-$CH_2O$, $CH_2S$ ; $H_6$ céphalo ; 5,80 (m) : $H_7$ céphalo ; ~ 5,18-5,30 : $OCH_2O$, $ArCO_2C\underline{H_2}$ ; ~ 6,48 (m) : $CH_2$-C<u>H</u>=($\Delta$E) ; 6,38 (s)-6,44 (s) : Ar-C<u>H</u>(-O-)$CO_2$- ; 6,73 (s)-6,75 (s) : $H_5$ thiazole ; - 6,76 à 7,40 : H aromatiques (groupements phényle), $CO_2C\underline{H}(C_6H_5)_2$ ; 7,96 (m, 1H)-8,10 (m,2H)-8,26 (m,2H)-8,49 (m,1H)-8,69 (m,1H)-10,90 (s dédoublé, 1H) : de l'isoquinoléine.

**Stade B : sel interne de (6R(3(E), 6alpha, 7béta (Z))) 2-(3-(7-(((2-amino 4-thiazolyl) ((carboxy (2,3-dihydroxy 4-fluorophényl) méthoxy) imino) acétyl) amino) 2-carboxy 8-oxo 5-thia 1-azabicyclo(4.2.0)oct-2-èn-3-yl) 2-propènyl) isoquinoléinium**

En opérant comme au stade I de l'exemple 16 à partir de 460 mg du produit obtenu ci-dessus, on obtient 176 mg du produit attendu.
<u>Spectre RMN</u> : DMSO 400 MHz
3,72 (m) : $CH_2S$ ; 5,18 (d, dédoublé) : $H_6$ céphalo ; 5,77 (dd, d après échange)- $H_7$ céphalo ; 5,52 (m) : $CH_2$-N ≡ ; 5,83 (s), 5,87 (s) : Ar-$C\underline{H_2}$(-O-)$CO_2$- ; 6,39 (m) : CH=C<u>H</u>-$CH_2$ ($\Delta$E) ; 7,10 (d,J = 10) : C<u>H</u>=CH-$CH_2$($\Delta$E) ; 6,59 (m), 6,80 (m) : $H_6$, $H_5$ fluorophényle ; 6,77 (s)-6,80 (s) : $H_5$ thiazole ; 8,09 (t)-8,28 (t) et 8,37 (d)-8,53 (d)-8,61 (d)-8,74 (m)-10,06 (s) : $H_6$'-$H_7$' et $H_3$'-$H_4$' et $H_5$' et $H_8$' et $H_1$' de l'isoquinoléine.

**EXEMPLE 20 : sel interne de (6R(3(E), 6alpha, 7béta (Z))) 1-(3-(7-(((2-amino 4-thiazolyl) ((carboxy (2,3-dihydroxy 4-fluorophényl) méthoxy) imino) acétyl) amino) 2-carboxy 8-oxo 5-thia 1-azabicyclo(4.2.0)oct-2-èn-3-yl) 2-propènyl) imidazo(1,2-a) pyridinium**

**Stade A : Iodure de 1-[3-[7béta-[[[[[1-[4-fluoro 2,3bis[(2-méthoxy éthoxy) méthoxy] phényl] 2-oxo 2-(diphényl méthoxy) éthyl] oxy] imino] [2-(triphénylméthyl) amino] thiazol-4-yl] acétyl amino] 2-[(4-méthoxy benzyloxy carbonyl] 8-oxo 5-thia 1-azabicyclo[4,2,0] [oct-2-en-3-yl] 2-propényl imidazo (1,2-a) pyridinium**

En opérant comme au stade H de l'exemple 16, à partir de 955 mg du produit obtenu au stade G de l'exemple 16, en utilisant 401 mg d'imidazo[1,2-a] pyridine, on obtient (après chromatographie sur silice, éluant chlorure de méthylène-méthanol (95-5)) 486 mg du produit attendu.
<u>Spectre RMN</u> : $CDCl_3$ 300 MHz
3,20 (s)-3,26 (s)-3,34 (s)-3,35 (s) : $CH_3O$ ; 3,40-3,95 : $OCH_2$-$CH_2O$, $CH_2S$ ; 4,89 (d) : $H_6$ céphalo ; 5,74 (m) : $H_7$ céphalo ; 5,20-5,42 (9H, excès) : $ArCO_2C\underline{H_2}$-$OCH_2O$, CH-$C\underline{H_2}$-N ≡ ; 6,28 (m) : =C<u>H</u>-$CH_2$($\Delta$E) ; 6,38 (s)-6,44 (s) : Ar-C<u>H</u>(-O-)$CO_2$- ; 6,88 (s)-6,90 (s) : $CO_2C\underline{H}(C_6H_5)_2$ ; ~ 6,75 à 7,35 : H aromatiques (groupements phényle) + 1H bicycle azoté ; 7,85 (m,1H)-8,03 (d,1H)-8,36 (d,1H)-9,09 (m,1H) ; autres H bicycle azoté ; 7,94 (d), 8,25 (d) : CONH.

**Stade B : sel interne de (6R(3(E), 6alpha, 7béta (Z))) 1-(3-(7-(((2-amino 4-thiazolyl) ((carboxy (2,3-dihydroxy 4-fluorophényl) méthoxy) imino) acétyl) amino) 2-carboxy 8-oxo 5-thia 1-azabicyclo(4.2.0)oct-2-èn-3-yl) 2-propènyl) imidazo(1,2-a) pyridinium**

En opérant comme au stade I de l'exemple 16 à partir de 476 mg du produit obtenu au stade A ci-dessus, on obtient 215 mg du produit recherché.
<u>Spectre RMN</u> : DMSO 400 MHz
3,70 (m) : $CH_2S$ ; 5,15 (d, dédoublé) : $H_6$ céphalo ; 5,74 (dd, d après échange)-5,82 (dd, d après échange) : $H_7$ céphalo ; 5,28 (m) : $CH_2$-N ≡ ; 5,83 (s), 5,86 (s) : Ar-C<u>H</u>(-O-)$CO_2$- ; 6,25 (m) : CH=C<u>H</u>-$CH_2$($\Delta$E) ; 6,89 (d, dédoublé, J = 15,5) :

C<u>H</u>=CH-$CH_2$(ΔE) ; 6,60 (m), 6,78 (m) : $H_6$, $H_5$ fluorophényle ; 6,76 (s)-6,80 (s) : $H_5$ thiazole ; 7,57 (m)-8,05 (m)-8,19 (m) et 8,28 (d,dédoublé, J = 2,5)-8,44 (d,dédoublé, J = 2,5)-8,96 (d, J = 6,5) : $H_5$'-$H_6$'-$H_7$'-$H_4$' et $H_2$', $H_3$' de l'imidazo (1,2-a) pyridinium ; 9,44 (d)-9,56 (d) : CON<u>H</u>-C ; 7,32 (m)-9,40 (m) : H mobiles.

**<u>EXEMPLE 21</u> : Sel interne de (6R-(3-(E) 6alpha, 7béta-(Z))) 1-(3-(7-(((2-amino 4-thiazolyl) ((carboxy (2,3-dihydroxy phényl) méthoxy) imino) acétyl) amino) 2-carboxy 8-oxo 5-thia 1-azabicyclo[4,2,0]oct-2-èn-3-yl) 2-propényl) 4-(méthylthio) pyridinium**

**Stade A : [(2,3-dihydroxy) phényl] hydroxy acétate d'éthyle**

A une solution refroidie à -20°C de 1,1 g de pyrocatéchol dans 20 $cm^3$ de chlorure de méthylène, on ajoute 10 $cm^3$ d'une solution molaire de chlorure de titane dans le chlorure de méthylène. On agite pendant 30 minutes à -20°C puis ajoute en 5 minutes une solution de 1,02 g de glyoxylate d'éthyle dans 10 $cm^3$ de chlorure de méthylène. On agite 2 heures à -20°C. On laisse le milieu revenir à température ambiante, le verse sur 50 $cm^3$ d'une solution saturée de chlorure d'ammonium, extrait avec du chlorure de méthylène, lave, sèche et évapore à sec sous pression réduite. On chromatographie le résidu sur silice (éluant : chlorure de méthylène-acétate d'éthyle (7-3)), on obtient ainsi 860 mg du produit recherché (F = 86°C).
<u>Spectre IR</u> : $CHCl_3$
3595 $cm^{-1}$, 3040 $cm^{-1}$ OH complexe + associé, 1730 $cm^{-1}$ = C=O, 1602-1402 $cm^{-1}$ : aromatique.
<u>Spectre RMN</u> : $CDCl_3$ 250 MHz ppm
5,33 (s) : C-C<u>H</u>(OH)COOEt ; 1,26 (t)-4,27 (m) : COOEt ; 3,75-5,86-7,45 : H mobile ; 6,7 à 6,95 : H aromatiques.

**Stade B : [2,3bis-[(2-méthoxy éthoxy) méthoxy] phényl] hydroxy acétate d'éthyle**

A une solution de 8,1 g du produit obtenu au stade A dans 750 $cm^3$ d'acétonitrile, on ajoute 15,69 g de chlorure de méthoxy éthoxy méthyle et 16,35 g de N-éthyle diisopropylamine, on agite 16 heures à 0°C, puis verse sur 100 $cm^3$ d'eau et évapore l'acétonitrile. On reprend avec du chlorure de méthylène, lave avec de l'acide chlorhydrique 1N, à l'eau puis avec une solution de carbonate de sodium à 10 %. On sèche et évapore à sec sous pression réduite, on recueille 12,3 g de produit que l'on chromatographie sur silice (éluant : cyclohexane-acétate d'éthyle (5-5)), on obtient ainsi 6,7 g du produit recherché.
<u>Spectre IR</u> : $CHCl_3$
3315-3440 $cm^{-1}$ : OH complexe ; 1735 $cm^{-1}$ : C=O ; 1601-1590 $cm^{-1}$ : aromatiques.
<u>Spectre RMN</u> : $CDCl_3$ 250 MHz
1,21 et 4,22 : $CO_2Et$ ; 3,37 : $OCH_3$ ; 3,57-3,83-3,93 : $CH_2$-$CH_2$-O ; 5,25-5,28 : O-$CH_2$-O ; 4,07 : OH ; 5,39 : C-CH(OH)-$CO_2Et$.

**Stade C : [2,3bis-[(2-méthoxy éthoxy) méthoxy] phényl] hydroxy acétate de diphényle méthyle**

a) <u>Saponification</u> :

A une solution de 8,54 g du produit obtenu au stade précédent dans 400 $cm^3$ d'éthanol, on ajoute 22 $cm^3$ de soude 2N. On agite pendant 3 heures à température ambiante. On acidifie à pH 2 avec de l'acide chlorhydrique N. On concentre à 1/2 volume sous pression réduite, dilue avec 200 $cm^3$ d'eau et extrait avec du chlorure de méthylène et de l'acétate d'éthyle. On sèche et évapore à sec sous pression réduite. On obtient 8,2 g d'acide [2,3bis-[(2-méthoxy éthoxy) méthoxy] phényl] hydroxy acétique.

b) <u>Estérification</u>

A une solution de 8,2 g de l'acide obtenu ci-dessus dans 300 $cm^3$ de chlorure de méthylène refroidie à 0°C, on ajoute 109 $cm^3$ de diphényldiazométhane en solution 0,3 M dans l'éther éthylique. On agite 16 heures à température ambiante. On ajoute à 0°C de l'acide acétique puis évapore à sec sous pression réduite, on recueille 13 g de produit que l'on chromatographie sur silice, éluant cyclohexane-acétate d'éthyle (6-4), on obtient ainsi 10,2 g de produit recherché.
<u>Spectre IR</u> : $CHCl_3$
3530-3520 $cm^{-1}$ : OH complexe ; 1742 $cm^{-1}$ : C=O ; 1600-1585-1495 $cm^{-1}$ : aromatiques.
<u>Spectre RMN</u> : $CDCl_3$ 300 MHz
3,34-3,37 : $CH_3$ ; 3,54-3,83 : O-$CH_2$-$CH_2$ ; 4,08 : OH ; 5,18-5,28 : $OCH_2O$ ; 5,53 : C-C<u>H</u>(OH)C-$(C_6H_5)_2$.

**Stade D : [2,3bis-[(2-méthoxy éthoxy) méthoxy] phényl] phtalimidoxy acétate de diphényle méthyle**

A une solution de 227 mg du produit obtenu au stade précédent dans 25 $cm^3$ de tétrahydrofuranne, on ajoute à température ambiante, 226 mg de triphényl phosphine et 77 mg de N-hydroxy phtalimide. On refroidit à -5°C et ajoute en 2 heures 136 µl (150 mg) de diéthylazodicarboxylate. On agite 2 heures en laissant la température remonter à l'ambiance. On verse dans 25 $cm^3$ d'eau glacée, extrait avec du chlorure de méthylène puis de l'acétate d'éthyle, sèche et évapore à sec sous pression réduite, on recueille 760 mg de résidu que l'on chromatographie sur silice (éluant : chlorure de méthylène-acétone (97-3)), on obtient ainsi 230 mg du produit recherché.
Spectre IR : $CHCl_3$
1794-1738 $cm^{-1}$ : C=O ; 1602-1588-1488 $cm^{-1}$ : aromatiques.
Spectre RMN : $CDCl_3$ 300 MHz
3,31-3,36 : $CH_3$ ; 3,46-3,52-3,79-3,89 : $CH_2$-$CH_2$-O ; 5,24 : O-$CH_2$-O ; 6,43 : C-C$\underline{H}$(ON=)$CO_2$C-$(C_6H_5)_2$ ; 6,95 : C$\underline{H}$-$(C_6H_5)_2$ ; 7,72 : Ar-$\underline{H}$ phtalimido ; 7,0-7,35 : Ar-H aromatique.

**Stade E : aminoxy [2,3bis-[(2-méthoxy éthoxy) méthoxy] phényl] acétate de diphényle méthyle**

A une solution de 6,2 g du produit obtenu au stade précédent dans 400 $cm^3$ d'éthanol et 20 $cm^3$ de chlorure de méthylène, on ajoute 1,42 $cm^3$ (1,47 g) d'hydrate d'hydrazine et agite pendant 3 heures à température ambiante. On évapore à sec sous pression réduite, reprend l'extrait sec dans du chlorure de méthylène, filtre l'insoluble et amène le filtrat à sec sous pression réduite, on obtient 5,0 g du produit recherché.
Spectre IR $CHCl_3$
3335 $cm^{-1}$ : $ONH_2$ ; 1745 $cm^{-1}$ : C=O ; aromatiques 1602, 1589, 1577, 1495 $cm^{-1}$ : aromatiques.
Spectre RMN : $CDCl_3$ 250 MHz
3,35-3,37 : $OCH_3$ ; 3,54-3,83-3,95 : O-$CH_2$-$CH_2$-O ; 5,26-5,29 : O-$CH_2$-O ; 5,75 : -C-C$\underline{H}$($ONH_2$)$CO_2$C-$(C_6H_5)_2$ ; 5,86 : O-$NH_2$ ; 6,95 : COO-C$\underline{H}$-$(C_6H_5)_2$.

**Stade F : Acide [[[1-[2,3bis[(méthoxy éthoxy) méthoxy] phényl] 2-oxo 2-(diphényl méthoxy) éthyl] oxy] imino] [2-[(triphényl méthyl) amino] thiazol 4-yl] acétique**

On agite pendant 6 heures 5,0 g du produit obtenu au stade précédent en solution dans 60 $cm^3$ de méthanol avec 4,016 g d'acide [2-[(triphényl méthyl) amino] thiazol 4-yl] acétique (préparé tel que décrit dans la demande de brevet belge n° 864828). On évapore à sec, chromatographie le résidu sur silice, éluant acétate d'éthyle-éthanol (7-3), on obtient ainsi : 5,55 g du produit recherché.
Spectre IR : $CHCl_3$
=C-NH : 3405 $cm^{-1}$ ; C=O : 1735 $cm^{-1}$ ; C=N, aromatique et
$CO_2^{\ominus}$ : 1619-1602-1529-1494 $cm^{-1}$
Spectre RMN : $CDCl_3$ 250 MHz
3,13-3,31 : $CH_3$ ; 3,34-3,49-3,76 : O-$CH_2$-$CH_2$-O ; 5,14-5,18 : O-$CH_2$-O ; 6,11 : -C-C$\underline{H}$(ON=)$CO_2$C- ; 6,46 : -$CO_2$-C$\underline{H}$ $(C_6H_5)_2$ ;
6,81 : C$\underline{H}$ thiazol ; 6,75 à 7,3 H aromatiques.

**Stade G : 7béta-[[[[[1-[2,3bis[(2-méthoxy éthoxy) méthoxy] phényl] 2-oxo 2-(diphényl méthoxy) éthyl] oxy] imino] [2-[(triphényl méthyl) amino] thiazol 4-yl] acétyl] amino] 3-[(Z) 3-chloro 1-propényl] 8-oxo 5-thia 1-azabicyclo[4,2,0] oct-2-èn-2-carboxylate de 4-méthoxy benzyle**

A une solution de 6,18 g du produit obtenu au stade F, dans 150 $cm^3$ de chlorure de méthylène et refroidie à 0°C, on ajoute 3,55 g de chlorhydrate de 7béta amino 3-[(Z) 3-chloro 1-propényl] 8-oxo 5-thia 1-azabicyclo[4,2,0] oct 2-èn-2-carboxylate de 4-méthoxy benzyle (préparé comme décrit dans la demande EP : 0333154) et 1,6 g de N-éthyl diméthylaminopropyl carbodiimide (EDAC) on agite 10 minutes à 0°C puis 2 heures en laissant la température remonter à 20°C, on verse sur une solution molaire d'hydrogénophosphate de potassium. On extrait avec du chlorure de méthylène puis avec de l'acétate d'éthyle. On sèche et évapore à sec. On chromatographie sur silice en éluant avec chlorure de méthylène-acétate d'éthyle (9-1). On obtient 5,1 g du produit recherché.
Spectre IR : $CHCl_3$
3404 $cm^{-1}$ : =C-NH ; 1790-1731-1684 $cm^{-1}$ : C=O ; aromatiques, hétéroatome, amide II : 1613-1587-1526-1517-1496 $cm^{-1}$.
Spectre RMN : $CDCl_3$ (300 MHz)
3,24 à 3,37 : $CH_2$-O-$CH_3$ et C-S-$CH_2$- ; 3,44 à 4,0 : $CH_2Cl$ et $C_6H_5OCH_3$ ; 3,44 à 4,0 : O-$CH_2$-$CH_2$- ; 5,15 à 5,28 : -O-$CH_2$-O ; 5,67 à 5,88, 6,29 : C-C$\underline{H}$-C$\underline{H}$-$CH_2Cl$ ; 6,77 : CH thiazol ; 6,7 à 7,4 : aromatiques ; 8,01-8,37 : NH.

**Stade H : 7béta-[[[[[1-[2,3bis[(2-méthoxy éthoxy) méthoxy] phényl] 2-oxo 2-(diphényl méthoxy) éthyl] oxy] imino] [2-[(triphényl méthyl) amino] thiazol 4-yl] acétyl] amino] 3-[(Z) 3-iodo 1-propényl] 8-oxo 5-thia 1-azabicyclo [4,2,0] oct-2-èn-2-carboxylate de 4-méthoxy benzyle**

A une solution de 1,25 g du produit obtenu au stade G (ci-dessus) dans 5 $cm^3$ d'acétone, on ajoute 0,57 g d'iodure de sodium et 1 cristal d'iode et on agite pendant 1 heure 30 à température ambiante. On évapore à sec sous pression réduite. On reprend l'extrait sec avec du chlorure de méthylène, lave avec une solution de thiosulfate de sodium à 10 %, puis avec de l'eau salée. On sèche puis évapore à sec sous pression réduite. On recueille 1,28 g de produit attendu que l'on utilise tel quel pour le stade suivant.

**Stade I : iodure de 1-[3-[7béta-[[[[[1-[2,3bis[(2-méthoxy éthoxy) méthoxy] phényl] 2-oxo 2-(diphényl méthoxy) éthyl] oxy] imino] [2-[(triphénylméthyl) amino] thiazol 4-yl] acétyl] amino] 2-[(4-méthoxy benzyloxy) carbonyl] 8-oxo 5-thia 1-azabicyclo[4,2,0] [oct-2-èn-3-yl] 2-propényl] 4-méthyl thio pyridinium**

On mélange 1,28 g du produit obtenu au stade H, 1,15 g de thiométhyl pyridine et 3 $cm^3$ de chlorure de méthylène. On évapore à sec sous pression réduite. On chromatographie sur silice (éluant chlorure de méthylène-méthanol (97-3) puis (96-4) et (95-5). On obtient ainsi 415 mg du produit recherché.
Spectre de RMN : $CDCl_3$ 250 MHz
2,6 : $S-CH_3$ ; 3,21-3,25-3,35-3,36-3,37 : $-OCH_3$ ; 3,4 à 3,9 : $-O-CH_2-CH_2-O$ ; 3,77-3,79 $C_6H_5-OCH_3$.

**Stade J : Sel interne de (6R-(3-(E) 6alpha, 7béta-(Z))) 1-(3-(7-(((2-amino 4-thiazolyl) ((carboxy (2,3-dihydroxy phényl) méthoxy) imino) acétyl) amino) 2-carboxy 8-oxo 5-thia 1-azabicyclo[4,2,0]oct-2-èn-3-yl) 2-propényl) 4-(méthylthio) pyridinium**

On agite 2 heures à température ambiante, 400 mg du produit obtenu au stade I, 4,5 $cm^3$ d'acide trifluoroacétique et 0,5 $cm^3$ d'anisole. On ajoute ensuite 0,5 $cm^3$ d'eau et agite 2 heures à température ambiante. On filtre, rince avec 2 fois 3 $cm^3$ d'acide trifluoroacétique et ajoute 15 $cm^3$ d'éther éthylique. On agite 15 minutes, essore et sèche sous pression réduite, on recueille 222 mg du produit recherché.
Spectre RMN : DMSO (300 MHz) ppm
2,71 : $SCH_3$ ; 3,72 : $C-S-CH_2-C$ ; 5,19-5,76-5,83 :
$- S-C\underline{H}-(-N=)-C\underline{H}-NH-$ ; 5,22 : $-C=C-CH_2-N^{\oplus}$ ; 5,91-5,95 : H iminocarboxybenzyle ; 6,28 : $-CH=C\underline{H}-CH_2-N^{\oplus}$ ; 6,98 : $-C\underline{H}=CH-CH_2-N^{\oplus}$ ; 6,59-6,79 : H aromatiques ; 6,79-6,83 : H thiazol ; 9,49 : NH.

**<u>EXEMPLE 22</u> : Sel interne de (6R-(3-(E) 6alpha, 7béta-(Z))) 7-(3-(7-(((2-amino 4-thiazolyl) ((carboxy (2,3-dihydroxy phényl) méthoxy) imino) acétyl) amino) 2-carboxy 8-oxo 5-thia 1-azabicyclo[4,2,0]oct-2-èn-3-yl) 2-propényl) thiéno(2,3-b) pyridinium**

**Stade A : iodure de 1-[3-[7béta-[[[[[1-[2,3bis[(2-méthoxy éthoxy) méthoxy] phényl] 2-oxo 2-(diphényl méthoxy) éthyl] oxy] imino] [2-[(triphénylméthyl) amino] thiazol 4-yl] acétyl] amino] 2-[(4-méthoxy benzyloxy) carbonyl] 8-oxo 5-thia 1-azabicyclo[4,2,0] [oct-2-èn-3-yl] 2-propényl] thiéno(2,3-b) pyridinium**

On opère comme au stade I de l'exemple 21 à partir de 1,24 g du produit obtenu comme au stade H de l'exemple 21 en utilisant 1,28 g de thiéno(2,3-b) pyridine, on obtient, après chromatographie sur silice, éluant chlorure de méthylène-méthanol (97-3 puis 96-4 et 95-5), 450 mg du produit recherché.
Spectre IR : $CHCl_3$
=C-NH : 3404 $cm^{-1}$ ; C=O : 1790-1731-1684 $cm^{-1}$ ; C=C, aromatiques, hétéroatomes, amide II : 1613-1587-1526-1517-1496 $cm^{-1}$ ; thiéno pyridine : 1599 $cm^{-1}$.
Spectre RMN : $CDCl_3$ 300 MHz
3,21-3,26-3,34-3,37 : $CH_2-O-CH_3$ ; 3,43-3,55-3,82 : $-O-CH_2-O-C\underline{H_2}-CH_2$ ; 3,80 : $C_6H_5O-CH_3$ ; 5,2 à 5,3 : $O-C\underline{H_2}-O-CH_2-$, $CO_2C\underline{H_2}C_6H_5-O-$ ; 5,90 : $-CH-CH_2-N^{\oplus}$ ; 6,45 à 6,6 : $C-C\underline{H}=CH-CH_2-N^{\oplus}$ ; 6,51 à 6,56 : $CO_2-C\underline{H}(C_6H_5)_2$ ; 6,73 : H thiazol ; 6,85 à 8,78 : H aromatiques.

**Stade B : Sel interne de (6R-(3-(E) 6alpha, 7béta-(Z))) 7-(3-(7-(((2-amino 4-thiazolyl) ((carboxy (2,3-dihydroxy phényl) méthoxy) imino) acétyl) amino) 2-carboxy 8-oxo 5-thia 1-azabicyclo[4,2,0]oct-2-èn,3-yl) 2-propényl) thiéno(2,3-b) pyridinium**

On opère comme au stade J de l'exemple 21 à partir de 430 mg du produit obtenu au stade précédent et obtient 206 mg du produit recherché.

Spectre IR : nujol
C=O : 1775 $cm^{-1}$ (β-lactame) 1670 $cm^{-1}$ (complexe) ; système conjugué, aromatique, $NH_2$, amide : 1599-1580-1520 $cm^{-1}$.
Spectre RMN : $CDCl_3$ 400 MHz
3,69 : -S-C$\underline{H}_2$-C- ; 5,18-5,78-5,86 : -NH-C$\underline{H}$-C$\underline{H}$-S- ; 5,67 : =CH-C$\underline{H}_2$-N⊕ ; 5,91-5,95 : -C-CH(O-N=)$CO_2$H ; 6,31 et 7,15 : les H du propylène ; 6,59 et 6,79 : H aromatiques ; 6,78 et 6,82 : H thiazol ; 7,89 à 9,22 : thiénopyridine ; 9,49 et 9,61 : amide.

**EXEMPLE 23 : Sel interne de (6R-(3-(E) 6alpha, 7béta-(Z))) 7-(3-(7-(((2-amino 4-thiazolyl) ((carboxy (2,3-dihydroxy phényl) méthoxy) imino) acétyl) amino) 2-carboxy 8-oxo 5-thia 1-azabicyclo[4,2,0]oct-2-èn-3-yl) 2-propényl) imidazo(1,2-a) pyridinium**

**Stade A : iodure de 1-[3-[7béta-[[[[[1-[2,3bis[(2-méthoxy éthoxy) méthoxy] phényl] 2-oxo 2-(diphényl méthoxy) éthyl] oxy] imino] [2-[(triphénylméthyl) amino] thiazol 4-yl] acétyl] amino] 2-[(4-méthoxy benzyloxy) carbonyl] 8-oxo 5-thia 1-azabicyclo[4,2,0] [oct-2-èn-3-yl] 2-propényl] imidazo(1,2-a) pyridinium**

On opère comme au stade I de l'exemple 21 à partir de 1,24 g du produit obtenu comme au stade H de l'exemple 21 et en utilisant 1,28 g d'imidazopyridine. On obtient ainsi 270 mg du produit attendu.
Spectre RMN $CDCl_3$ 400 MHz
3,25-3,20-3,35-3,37 : $CH_2OCH_3$ ; 3,30 à 3,90 : $OCH_2$-C$\underline{H}_2$-$OCH_3$ ; 3,30 à 3,90 : -S-C$\underline{H}_2$-C- ; 3,78-3,79 : -$C_6H_5$-$OCH_3$ ; 4,88 et 5,72 : NH-C$\underline{H}$-C$\underline{H}$-S ; 5,10 à 5,50 : C-O-C$\underline{H}_2$-$OCH_2$-, =C-C$\underline{H}_2$-N⊕ ; 6,28 : C-CH=C$\underline{H}$-$CH_2$- ; 6,51-6,56 : -C-C$\underline{H}$ (O-N=)$CO_2$CH ; 6,7 à 7,4 : H aromatique, H thiazol, H propylène ; 7,93-8,20 : NH.

**Stade B : Sel interne de (6R-(3-(E) 6alpha, 7béta-(Z))) 7-(3-(7-(((2-amino 4-thiazolyl) ((carboxy (2,3-dihydroxy phényl) méthoxy) imino) acétyl) amino) 2-carboxy 8-oxo 5-thia 1-azabicyclo[4,2,0]oct-2-èn-3-yl) 2-propényl) imidazo(1,2-a) pyridinium**

On opère comme au stade J de l'exemple 21, à partir de 280 mg de produit obtenu comme au stade précédent. On obtient 140 mg de produit recherché.
Spectre IR :
C=O : 1775-1670 $cm^{-1}$ ; système conjugué, aromatique, amide II : 1598-1530-1510 $cm^{-1}$.
Spectre RMN : DMSO 300 MHz
3,45 à 4,20 : -S-$CH_2$- ; 5,16-5,76-5,82 : -NH-C$\underline{H}$-C$\underline{H}$-S- ; 5,29 : -$CH_2$-N⊕- ; 5,91-5,94 : C-C$\underline{H}$(-O-N=)$CO_2$H ; 6,26 : 1H propylène ; 6,58-6,70 à 6,95 : H thiazol, aromatique, 1H propylène ; 9,47-9,58 : NH ; 7,58-8,06-8,20-8,96-8,29-8,44 : bicycle.

**EXEMPLE 24 : Sel interne de (6R-(3-(E) 6alpha, 7béta-(Z))) 7-(3-(7-(((2-amino 4-thiazolyl) ((carboxy (2,3-dihydroxy phényl) méthoxy) imino) acétyl) amino) 2-carboxy 8-oxo 5-thia 1-azabicyclo[4,2,0]oct-2-èn-3-yl) 2-propényl) quinoléinium**

**Stade A : iodure de 1-[3-[7béta-[[[[[1-[2,3bis[(2-méthoxy éthoxy) méthoxy] phényl] 2-oxo 2-(diphényl méthoxy) éthyl] oxy] imino] [2-[(triphénylméthyl) amino] thiazol 4-yl] acétyl] amino] 2-[(4-méthoxy benzyloxy) carbonyl] 8-oxo 5-thia 1-azabicyclo[4,2,0] [oct-2-èn-3-yl] 2-propényl] quinoléinium**

On opère comme au stade I de l'exemple 21, à partir de 1,33 g du produit obtenu comme au stade H de l'exemple 21, en utilisant 1,2 g de quinoléine, on obtient, après chromatographie sur silice (éluant chlorure de méthylène-méthanol (97-3 puis 95-5)) 475 mg du produit recherché.
Spectre RMN :
3,42 à 3,95 : $OCH_2$-$CH_2$-$OCH_3$ ; 3,78-3,79 : -$C_6H_4$-$OCH_3$ ; 5,13 à 5,28 : -OC$\underline{H}_2$-O-$CH_2$- , =C-C$\underline{H}$-(O-N=) ; 5,96-6,11 : -$CH_2$-N⊕ ; 6,49-6,55 : -$CO_2$-C$\underline{H}$-$(C_6H_5)_2$ ; 6,85-8,93 : aromatiques.

**Stade B : Sel interne de (6R-(3-(E) 6alpha, 7béta-(Z))) 7-(3-(7-(((2-amino 4-thiazolyl) ((carboxy (2,3-dihydroxy phényl) méthoxy) imino) acétyl) amino) 2-carboxy 8-oxo 5-thia 1-azabicyclo[4,2,0]oct-2-èn-3-yl) 2-propényl) quinoléinium**

On opère comme au stade J de l'exemple 21, à partir de 465 mg du produit obtenu ci-dessus. On obtient 255 mg du produit recherché.
Spectre RMN : DMSO 300 MHz

3,3 à 3,8 : -S-$CH_2$- ; 5,15 et 5,78 : -NH-C<u>H</u>-C<u>H</u>-S- ; 5,91 : -C-C<u>H</u>(O-N=)$CO_2$H et $CH_2$-$N^{\oplus}$- ; 6,39 : ≡C-CH=C<u>H</u>- ; 6,99 : ≡C-C<u>H</u>-CH- ; 6,7 à 6,9 : H aromatiques ; 8,07 à 9,58 : H bicycle.

**<u>EXEMPLE 25</u> : Préparation du 7béta [[[[[1-[2,5-dichloro 3,4bis-[(2-méthoxy éthoxy) méthoxy] phényl] 2-oxo 2-(diphénylméthoxy) éthyl] oxy] imino] [2-[(triphénylméthyl) amino] thiazol 4-yl] acétyl] amino] 3-[(Z) 3-iodo 1-propényl] 8-oxo 5-thia 1-azabicyclo[4,2,0]oct-2-èn-2-carboxylate de 4-méthoxy benzyle.**

**Stade A : Acide [[[1-[2,5-dichloro 3,4bis-[(2-méthoxy éthoxy) méthoxy] phényl] 2-oxo 2-(diphényl méthoxy) éthyl] oxy] imino] [2-[(triphénylméthyl) amino] thiazol 4-yl] acétique**

On opère comme au stade E de l'exemple 1 à partir de 3,5 g d'aminoxy [2,5-dichloro 3,4bis-[(2-méthoxy éthoxy) méthoxy] phényl] acétate de diphényle méthyle (obtenu au stade E de l'exemple 9) avec 2,37 g d'acide oxo [2-[(triphényl méthyl) amino] thiazol 4-yl] acétique (décrit dans la demande de brevet belge n° 864828). On obtient, après chroma-tographie sur silice (éluant acétate d'éthyle-éthanol (9-1)) 5,14 g du produit recherché.
<u>Spectre RMN</u> : 300 MHz $CDCl_3$
3,30 (s) : $OCH_3$ ; 3,51-3,92 (m) : -O-C<u>$H_2$</u>-C<u>$H_2$</u>-$OCH_3$ ; 5,16 (s)-5,18 (s) : O-$CH_2$-O ; 6,25 : C<u>H</u>-CO-CH-$\Phi_2$, $H_6$ ; 7,20 à 7,30 : H aromatiques.

**Stade B : 7béta [[[[[1-[2,5-dichloro 3,4bis-[(2-méthoxy éthoxy) méthoxy] phényl] 2-oxo 2-(diphényl méthoxy) éthyl] oxy] imino] [2-[(triphénylméthyl) amino] thiazol 4-yl] acétyl] amino] 3-[(Z) 3-chloro 1-propényl] 8-oxo 5-thia 1-azabicyclo[4,2,0]oct-2-èn-2-carboxylate de 4-méthoxy benzyle**

On opère comme au stade F de l'exemple 1 à partir de 4,8 g du produit obtenu ci-dessus avec 2,26 g de chlorhydrate de 7béta-amino 3-[(Z) 3-chloro 1-propényl] 8-oxo 5-thia 1-azabicyclo[4,2,0]oct-2-èn-2-carboxylate de 4-méthoxy benzyle (décrit dans EP 0333154). Après chromatographie sur silice (éluant chlorure de méthylène-éther éthylique (9-1)), on obtient 4,6 g du produit recherché.
<u>Spectre RMN</u> : 300 MHz $CDCl_3$
3,05 (m)-3,45 (m) : -$CH_2$-Cl ; 3,36-3,37 (s) : -O-$CH_3$ ; 3,57 (m)-3,97 (m) : O-C<u>$H_2$</u>-C<u>$H_2$</u>-O- ; 3,81 (s,d) : -Φ-OC<u>$H_3$</u> ; 4,99-5,02 (d) : NH-CH-C<u>H</u>-S ; 5,86-5,90 : NH-C<u>H</u>-CH-S ; 5,15 à 5,26 : O-C<u>$H_2$</u>-O- ; 5,75 (m) : $H_2$ propylène ; 6,26 (J=11,5) (d)-6,35 (J=11,5) (d) : $H_1$ propylène ; 6,47 à 6,50 (s) -$CO_2$-C<u>H</u>-$\Phi_2$ ; 6,85 à 7,40 les H aromatiques.

**Stade C : 7béta [[[[[1-[2,5-dichloro 3,4bis-[(2-méthoxy éthoxy) méthoxy] phényl] 2-oxo 2-(diphényl méthoxy) éthyl] oxy] imino] [2-[(triphénylméthyl) amino] thiazol 4-yl] acétyl] amino] 3-[(Z) 3-iodo 1-propényl] 8-oxo 5-thia 1-azabicyclo[4,2,0]oct-2-èn-2-carboxylate de 4-méthoxy benzyle**

On opère comme au stade G de l'exemple 1 à partir de 150 mg du produit obtenu au stade ci-dessus, on obtient 160 mg du produit recherché.

**<u>EXEMPLE 26</u> : Sel interne de (6R-(3-(E) 6alpha, 7béta-(Z))) 1-(3-(7-(((2-amino 4-thiazolyl) ((carboxy (2,5-dichloro 3,4-dihydroxy phényl) méthoxy) imino) acétyl) amino) 2-carboxy 8-oxo 5-thia 1-azabicyclo[4,2,0]oct-2-èn-3-yl) 2-propényl) imidazo(1,2-a) pyridinium**

**Stade A : iodure de 1-[3-[7béta-[[[[[1-[2,5-dichloro 3,4bis-[(2-méthoxy éthoxy) méthoxy] phényl] 2-oxo 2-(diphényl méthoxy) éthyl] oxy] imino] [2-[(triphénylméthyl) amino] thiazol 4-yl] acétyl] amino] 2-[(4-méthoxy benzyloxy) carbonyl] 8-oxo 5-thia 1-azabicyclo[4,2,0] oct-2-èn-3-yl] 2-propényl] imidazo(1,2-a) pyridinium**

On opère comme au stade H de l'exemple 1, à partir de 1,24 g de produit obtenu comme au stade C de l'exemple 25 en utilisant 0,497 g d'imidazo(1,2-a) pyridine. On obtient, après chromatographie sur silice (éluant chlorure de méthylène-méthanol (95-5)), 626 mg du produit recherché.
<u>Spectre RMN</u> : $CDCl_3$ 400 MHz
3,34-3,36 : -$CH_2$-O-C<u>$H_3$</u> ; 3,50-3,87 : O-C<u>$H_2$</u>-C<u>$H_2$</u>-OMe ; 3,80 (s) -Φ-OC<u>$H_3$</u> ; 4,94 (d,d) : -NH-CH-C<u>H</u>-S- ; 5,20-5,24 : -O-C<u>$H_2$</u>-O-, $CO_2$-C<u>$H_2$</u>-Φ- ; 5,86 : NH-C<u>H</u>-CH-S ; 6,15-6,35 : H propylène ; 6,47-6,51 : =N-O-C<u>H</u>- ; 6,77 : H thiazole ; 6,90 à 7,40 : les H aromatiques ; 7,88-8,04 : H de l'imidazole ; 7,88 : -N<u>H</u>-C$\Phi_3$.

**Stade B : Sel interne de (6R-(3-(E) 6alpha, 7béta-(Z))) 1-(3-(7-(((2-amino 4-thiazolyl) ((carboxy (2,5-dichloro 3,4-dihydroxy phényl) méthoxy) imino) acétyl) amino) 2-carboxy 8-oxo 5-thia 1-azabicyclo[4,2,0]oct-2-èn-3-yl) 2-propényl) imidazo(1,2-a) pyridinium**

On opère comme au stade I de l'exemple 1 à partir du produit obtenu au stade A, on obtient 327 mg du produit recherché.
Spectre RMN : $CDCl_3$ 300 MHz
3,50 à 3,75 : -S-C<u>H</u>$_2$-C≡ ; 5,16 : -NH-C<u>H</u>-CH-S- ; 5,79 (m) : -NH-CH-C<u>H</u>-S- ; 6,24 (m) : ≡C-CH=C<u>H</u>-C- ; 6,91 (dd) : ≡C-C<u>H</u>=CH-C- ; 6,83 (dd) : H thiazole ; 7,58-8,06 (t) et 8,21-8,96 (d) : H pyridine ; 8,29-8,45 : H imidazole ; 9,62 : NH ; 9,93 : $NH_2$, $CO_2H$.

**EXEMPLE 27 : Sel interne de (6R-(3-(E) 6alpha, 7béta-(Z))) 1-(3-(7-(((2-amino 4-thiazolyl) ((carboxy (2,5-dichloro 3,4-dihydroxy phényl) méthoxy) imino) acétyl) amino) 2-carboxy 8-oxo 5-thia 1-azabicyclo[4,2,0]oct-2-èn-3-yl) 2-propényl) 6,7-dihydro 5-H-pyrindinium**

**Stade A : iodure de 1-[3-[7béta-[[[[[1-[2,5-dichloro 3,4bis-[(2-méthoxy éthoxy) méthoxy] phényl] 2-oxo 2-(diphényl méthoxy) éthyl] oxy] imino] [2-[(triphénylméthyl) amino] thiazol 4-yl] acétyl] amino] 2-[(4-méthoxy benzyloxy) carbonyl] 8-oxo 5-thia 1-azabicyclo[4,2,0] oct-2-èn-3-yl] 2-propényl] 6,7-dihydro 5H-pyrindinium**

On opère comme au stade H de l'exemple 1, à partir de 1,13 g de produit obtenu comme au stade C de l'exemple 25 en utilisant 457 mg de cyclopentano[2,3]pyridine. Après chromatographie sur silice (éluant chlorure de méthylène-méthanol (9-1)) on obtient 460 mg du produit recherché.
Spectre RMN : $CDCl_3$ 300 MHz ppm
2,41-3,23-3,45 : -S-C<u>H</u>$_2$ et $CH_2$ du cyclopentane ; 3,34 à 3,37 : O-$CH_3$ ; 3,57 à 3,98 : -O-C<u>H</u>$_2$-C<u>H</u>$_2$-O- ; 3,81 : Φ-O-$CH_3$ ; 4,97-5,88 : NH-C<u>H</u>-C<u>H</u>-S- ; 5,12 à 5,25 : -O-C<u>H</u>$_2$-O- ; 6,22 et 6,45 (ΔE) ≡C-CH=C<u>H</u>-$CH_2$- ; 6,85 à 7,35 : ≡C-C<u>H</u>=CH-$CH_2$- ; 6,46-6,50 (s) =N-O-C<u>H</u>-$CO_2$- ; 6,72 : H thiazole ; 6,85 à 7,35 : H aromatiques ; 7,97-8,23 (d) : NH ; 7,79-9,20 : H pyridine.

**Stade B : Sel interne de (6R-(3-(E) 6alpha, 7béta-(Z))) 1-(3-(7-(((2-amino 4-thiazolyl) ((carboxy (2,5-dichloro 3,4-dihydroxy phényl) méthoxy) imino) acétyl) amino) 2-carboxy 8-oxo 5-thia 1-azabicyclo[4,2,0]oct-2-èn-3-yl) 2-propényl) 6,7-dihydro 5-H-pyrindinium**

On opère comme au stade I de l'exemple 1, à partir de 447 mg du produit obtenu au stade précédent. On obtient 194 mg du produit recherché.
Spectre RMN : DMSO 300 MHz
2,23 (m) : les $CH_2$ centraux ; 3,19 (t) les =C-$CH_2$ ; 5,17 (d) : $H_6$ ; 5,33 (m) : =CH-$CH_2$-N$^\oplus$ ; 5,78 (m) (2H) : les $H_7$ et O-CH-Φ ; 6,23 (m) : $CH_2$-CH=ΔE ; 6,82 (s) (d) : les $H_5$ thiazole ; 6,89 (d)d) les =C-CH=CH (ΔE) ; 7,01 (S,d) H aromatique ; 7,91 (t) : $H_5$' ; 8,42 (d) : $H_4$' ; 8,76 (d) : $H_6$' ; 7,35 (1) : $NH_2$ ; 9,56 à 10,0 les H mobiles.

**EXEMPLE 28 : Sel interne de (6R-(3-(E) 6alpha, 7béta-(Z))) 1-(3-(7-(((2-amino 4-thiazolyl) ((carboxy (2,5-dichloro 3,4-dihydroxy phényl) méthoxy) imino) acétyl) amino) 2-carboxy 8-oxo 5-thia 1-azabicyclo[4,2,0]oct-2-èn-3-yl) 2-propényl) 1-méthyl pyrrolidinium**

**Stade A : iodure de 1-[3-[7béta-[[[[[1-[2,5-dichloro 3,4bis-[(2-méthoxy éthoxy) méthoxy] phényl] 2-oxo 2-(diphényl méthoxy) éthyl] oxy] imino] [2-[(triphénylméthyl) amino] thiazol 4-yl] acétyl] amino] 2-[(4-méthoxy benzyloxy) carbonyl] 8-oxo 5-thia 1-azabicyclo[4,2,0] oct-2-èn-3-yl] 2-propényl] 1-méthyl pyrrolidinium**

On opère comme au stade H de l'exemple 1 à partir de 605 mg du produit obtenu comme au stade C de l'exemple 25, en utilisant 174 mg de N-méthyl pyrrolidine. Après chromatographie sur silice (éluant chlorure de méthylène-méthanol (9-1)). On obtient 210 mg du produit recherché.
Spectre RMN : 400 MHz dans $CDCl_3$
3,49 à 3,72 et 3,97 : les $CH_2$ centraux et N$^\oplus$$CH_3$ ; 3,20-3,33-3,34 : les $OCH_3$ ; 2,6 : pyrrolidine ; 4,01-4,28-4,52 : N$^\oplus$-C<u>H</u>$_2$ ; 5,25 : $CO_2$-C<u>H</u>$_2$-Φ et O-C<u>H</u>$_2$-O ; 6,09 et 6,17 ≡C-CH=C<u>H</u>-$CH_2$- ; 6,85 à 7,42 : ≡C-C<u>H</u>=$CH_2$- et $CO_2$-CH-$Φ_2$.

**Stade B : Sel interne de (6R-(3-(E) 6alpha, 7béta-(Z))) 1-(3-(7-(((2-amino 4-thiazolyl) ((carboxy (2,5-dichloro 3,4-dihydroxy phényl) méthoxy) imino) acétyl) amino) 2-carboxy 8-oxo 5-thia 1-azabicyclo[4,2,0]oct-2-èn-3-yl) 2-propényl) 1-méthyl pyrrolidinium**

On opère comme au stade I de l'exemple 1, à partir de 200 mg du produit obtenu au stade précédent. On obtient 93 mg du produit recherché.
Spectre RMN : DMSO 300 MHz ppm
2,09 (sl) : $CH_2$ en 3' 4' ; ~ 3,45 (sl) $CH_2$ en 2' 5' ; 2,99 (s) : $N^{\oplus}C\underline{H}_3$ ; 3,61 (d,d) et 3,80 (d) 3,86 (d) : $CH_2$-S ; 4,10 (d) : =CH-$C\underline{H}_2$-$N^{\oplus}$ ; 5,21 (d) : $H_6$ ; 5,84 (m) : $H_7$ ; 5,78 (s) 5,81 (s) =C-C$\underline{H}$-O ; 6,16 (m) : CH=C$\underline{H}$-$CH_2$ (ΔE) ; 7,03 (d,J=15) C$\underline{H}$=CH-$CH_2$ ; 7,01 (s)-7,06 (s)-6,83 (s)-6,84 (s) : $H_5$ du thiazole et 1H aromatique ; 9,60 (d)-9,66 (d) CON$\underline{H}$-CH ; 9,95 (m)-7,40 (m) : H mobiles.

**EXEMPLE 29 : Sel interne de (6R-(3-(E) 6alpha, 7béta-(Z))) 1-(3-(7-(((2-amino 4-thiazolyl) ((carboxy (2,5-dichloro 3,4-dihydroxy phényl) méthoxy) imino) acétyl) amino) 2-carboxy 8-oxo 5-thia 1-azabicyclo[4,2,0]oct-2-èn-3-yl) 2-propényl) thiéno(2,3-c) pyridinium**

**Stade A : iodure de 1-[3-[7béta-[[[[1-[2,5-dichloro 3,4bis-[(2-méthoxy éthoxy) méthoxy] phényl] 2-oxo 2-(diphényl méthoxy) éthyl] oxy] imino] [2-[(triphénylméthyl) amino] thiazol 4-yl] acétyl] amino] 2-[(4-méthoxy benzyloxy) carbonyl] 8-oxo 5-thia 1-azabicyclo[4,2,0] oct-2-èn-3-yl] 2-propényl] thiéno(2,3-c) pyridinium**

On opère comme au stade H de l'exemple 1 à partir de 1 g du produit obtenu comme au stade C de l'exemple 25 en utilisant 458 mg de thiéno[2,3-c]pyridine. Après chromatographie sur silice (éluant chlorure de méthylène-méthanol (9-1)), on obtient 520 mg du produit recherché.
Spectre RMN : $CDCl_3$ 300 MHz
3,56 (s)-3,98 (s) : les $CH_2$ centraux et S-$CH_2$ ; 3,79 (s) : Φ-$OCH_3$ ; 5,20 à 5,30 : O-$CH_2$-O et $CO_2$-$CH_2$-Φ ; 4,95-5,26 (m) : NH-C$\underline{H}$-C$\underline{H}$-S ; 6,33-6,48 (m) ΔE : C-CH=C$\underline{H}$- ; 6,80 à 7,40 : ≡C-C$\underline{H}$=CH- ; 5,59 (m) à 5,80 (m) : =CH-$C\underline{H}_2$-N≡ ; 6,80 à 7,40 : H aromatique ; 6,77 (sd) : H thiazole ; 11,50 (sl,d) NH,-N=CH-.

**Stade B : Sel interne de (6R-(3-(E) 6alpha, 7béta-(Z))) 1-(3-(7-(((2-amino 4-thiazolyl) ((carboxy (2,5-dichloro 3,4-dihydroxy phényl) méthoxy) imino) acétyl) amino) 2-carboxy 8-oxo 5-thia 1-azabicyclo[4,2,0]oct-2-èn-3-yl) 2-propényl) thiéno(2,3-c) pyridinium**

On opère comme au stade I de l'exemple 1 à partir du produit obtenu au stade A ci-dessus. On obtient 114 mg du produit recherché.
Spectre RMN : DMSO 300 MHz
5,19 (d) : $H_6$ ; 5,46 (d) $N^{\oplus}C\underline{H}_2$-CH= ; 5,79 (m) : -O-C$\underline{H}$-Φ, $H_7$ ; 6,35 (m) : =CH-$CH_2$ (ΔE) ; 6,81 (s)d : $H_5$ thiazole ; 7,00 (s) : H du phényl ; 7,07 (dJ=16) : =C-C$\underline{H}$=CH (ΔE) ; 7,34 (1) : $NH_2$ ; 7,94 (dJ=5,5) $H_3$' ; 8,86 (dJ=5,5) : $H_2$' ; 8,53 (d, J=6,5)-8,73 (d,J=6,5) : $H_4$' et $H_5$' ; 9,91 (s,l) : $H_6$' ; 9,59 (d,d) =C-N$\underline{H}$-CH ; 9,88 : H mobile.

**EXEMPLE 30 : Sel interne de (6R-(3-(E) 6alpha, 7béta-(Z))) 7-(3-(7-(((2-amino 4-thiazolyl) ((carboxy (4-cyano 2,3-dihydroxy phényl) méthoxy) imino) acétyl) amino) 2-carboxy 8-oxo 5-thia 1-azabicyclo[4,2,0]oct-2-èn-3-yl) 2-propényl) thiéno(2,3-b) pyridinium**

**Stade A : Acide [4-cyano 2,3bis [(2-méthoxy éthoxy) méthoxy] phényl] bromoacétique**

A un mélange de 4,26 mg de bromure de lithium, 598 mg de potasse et 5 $cm^3$ d'eau, on ajoute, à -5°C, 440 microlitres de bromoforme puis, goutte à goutte une solution de 825 mg de 4-cyano 2,3bis[(2-méthoxy éthoxy) méthoxy] benzaldéhyde (préparation donnée ci-après) dans 5 $cm^3$ de dioxanne, on agite 72 heures à 0°/-5°C. On verse le mélange réactionnel sur un mélange agité à 0°/-5°C comprenant 15 $cm^3$ d'acétate d'éthyle et 11 $cm^3$ d'acide chlorhydrique, on extrait avec de l'acétate d'éthyle, lave, sèche puis évapore à sec sous pression réduite, on obtient une huile que l'on utilise telle quelle pour le stade suivant.

**Stade B : [4-cyano 2,3bis-[(2-méthoxy éthoxy) méthoxy] phényl] bromoacétate de diphényle méthyle**

On dissout le produit obtenu au stade A dans 10 $cm^3$ de chlorure de méthylène et ajoute 470 mg de diphényl diazométhane. On évapore et chromatographie sur silice (éluant cyclohexane-acétate d'éthyle (2-1)) on obtient ainsi 450 mg du produit recherché.
Spectre RMN :

3,31 (s), 3,37 (s) : les $OCH_3$ ; 3,43 (m)-3,61 (m)-3,77 (m)-4,05 (m) les $CH_2$ centraux ; 5,18 (d)-5,25 (d)-5,29 (AB) les $OCH_2O$ ; 6,02 (s) : =CH-X

**Stade C : 4-cyano [2,3bis-[(2-méthoxy éthoxy) méthoxy] phényl] phtalimidoxy acétate de diphényl méthyle**

A une solution de 820 mg de produit obtenu ci-dessus dans 13 $cm^3$ de diméthyl formamide, on ajoute à 0°C : 326 mg de N-hydroxyphtalimide et 196 mg d'acétate de potassium. On agite 3 heures à température ambiante, évapore à sec et chromatographie sur silice (éluant : cyclohexane-acétate d'éthyle (1-1)), on obtient ainsi 640 mg de produit recherché, utilisé tel quel pour le stade suivant.

**Stade D : 4-cyano [2,3bis-[(2-méthoxy éthoxy) méthoxy] phényl] aminoxy acétate de diphényl méthyle**

On opère comme au stade D de l'exemple 1, à partir de 640 mg du produit obtenu ci-dessus, en utilisant 50 micro-litres d'hydrate d'hydrazine, on obtient 320 mg de produit attendu.

**Stade E : Acide [[[1-[4-cyano 2,3bis-[(2-méthoxy éthoxy) méthoxy] phényl] 2-oxo 2-(diphénylméthoxy) éthyl] oxy] imino] [2-[(triphénylméthyl) amino] thiazol 4-yl acétique**

On opère comme au stade E de l'exemple 1, à partir de 320 mg du produit obtenu ci-dessus, en utilisant 234 mg de l'acide triphénylamino thiazol 4-yl acétique, on obtient 550 mg du produit recherché.
Spectre RMN : $CDCl_3$ ppm
3,32 (s), 3,37 (s) : les $OCH_3$ ; 3,48 (t)-3,60 (t)-3,75 (m)-4,03 (m) : les $CH_2$ centraux ; 5,13 (AB)-5,24 (AB) : les O-$CH_2$-O ; 6,27 (s) :

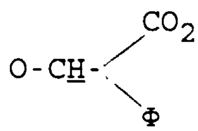 ;

6,74 (s) : $H_5$ thiazole ; 6,92 (s) : -$CO_2$-CH-$\Phi_2$ ; 7,09 à 7,32 : les H aromatiques ; 2,60 H mobile : NH.

**Stade F : 7béta[[[[[1-[4-cyano 2,3bis-[(2-méthoxy éthoxy) méthoxy] phényl] 2-oxo 2-(diphénylméthaxy) éthyl] oxy] imino] [2-[(triphénylméthyl) amino] thiazol 4-yl] acétyl] amino] 3-[(Z) 3-chloro 1-propényl] 8-oxo 5-thia 1-azabicyclo oct-2-èn-2-carboxylate de 4-méthoxy benzyle**

On opère comme au stade F de l'exemple 1, à partir de 550 mg du produit obtenu ci-dessus. Après chromatographie sur silice (éluant chlorure de méthylène-acétate d'éthyle (8-2)), on obtient 580 mg du produit recherché.

**Stade G : 7béta[[[[[1-[4-cyano 2,3bis-[(2-méthoxy éthoxy) méthoxy] phényl] 2-oxo 2-(diphénylméthaxy) éthyl] oxy] imino] [2-[(triphénylméthyl) amino] thiazol 4-yl] acétyl] amino] 3-[(Z) 3-iodo 1-propényl] 8-oxo 5-thia 1-azabicyclo oct-2-èn-2-carboxylate de 4-méthoxy benzyle**

On opère comme au stade G de l'exemple 1, à partir de 580 mg du produit obtenu ci-dessus, on obtient 615 mg du produit recherché.
Spectre RMN : $CDCl_3$
3,25 (s)-3,27 (s)-3,37 (s)-3,2 à 3,6 (m) : les $OCH_3$ et les $CH_2S$ ; 3,44 (m)-3,60 (m)-3,80 (m) : les $CH_2$ centraux ; 4,01 (m) : $CH_2$-I ; 4,93 (d) : $H_6$ ; 5,17 à 5,38 (m) : les O-$CH_2$-O ; 5,78 (dd)-5,86 (dd) : $H_7$ ; 6,12 (m) : CH=CH-$CH_2$ ; 6,43-6,46 (s) : O-CH-Φ ; 6,75 (s)-6,76 (s) : $H_5$ thiazole ; 6,83 à 7,45 (m) : H aromatiques, HC=, $CO_2$-CH-$\Phi_2$ ; 7,82-8,15 (d) : H mobile : CONH.

**Stade H : Iodure de 1-[3-[7béta[[[[[1-[4-cyano 2,3bis-[(2-méthoxy éthoxy) méthoxy] phényl] 2-oxo 2-(diphénylméthoxy) éthyl] oxy] imino] [2-[(triphénylméthyl) amino] thiazol 4-yl] acétyl] amino] 2-[(4-méthoxy benzyloxy) carbonyl] 8-oxo 5-thia 1-azabicyclo[4,2,0] [oct-2-èn-3-yl] 2-propényl] thiéno(2,3-b) pyridinium**

On opère comme au stade H de l'exemple 1, à partir de 615 mg du produit obtenu ci-dessus et en utilisant 290 micro-litres de thiéno(2,3-b) pyridine.
Après chromatographie sur silice (éluant chlorure de méthylène-méthanol (9-1)) on obtient 300 mg de produit

recherché.
Spectre RMN : $CDCl_3$ ppm
3,20 (s)-3,26 (s)-3,35 (s)-3,37 (s) : les $CH_3O$ ; 3,43 (m)-3,50 (m)-3,78 (m)-4,02 (m) : les $CH_2$ centraux ; 3,80-3,81 (s) : Φ-O-C<u>H</u>$_3$ ; 4,93 (d,d) : $H_6$ ; 5,76-5,84 (m) : $H_7$ ; 5,71-5,96 (m) : C-C<u>H</u>$_2$-N⊕ ; 6,40-6,45 (s) : O-CH-$CO_2$-CH-$\Phi_2$ ; 6,91 (m) =C<u>H</u>-$CH_2$ (ΔE) ; 6,73-6,74 (s) : $H_5$ thiazole ; 6,94 (m) : H aromatique, -O-C<u>H</u>-$\Phi_2$, C<u>H</u>=CH- (ΔE) ; 7,05 à 7,45 : les H aromatiques.

**Stade I : Sel interne de (6R-(3-(E) 6alpha, 7béta-(Z))) 7-(3-(7-(((2-amino 4-thiazolyl) ((carboxy (4-cyano 2,3-dihydroxy phényl) méthoxy) imino) acétyl) amino) 2-carboxy 8-oxo 5-thia 1-azabicyclo[4,2,0]oct-2-èn-3-yl) 2-propényl) thiéno(2,3-b)pyridinium**

On opère comme au stade I de l'exemple 1, à partir de 292 mg du produit obtenu ci-dessus, on obtient 176 mg du produit recherché.
Spectre RMN : DMSO
5,18 (d,d) : $H_6$ ; 5,82 (m) : $H_7$ ; 5,68 (d) : =C-$CH_2$-N⊕ ; 5,93 (s,d) : O-C<u>H</u>-Φ ; 6,29 (m) : $CH_2$-C<u>H</u>=CH- (ΔE) ; 6,79 (s,d) : $H_5$ thiazole ; 6,94 (d,d), 7,03 (d) : H aromatique ; 7,89 (d) $H_3'$, 8,28 $H_2'$, 8,15 $H_5'$, 9,22 $H_4'$ : thiénopyridine ; 9,54-9,69 : CO-NH-CH ; 10,33 : H mobile.

**<u>PREPARATION EXEMPLE 30</u> : 4-cyano 2,3bis-[(2-méthoxy éthoxy) méthoxy] benzaldéhyde**

**Stade A : 1,4-dicyano 2,3-dihydroxyphényl**

A un mélange de 400 $cm^3$ de tétrahydrofuranne, 50 g de dicyanoéthyl sulfure et 52 g de diéthyl oxalate, on ajoute 46 g d'éthylate de sodium. On agite 1 heure, concentre sous pression réduite et reprend avec 600 $cm^3$ d'eau et 250 $cm^3$ d'acétate d'éthyle, lave avec de l'acétate d'éthyle et acidifie la phase aqueuse avec de l'acide chlorhydrique concentre. On extrait avec de l'acétate d'éthyle et évapore à sec sous pression réduite, l'extrait sec obtenu est repris avec 400 $cm^3$ de nitrométhane, on essore et lave à l'eau. On obtient 12,46 g du produit recherché. Rf 0,31 dans $CH_2Cl_2$-MeOH 85-15.

**Stade B : 1,4-dicyano 2,3-diméthoxy phényl**

On agite 16 heures au reflux un mélange de 1 g du produit obtenu ci-dessus, 60 $cm^3$ d'acétone, 3,15 g d'acétate de potassium et 1,5 $cm^3$ de sulfate diméthylique, on refroidit, filtre et évapore à sec sous pression réduite. On chromatographie le résidu obtenu sur silice (éluant chlorure de méthylène-hexane (9-1)), on obtient ainsi 820 mg du produit recherché.
Spectre IR : ($CHCl_3$)
CN conjugué 2240 $cm^{-1}$
Aromatique 1595-1555 $cm^{-1}$

**Stade C : 4-cyano 2,3-diméthoxy benzaldéhyde**

A une solution de 15,70 g de produit obtenu comme ci-dessus, on ajoute 900 $cm^3$ de toluène puis à -74°C, on ajoute en 15 minutes 61 $cm^3$ d'une solution 1,5 M d'hydrure de diisobutylaluminium dans le toluène, on agite pendant 30 minutes à -70/-74°C, on ajoute lentement 20 $cm^3$ d'acétone, agite 10 minutes et verse le mélange réactionnel sur 700 $cm^3$ d'acide chlorhydrique 1N et 200 $cm^3$ d'acétate d'éthyle, on agite 30 minutes à température ambiante, décante et extrait à l'acétate d'éthyle, sèche et évapore à sec, on obtient 15,5 g de produit recherché. A l'extrait sec obtenu, on ajoute 4,9 g d'une précédente venue et chromatographie les 20,4 g de produit brut, sur silice par 3 fois, en éluant avec toluène-éthanol (95-5). On recueille ainsi 7,5 g du produit recherché.
Spectre RMN : $CDCl_3$
4,06-4,10 : les O-$CH_3$ ; 7,38 (d)-7,60 (d) : 2H aromatique, couplage ortho ; 10,42 (s) : CHO.

**Stade D : 4-cyano 2,3bis-[(2-méthoxy éthoxy) méthoxy] benzaldéhyde**

A une solution de 5,9 g de produit obtenu comme ci-dessus dans 150 $cm^3$ de chlorure de méthylène, on ajoute à-70°C, 124 $cm^3$ d'une solution molaire de tribromure de bore. On agite 72 heures à température ambiante. On refroidit le milieu à -30°C et ajoute lentement, 30 $cm^3$ de méthanol, on agite 30 minutes, évapore à sec sous pression réduite et reprend avec 200 $cm^3$ d'acétate d'éthyle, lave à l'eau, sèche et évapore à sec sous pression réduite, on obtient 6,4 g de résidu auxquels on ajoute 190 $cm^3$ de chlorure de méthylène. On refroidit à 0°C et ajoute 21 $cm^3$ de

diisopropyléthyl amine et 141 $cm^3$ de chlorure de méthoxy éthoxy méthyle. On agite 3 heures à température ambiante. On lave la solution réactionnelle avec de l'acide chlorhydrique N, de la soude N et à l'eau, sèche et évapore à sec. Le résidu est chromatographié sur silice (éluant chlorure de méthylène-acétate d'éthyle (9-1)). On obtient 4,93 g du produit recherché.

Spectre RMN : $CDCl_3$

3,35-3,38 (s) : les O-$CH_3$ ; 3,53-3,62-3,88-4,08 (m) : les $CH_2$ centraux ; 5,33-5,39 (s) : O-$CH_2$-O ; 7,45 (dd=J=8 et 0,5 Hz) : $H_6$ ; 7,67 (d=J=8 Hz) : $H_5$ ; 10,39 (d,J = 0,5 Hz) : CHO.

**EXEMPLE 31 : Sel interne de (6R-(3-(E) 6alpha, 7béta-(Z))) 7-(3-(7-(((2-amino 4-thiazolyl) ((carboxy (4-cyano 2,3-dihydroxy phényl) méthoxy) imino) acétyl) amino) 2-carboxy 8-oxo 5-thia 1-azabicyclo[4,2,0]oct-2-èn-3-yl) 2-propényl) quinoléinium**

**Stade A : Iodure de 1-[3-[7béta[[[[[1-[4-cyano 2,3bis-[(2-méthoxy éthoxy) méthoxy] phényl] 2-oxo 2-(diphénylméthoxy) éthyl] oxy] imino] [2-[(triphénylméthyl) amino] thiazol 4-yl] acétyl] amino] 2-[(4-méthoxy benzyloxy) carbonyl] 8-oxo 5-thia 1-azabicyclo[4,2,0] [oct-2-èn-3-yl] 2-propényl] quinoléinium**

On opère comme au stade H de l'exemple 1, à partir de 615 mg du produit obtenu au stade G de l'exemple 30, en utilisant 200 microlitres de quinoléine. Après chromatographie sur silice (éluant : chlorure de méthylène-méthanol (91-9)), on obtient 145 mg du produit recherché.

**Stade B : Sel interne de (6R-(3-(E) 6alpha, 7béta-(Z))) 7-(3-(7-(((2-amino 4-thiazolyl) ((carboxy (4-cyano 2,3-dihydroxy phényl) méthoxy) imino) acétyl) amino) 2-carboxy 8-oxo 5-thia 1-azabicyclo[4,2,0]oct-2-èn-3-yl) 2-propényl) quinoléinium**

On opère comme au stade I de l'exemple 1, à partir de 230 mg de produit obtenu comme au stade précédent. On obtient 137 mg de produit recherché.

Spectre RMN : DMSO

3,30 à 3,80 (m) : $CH_2$-S ; 5,15 (d) : $H_6$ ; 5,60 (dd) : $H_7$ ; 5,91-5,93 (s) : O-CH-Φ ; 5,90 (m) : $CH_2$-$N^{\oplus}$ ; 6,37 (m) : HC=CH-$CH_2$ ; 6,93 (d,J = 16) HC=CH-$CH_2$ ; 6,78-6,94 (d) : $H_5$', $H_6$' ; 7,01 (s) : H thiazole ; 8,07-8,28 (t) : $H_6$", $H_7$" ; 8,23 (dd) (J = 6 et 8 Hz) : $H_3$" ; 8,51 (d)-8,55 (dd) : $H_5$" et $H_4$" ou $H_8$" ; 9,34 (d,J = 8 Hz) $H_4$" ou $H_8$" ; 9,59 (d,J = 6 Hz) : $H_2$" ; 9,52 (d)-9,68 (d) : HC-NH-CO.

**EXEMPLE 32 : Sel interne de (6R-(3-(E) 6alpha, 7béta-(Z))) 7-(3-(7-(((2-amino 4-thiazolyl) ((carboxy (5-cyano 3,4-dihydroxy phényl) méthoxy) imino) acétyl) amino) 2-carboxy 8-oxo 5-thia 1-azabicyclo[4,2,0]oct-2-èn-3-yl) 2-propényl) thiéno(2,3b)pyridinium (isomère RS)**

**Stade A : Iodure de 1-[3-[7béta-[[[[[1-[5-cyano 3,4bis-[(2-méthoxy éthoxy) méthoxy] phényl] 2-oxo 2-(diphénylméthoxy)éthyl] oxy] imino] [2-[(triphénylméthyl) amino] thiazol 4-yl] acétyl] amino] 2-[(4-méthoxy benzyloxy) carbonyl] 8-oxo 5-thia 1-azabicyclo[4,2,0] [oct-2-èn-3-yl] 2-propényl) thiéno(2,3b)pyridinium (isomère RS)**

On opère comme au stade H de l'exemple 2 à partir de 1,5 g de produit obtenu au stade G de l'exemple 2, en utilisant 1,35 g de thiéno[2,3-b]pyridine.

On obtient 1,22 g du produit attendu (mélange R/S).

Spectre RMN : $CDCl_3$ 300 MHz

3,27 (s)-3,29 (s)-3,35 (s)-3,36 (s) : les C=OMe ; 3,80 (s)-3,81 (s) : les =C-OMe ; ~ 3,30 à 4,10 : les $CH_2$ centraux et $CH_2S$ ; ~ 5,00 à 5,11 : les $H_6$ ; ~ 5,20 à 5,40 : les O-$CH_2$-O ; 6,76 (s) et 6,77 (s) : les $H_5$ thiazoles ; - 6,90 à 7,50 : les $COOCH\Phi_2$ et $C_6H_5$ ; 9,58 (d) dédoublé : les $H_6$' ; 7,52 à 8,90 les autres H quinoléine ; 8,22 et 8,34 (d) : les =C-NH-CH.

**Stade B : Sel interne de (6R-(3-(E) 6alpha, 7béta-(Z))) 7-(3-(7-(((2-amino 4-thiazolyl) ((carboxy (5-cyano 3,4-dihydroxy phényl) méthoxy) imino) acétyl) amino) 2-carboxy 8-oxo 5-thia 1-azabicyclo[4,2,0]oct-2-èn-3-yl) 2-propényl) thiéno(2,3b)-pyridinium (mélange R/S)**

On opère comme au stade I de l'exemple 1, à partir de 1,2 g du produit obtenu au stade A, on obtient 730 mg du produit recherché.

Spectre RMN : DMSO 300 MHz

5,17 (d) dédoublé : $H_6$ ; 5,41 (s) : O-CH-Φ ; 5,68 (d) : $CH_2$-$N^{\oplus}$ ; 5,79 (m) : $H_7$ ; 6,87 (m) : =C-CH=CH-$CH_2$ (ΔE) ; 6,77 (s) et 6,79 (s) : $H_5$ thiazole ; 7,12 et 7,17 : aromatique et =C-CH=CH- ; 7,89 (d) : $H_3$' ; 8,28 (d) : $H_2$' ; 8,15 (dd) : $H_5$' ; 9,09 (d) : $H_4$' ; 9,23 (d) dédoublé $H_6$' ; 9,68 (d) dédoublé : NH ; 10,38 : H mobile ; 7,39 (m) : $NH_2$.

**EXEMPLE 33 : Sel interne de (6R-(3-(E) 6alpha, 7béta-(Z))) 7-(3-(7-(((2-amino 4-thiazolyl) ((carboxy (5-cyano 3,4-dihydroxy phényl) méthoxy) imino) acétyl) amino) 2-carboxy 8-oxo 5-thia 1-azabicyclo[4,2,0]oct-2-èn-3-yl) 2-propényl) thiéno(2,3b)pyridinium (isomère S)**

On chromatographie 240 mg du produit obtenu sur colonne microbondapack, éluant eau-acétonitrile 82/18 (pH = 2,7). On obtient 45 mg du produit recherché.

Spectre RMN : DMSO 300 MHz

5,15 (d,J = 5) : $H_6$ ; 5,40 (s) : O-CH-Φ ; 5,67 (d) : =CH-$CH_2$-$N^{\oplus}$ ; 5,80 (dd,J = 5 et 8) : $H_7$ ; 6,25 (m) : =CH-$CH_2$ ; 6,79 (s) : $H_5$ thiazole ; 7,13 (m) : aromatique et =CH-CH (ΔE) ; 7,89 (d,J = 6)-8,28 (d,J = 6) : $H_3$' et $H_2$' ; 8,15 (m) : $H_5$' ; 9,09 (d,J = 8) : $H_4$' ; 9,22 (d,J = 6) : $H_6$' ; 9,62 (d,J = 8) : =C-NH-CH ; 10,34-10,43 : les OH ; 7,33 : les $NH_2$.

**EXEMPLE 34 : Sel interne de (6R-(3-(E) 6alpha, 7béta-(Z))) 1-(3-(7-(((2-amino 4-thiazolyl) ((carboxy (3-chloro 4,5-dihydroxy phényl) méthoxy) imino) acétyl) amino) 2-carboxy 8-oxo 5-thia 1-azabicyclo[4,2,0]oct-2-èn-3-yl) 2-propényl) imidazo(1,2-a)pyridinium**

**Stade A : Acide [[[1-[3-chloro 4,5bis-[(2-méthoxy éthoxy) méthoxy] phényl] 2-oxo 2-(diphénylméthaxy) éthyl] oxy] imino] [2-[(triphénylméthyl) amino] thiazol 4-yl] acétique**

On opère comme au stade E de l'exemple 1, à partir de 2,56 g du produit obtenu au stade D de l'exemple 4 en utilisant 1,84 g de l'acide oxo-[2-[(triphénylméthyl) amino] thiazol 4-yl] acétique (demande de brevet belge n° 864 828). Après chromatographie sur silice (éluant chlorure de méthylène-méthanol (97-3)), on obtient 3,34 g du produit recherché (Rf 0,54 : $CH_2Cl_2$-MeOH (9-1)).

**Stade B : 7béta-[[[[[1-[3-chloro 4,5bis-[(2-méthoxy éthoxy) méthoxy] phényl] 2-oxo 2-(diphénylméthoxy) éthyl] oxy] imino] [2-[(triphénylméthyl) amino] thiazol 4-yl] acétyl] amino] 3-[(Z) 3-chloro 1-propényl] 8-oxo 5-thia 1-azabicyclo[4,2,0]oct-2-èn-2-carboxylate de 4-méthoxy benzyle**

On opère comme au stade F de l'exemple 1, à partir de 1,42 g de chlorhydrate de 7béta-amino 3-[(Z) 3-chloro 1-propényl] 8-oxo 5-thia 1-azabicyclo[4,2,0]oct-2-èn-2-carboxylate de 4-méthoxy benzyle (EP 0 333 154) et 3,34 g du produit obtenu au stade A ci-dessus. On obtient 2,88 g du produit recherché. (Rf 0,44 : $CH_2Cl_2$-ACOEt (8-2)).

**Stade C : 7béta-[[[[[1-[3-chloro 4,5bis-[(2-méthoxy éthoxy) méthoxy] phényl] 2-oxo 2-(diphénylméthaxy) éthyl] oxy] imino] [2-[(triphénylméthyl) amino] thiazol 4-yl] acétyl] amino] 3-[(Z) 3-iodo 1-propényl] 8-oxo 5-thia 1-azabicyclo[4,2,0]oct-2-èn-2-carboxylate de 4-méthoxy benzyle**

On opère comme au stade G de l'exemple 1, à partir de 1,88 g du produit obtenu au stade B. On obtient 1,75 g du produit recherché (Rf 0,52 $CH_2Cl_2$-ACOEt (8-2)).

**Stade D : Iodure de 1-[3-[7béta-[[[[[1-[3-chloro 4,5bis-[(2-méthoxy éthoxy) méthoxy] phényl] 2-oxo 2-(diphénylméthoxy) éthyl] oxy] imino] [2-[(triphénylméthyl) amino] thiazol 4-yl] acétyl] amino] 2-[(4-méthoxy benzyloxy carbonyl] 8-oxo 5-thia 1-azabicyclo[4,2,0] [oct-2-èn-3-yl] 2-propényl] imidazo(1,2-a) pyridinium**

On opère comme au stade H de l'exemple 1 à partir de 876 mg du produit obtenu au stade C, en utilisant 0,308 $cm^3$ d'imidazo[1,2-a]pyridine. Après chromatographie sur silice (éluant : chlorure de méthylène-méthanol (97-3)) on obtient 450 mg de produit recherché.

Spectre RMN : $CDCl_3$ 300 MHz

3,25-3,29 (s)-3,35-3,36 (s) : les $OCH_3$ ; 3,43-3,55-3,72-4,01 (m) : les $CH_2$ centraux et $CH_2S$ ; 3,78 (s,d) Φ-O-Me ; 4,94 (d,d) et 5,83 (m) : $H_6$ et $H_7$ (cis) ; 5,17 à 5,35 et 5,43 (dt) : $OCH_2O$, $CO_2CH_2$-Φ, $NCH_2$-CH= ; 5,94 (s) : O-CH-Φ ; 6,25 (m) : =CH-$CH_2$ (ΔE) ; 6,76 (s) : $H_5$ thiazole ; 6,89 -CH-$Φ_2$ ; 6,85 à 7,40 : Φ-C, aromatique, CH=CH-C= (ΔE) ; 7,85 (d, d) : $H_5$', $H_6$' ; 8,05 (d,d) : $H_4$' ; 8,38 (d,d) : $H_3$' ; 8,64 (d,d) : $H_1$' ; 9,11 (d,d) : $H_7$' ; 7,97 à 8,19 (d) les NH.

**Stade E : Sel interne de (6R-(3-(E) 6alpha, 7béta-(Z))) 1-(3-(7-(((2-amino 4-thiazolyl) ((carboxy (3-chloro 4,5-dihydroxy phényl) méthoxy) imino) acétyl) amino) 2-carboxy 8-oxo 5-thia 1-azabicyclo[4,2,0]oct-2-èn-3-yl) 2-propényl) imidazo(1,2-a)pyridinium**

On opère comme au stade I de l'exemple 1, à partir de 489 mg du produit obtenu au stade D ci-dessus, on obtient 187 mg du produit attendu.

Spectre RMN : DMSO 300 MHz

3,62 : $CH_2S$ ; 5,29 (m) : $N^{\oplus}$-$CH_2$-CH= ; 5,34 (s,d) : O-C<u>H</u>-Φ ; 6,22 (m) : =C<u>H</u>-$CH_2$ (ΔE) ; 6,77 (s) : $H_5$ thiazole ; 6,85 (s)-6,90 : -C<u>H</u>-$Φ_2$, aromatique et C<u>H</u>=CH-$CH_2$ (ΔE) ; 7,33 : $NH_2$ et Φ-C ; 5,13 et 5,75 : $H_6$-$H_7$ ; 7,58 (t)-8,06 (t) : $H_5$'-$H_6$' ; 8,28 (m)-8,44 (sl) : $H_3$'-$H_2$' ; 8,96 (d) : $H_4$' ; 9,59 (d, d) : $H_7$' ; 9,29 (sl)-13,03-13,66 : H mobile.

**<u>EXEMPLE 35</u> : Sel interne de (6R-(3-(E) 6alpha, 7béta-(Z))) 1-(3-(7-(((2-amino 4-thiazolyl) ((carboxy (3-chloro 4,5-dihydroxy phényl) méthoxy) imino) acétyl) amino) 2-carboxy 8-oxo 5-thia 1-azabicyclo[4,2,0]oct-2-èn-3-yl) 2-propényl) 4-méthylthio pyridinium**

**Stade A : Iodure de 1-[3-[7béta-[[[[1-[3-chloro 4,5bis-[(2-méthoxy éthoxy) méthoxy] phényl] 2-oxo 2-(diphénylméthaxy) éthyl] oxy] imino] [2-[(triphénylméthyl) amino] thiazol 4-yl] acétyl] amino] 2-[(4-méthoxy benzyloxy carbonyl] 8-oxo 5-thia 1-azabicyclo[4,2,0] [oct-2-èn-3-yl] 2-propényl] 4-méthylthio pyridinium**

On opère comme au stade H de l'exemple 1, à partir de 875 mg du produit obtenu au stade C de l'exemple 34 en utilisant 385 mg de 4-S-méthyl thiopyridine. On obtient, après chromatographie sur silice (éluant chlorure de méthylène-méthanol (97-3 puis 90-10)), 389 mg du produit recherché.
<u>Spectre RMN</u> : $CDCl_3$ 300 MHz
2,61-2,63 : S-$CH_3$ ; 3,25-3,28-3,36-3,37 : les $OCH_3$ ; 3,78 (s)-3,79 (s) : les ΦOMe ; 3,44-3,58-3,72-4,01 : les $CH_2$ centraux et $CH_2$-S ; 4,97 (d,d) : $H_6$ ; 5,05 à 5,35 : $OCH_2O$, $CO_2C$<u>$H_2$</u>-Φ, $CH_2N^{\Phi}$ (1H) ; 5,56 (m)-5,85 : $CH_2N^{\Phi}$ (1H) ; 6,24-6,39 (m) : CH=C<u>H</u>-$CH_2$ (ΔE) ; 6,78 (sl) : $H_5$ thiazole ; 6,89 (sl) $CO_2C$<u>$H_2$</u>Φ ; de 7 à 7,40 : ΦC, aromatique et =C-C<u>H</u>=CH-(ΔE) ; 7,66 (d,d) et 8,90 (m) thiopyridinium.

**Stade B : Sel interne de (6R-(3-(E) 6alpha, 7béta-(Z))) 1-(3-(7-(((2-amino 4-thiazolyl) ((carboxy (3-chloro 4,5-dihydroxy phényl) méthoxy) imino) acétyl) amino) 2-carboxy 8-oxo 5-thia 1-azabicyclo[4,2,0]oct-2-èn-3-yl) 2-propényl) 4-méthylthio pyridinium**

On opère comme au stade I de l'exemple 1, à partir de 389 mg du produit obtenu au stade A. On obtient 174,7 mg du produit recherché.
<u>Spectre RMN</u> : DMSO 300 MHz
2,72 : $SCH_3$ ; 3,49 (s)-3,54 (d)-3,71 (d) : $CH_2S$ ; 5,13-5,17 (d) : $H_6$ (cis) ; 5,76 (m) : $H_7$ (cis) ; 5,23 (m) : $N^{\oplus}$-$CH_2$ ; 6,24 : =C<u>H</u>-$CH_2$ (ΔE) ; 6,77 et 6,78 (s) : $H_5$ thiazole ; 6,85 à 7,02 : aromatique et =CH-C<u>H</u>= ; 7,95-8,70 : pyridinium ; 9,30 (sl)-9,62 (d,d)-9,96 (ml) : les Hmobiles ; 7,34 : $NH_2$.

**<u>EXEMPLE 36</u> : Sel interne de (6R-(3-(E) 6alpha, 7béta-(Z))) 1 (3-(7-(((2-amino 4-thiazolyl) ((carboxy (3-chloro 4,5-dihydroxy phényl) méthoxy) imino) acétyl) amino) 2-carboxy 8-oxo 5-thia 1-azabicyclo[4,2,0]oct-2-èn-3-yl) 2-propényl) quinoléinium**

**Stade A : Iodure de 1-[3-[7béta-[[[[[1-[3-chloro 4,5bis-[(2-méthoxy éthoxy) méthoxy] phényl] 2-oxo 2-(diphénylméthoxy) éthyl] oxy] imino] [2-[(triphénylméthyl) amino] thiazol 4-yl acétyl] amino] 2-[(4-méthoxy benzyloxy carbonyl] 8-oxo 5-thi 1-azabicyclo[4,2,0] [oct-2-èn-3-yl] 2-propényl] quinoléinium**

On opère comme au stade H de l'exemple 1, à partir de 900 mg du produit obtenu au stade C de l'exemple 34, en utilisant 0,366 $cm^3$ de quinoléine. Après chromatographie sur silice (éluant chlorure de méthylène-méthanol (97-3)), on obtient 384 mg du produit recherché.
<u>Spectre RMN</u> : $CDCl_3$ 300 MHz
3,24 (s)-3,29 (s)-3,36 (s,d) : les O-$CH_3$ ; 3,78 (s) : les Φ-OMe ; 3,40 (m)-3,58 (m)-3,71 (m)-4,00 (m) : les $CH_2$ centraux ; 5,07 et 5,26 : $CO_2CH_2$Φ et O-$CH_2$-O ; 4,94 (s) : $H_6$ ; 5,04 (m) : $H_7$ ; 6,00 à 6,20 : =CH-$CH_2$-$N^{\oplus}$ ; 5,99 (s) dédoublé : Φ-C<u>H</u>-O ; 6,38 (m)-6,55 (m) : =C<u>H</u>-$CH_2$ (ΔE) ; 6,75 (s) dédoublé : $H_5$ thiazole ; 6,85 à 7,40 : aromatiques et =C-C<u>H</u>=CH-$CH_2$ ; 7,91 (m)-8,08 à 8,26 (m)-8,42 (m)-8,95 (m)-10,49 (d) : quinoléine et H mobile.

**Stade B : Sel interne de (6R-(3-(E) 6alpha, 7béta-(Z))) 1-(3 (7-(((2-amino 4-thiazolyl) ((carboxy (3-chloro 4,5-dihydroxy phényl) méthoxy) imino) acétyl) amino) 2-carboxy 8-oxo 5-thi 1-azabicyclo[4,2,0]oct-2-èn-3-yl) 2-propényl) quinoléinium**

On opère comme au stade I de l'exemple 1, à partir de 384 mg du produit obtenu au stade précédent. On obtient 192 mg du produit recherché.
<u>Spectre RMN</u> : DMSO 300 MHz
3,35 à 3,70 : $CH_2S$ ; 5,11 (d,J = 5)-5,15 (dJ = 5) : $H_6$ ; 5,3 (o)-5,34 (s) :

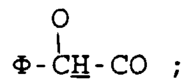

5,74 (m) : $H_7$ ; 5,89 (m) : =CH-C<u>H</u>$_2$-N$^{\oplus}$ ; 6,36 (m) : =C<u>H</u>-$CH_2$ ; 6,75 à 7,00 (m) : autre CH=, aromatique et $H_5$ thiazole ; 7,33 : H mobiles, 8,07-8,26-8,52-9,33 : quinoléine ; 9,57-9,29-9,60-9,91 (m) 13,00 (eq)-13,70 (m) : H mobiles.

**<u>EXEMPLE 37</u> : Sel interne de (6R-(3-(E) 6alpha, 7béta-(Z))) 1-(3-(7-(((2-amino 4-thiazolyl) ((carboxy (3,4-dihydroxy 2-fluorophényl) méthoxy) imino) acétyl) amino) 2-carboxy 8-oxo 5-thia 1-azabicyclo[4,2,0]oct-2-èn-3-yl) 2-propényl) quinoléinium**

**Stade A : Acide 1α-(hydroxy) [2-fluoro (3,4bis-[(2-méthoxy éthoxy) méthoxy] phényl acétique**

On opère comme au stade A de l'exemple 1, à partir de 15,5 g de 2-fluoro 3,4bis-[(2-méthoxy éthoxy] méthoxy] benzaldéhyde, préparation donnée ci-après. On obtient 20,7 g du produit recherché utilisé tel quel pour le stade suivant.

**Stade B : [2-fluoro (3,4bis-[(2-méthoxy éthoxy) méthoxy] phényl] hydroxy acétate de diphényl méthyle**

On opère comme au stade B de l'exemple 1, à partir de 20,6 g du produit obtenu au stade A ci-dessus, en utilisant 9,08 g de diphényl diazométhane. Après chromatographie sur silice (éluant cyclohexane-acétate d'éthyle (7-3)), on obtient 3,2 g du produit recherché.

| Spectre IR : $CHCl_3$ | |
|---|---|
| OH | 3600 $cm^{-1}$ et 3530 $cm^{-1}$ |
| C=O | 1733 $cm^{-1}$ |
| Aromatiques | 1620 - 1603 - 1588 - 1498 $cm^{-1}$ |

**Stade C : [2-fluoro (3,4bis-1(2-méthoxy éthoxy) méthoxy] phényl] phtalimidoxy acétate de diphényl méthyle**

On opère comme au stade C de l'exemple 1 à partir de 3,2 g du produit obtenu ci-dessus. Après chromatographie sur silice, (éluant : chlorure de méthylène-acétone (95-5)), on obtient 2,9 g du produit recherché.

| Spectre IR : $CHCl_3$ | |
|---|---|
| C=O | 1794 - 1754 - 1737 $cm^{-1}$ |
| Aromatiques | 1619 - 1597 - 1498 $cm^{-1}$ |

**Stade D : Aminoxy [2-fluoro (3,4bis-[(2-méthoxy éthoxy) méthoxy] phényl] acétate de diphényl phényl**

On opère comme au stade D de l'exemple 1, on obtient 1,05 g du produit recherché.

| Spectre IR : $CHCl_3$ | |
|---|---|
| O-$NH_2$ | 3340 $cm^{-1}$ |
| C=O | 1744 $cm^{-1}$ |
| $NH_2$ def + Aromatique | 1620 - 1580 - 1498 $cm^{-1}$ |

**Stade E : Acide [[[1-[2-fluoro (3,4bis-[(2-méthoxy éthoxy) méthoxy] phényl] 2-oxo 2-(diphénylméthaxy) éthyl] oxy] imino] [2-[(triphényl) amino] thiazol 4-yl acétique**

On opère comme au stade E de l'exemple 1, à partir de 1,05 g du produit obtenu ci-dessus, en utilisant 0,777 g d'acide oxo-[2-[(triphénylméthyl] amino] thiazol-4-yl acétique (demande belge n° 864 828), on obtient 1,74 g du produit recherché utilisé tel quel pour le stade suivant.

**Stade F : 7béta-[[[[[1-[2-fluoro (3,4bis-[(2-méthoxy éthoxy) méthoxy] phényl] 2-oxo 2-(diphénylméthaxy) éthyl] oxy] imino] [2-[(triphénylméthyl) amino] thiazol-4-yl] acétyl] amino] 3-[(Z) 3-chloro 1-propényl 8-oxo 5-thia 1-azabicyclo[4,2,0]oct-2-èn-2-carbaxylate de 4-méthoxy benzyle**

On opère comme au stade F de l'exemple 1, à partir de 1,74 g du produit obtenu ci-dessus, en utilisant 0,784 g de chlorhydrate de 7béta amino 3-[(Z) 3-chloro 1-propényl] 8-oxo 5-thia 1-azabicyclo[4,2,0]oct-2-èn-2-carboxylate de 4-méthoxy benzyle, on obtient, après chromatographie sur silice (éluant chlorure de méthylène-acétate d'éthyle (8-2)), 1,77 g du produit recherché utilisé tel quel pour le stade suivant.

**Stade G : 7béta-[[[[[1-[2-fluoro (3,4bis-[(2-méthoxy éthoxy) méthoxy] phényl] 2-oxo 2-(diphénylméthaxy) éthyl] oxy] imino] [2-[(triphénylméthyl) amino] thiazol-4-yl] acétyl] amino] 3-[(Z) 3-iodo 1-propényl 8-oxo 5-thia 1-aza-bicyclo[4,2,0]oct-2-èn-2-carboxylate de 4-méthoxy benzyle**

On opère comme au stade G de l'exemple 1, à partir de 840 mg du produit obtenu ci-dessus, on obtient 814 mg du produit recherché. Rf 0,45 ($CH_2Cl_2$-ACOEt (8-2)).

**Stade H : Iodure de 1-[3-[7béta-[[[[[1-[2-fluoro (3,4bis-[(2-méthoxy éthoxy) méthoxy] phényl] 2-oxo 2-(diphénylméthoxy) éthyl] oxy] imino] [2-[(triphénylméthyl) amino] thiazol-4-yl] acétyl] amino] 2-[(4-méthoxy benzyloxy) carbonyl] 8-oxo 5-thia 1-azabicyclo[4,2,0]oct-2-èn-3-yl] 2-propényl quinoléinium**

On opère comme au stade H de l'exemple 1, à partir de 814 mg du produit obtenu ci-dessus, en utilisant 0,337 $cm^3$ de quinoléine. Après chromatographie sur silice (éluant chlorure de méthylène-méthanol (98-2), on obtient 368 mg de produit attendu.
<u>Spectre RMN</u> : $CDCl_3$ 300 MHz
3,31 à 3,35 : les O-$CH_3$ ; 3,45 (dJ = 16) : $CH_2$S ; 3,51-3,81-3,94 : les $CH_2$ centraux ; 3,79 (s)-3,80 (s) : les Φ-O-$CH_3$ ; 4,91 (m) : $H_6$ ; 5,15 à 5,30 :

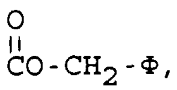

O-$CH_2$-O ; 5,83 : $H_7$ ; 6,05 (ml) : =C-C<u>$H_2$</u>-N$^{\oplus}$ ; 6,31 et 6,35 (m) : les O-C<u>H</u>-Φ ; 6,49 (ml) : -$CH_2$-C<u>H</u>-CH ; 6,72-6,75 : $H_5$ thiazole ; 6,87 à 7,40 : aromatiques, $CO_2$C<u>H</u>-$\Phi_2$, CH=C<u>H</u>-C ; 7,90 à 8,20 : quinoléine, 8,40 (d,d) : $H_4$' ; 8,90 (d,d) : $H_2$'.

**Stade I : Sel interne de (6R-(3-(E) 6alpha, 7béta-(Z))) 1-(3**-(7-(((2-amino 4-thiazolyl) ((carboxy (3,4-dihydroxy 2-fluorophényl) méthoxy) imino) acétyl) amino) 2-carboxy 8-oxo 5-thia 1-azabicyclo[4,2,0]oct-2-èn-3-yl) 2-propényl) quinoléinium

On opère comme au stade I de l'exemple 1, à partir de 357 mg du produit obtenu ci-dessus, on recueille 176,9 mg du produit recherché.
<u>Spectre RMN</u> : DMSO 300 MHz
5,14 : les $H_6$ ; 5,41 (s) dédoublé : O-C<u>H</u>-Φ ; - 5,70 à 5,90 : $CH_2N^{\Phi}$ et $H_7$ ; 6,54 (dJ = 8,5) et 6,70 à 6,80 : aromatique et $H_5$ thiazole ; 6,37 (m) : -CH=C<u>H</u>-$CH_2$ (ΔE) ; 6,97 (d,d) : -C<u>H</u>=CH-$CH_2$ ; 7,34 (1) : $NH_2$ ; 8,07 à 9,58 : quinoléine ; 9,16-9,49-9,69 : H mobiles.

<u>PREPARATION DE L'EXEMPLE 37</u> **: 2-fluoro 3,4bis-[(2-méthoxy** éthoxy) méthoxy] benzaldéhyde

Stade A : 2-fluoro 3,4bis-dihydroxy benzaldéhyde

On agite pendant deux heures à 80°C, un mélange de 74,3 g d'hexaméthylène tétramine et 125 $cm^3$ d'acide trifluoroacétique, on ajoute ensuite une solution de 34 g de 3-fluorocatéchol dans 130 $cm^3$ d'acide trifluoroacétique et poursuit l'agitation pendant 2 heures à 80°C et 16 heures à température ambiante. On distille l'acide trifluoroacétique sous pression réduite, reprend le résidu à l'eau et neutralise par addition de carbonate de potassium jusqu'à pH 7. On filtre, extrait à l'éther, sèche et évapore à sec sous pression réduite, on obtient 39 g de produit utilisé tel quel pour le stade suivant.

Stade B : 2-fluoro 3,4bis-[(2-méthoxy éthoxy) méthoxy] benzaldéhyde

A une solution de 39 g du produit obtenu ci-dessus, dans 390 $cm^3$ d'acétonitrile, on ajoute 261 $cm^3$ de N-éthyl diisopropylamine, on refroidit à -5°C et ajoute lentement 113 $cm^3$ de chlorure de 2-méthoxyéthoxy) méthyle, on agite 16 heures à -5°C. On évapore l'acétonitrile, reprend avec 250 $cm^3$ de chlorure de méthylène puis lave avec de l'acide chlorhydrique N, à l'eau, avec de la soude N puis à l'eau, on sèche, filtre et évapore à sec sous pression réduite. On recueille 49,5 g de produit que l'on chromatographie sur silice (éluant chlorure de méthylène-acétone (96-4)) on obtient 10,4 g du produit recherché.
Spectre RMN : $CDCl_3$ 200 MHz
3,37-3,38 (s) : les $OCH_3$ ; 5,24 (s)-5,37 (s) : les O-$CH_2$-O ; 3,56 (m)-3,83 (m)-4,00 (m) : les $CH_2$ centraux ; 10,26 (s) : CHO ; 7,09 (dd J = 1,5-9) : $H_4$ ; 7,59 (dd J = 7,5-9) : $H_3$.

**EXEMPLE 38 : Sel interne de (6R-(3-(E) 6alpha, 7béta-(Z))) 1**-(3-(7-(((2-amino 4-thiazolyl) ((carboxy (3,4-dihydroxy 2-fluorophényl) méthoxy) imino) acétyl) amino) 2-carboxy 8-oxo 5-thia 1-azabicyclo[4,2,0]oct-2-èn-3-yl) 2-propényl) imidazo(1,2-a) pyridinium

Stade A : Iodure de 1-[3-[7béta-[[[[1-[2-fluoro (3,4bis-[(2-méthoxy éthoxy) méthoxy] phényl] 2-oxo 2-(diphénylméthoxy) éthyl] oxy] imino] [2-[(triphénylméthyl) amino] thiazol-4-yl] acétyl] amino] 2-[(4-méthoxy benzyloxy) carbonyl] 8-oxo 5-thia 1-azabicyclo[4,2,0]oct-2-èn-3-yl] 2-propényl imidazo(1,2-a) pyridinium

On opère comme au stade H de l'exemple 1, à partir de 704,5 g du produit obtenu au stade G de l'exemple 37, en utilisant 0,25 $cm^3$ d'imidazo(1,2-a)pyridine. Après chromatographie sur silice (éluant chlorure de méthylène-méthanol (95-5)), on obtient 275 mg du produit attendu.
Spectre RMN : $CDCl_3$ 400 MHz
3,32-3,33 (s) : les $OCH_3$ ; 3,46-3,47 (d) : $CH_2S$ ; 3,78 (m) : les Φ-O-$CH_3$ ; 3,53-3,81-3,95 : les $CH_2$ centraux ; 4,82 : $H_6$ ; 5,78 : $H_7$ ; 5,00 à 5,32 : $OCH_2O$-, $CO_2CH_2Φ$, =CH-$CH_2N^⊕$ ; 5,40 : l'autre H de $CH_2N^⊕$ ; 6,32-6,37 (s) : O-CH-Φ ; 6,28 (m) : $CH_2$-C<u>H</u>=CH (ΔE) ; 6,74 (s)-6,76 (s) : $H_5$ thiazole ; 6,8 à 7,40 : Φ-C, COC<u>H</u>$_2$-$Φ_2$, aromatique, =CH-C<u>H</u>=CH (ΔE) ; 7,84 (m) à 9,13 (5H) imidazo pyridinium.

**Stade B : Sel interne de (6R-(3-(E) 6alpha, 7béta-(Z))) 1-(3-(7-(((2-amino 4-thiazolyl) ((carboxy (3,4-dihydroxy 2-fluorophényl) méthoxy) imino) acétyl) amino) 2-carboxy 8-oxo 5-thia 1-azabicyclo[4,2,0]oct-2-èn-3-yl) 2-propényl) imidazo(1,2-a) pyridinium**

On opère comme au stade I de l'exemple 1, à partir de 275 mg de produit obtenu au stade précédent, on obtient 116,2 mg de produit recherché.
Spectre RMN : DMSO 300 MHz
3,60 : $CH_2S$ ; 5,11-5,14 (d) : $H_6$ ; 5,75 (dd)-5,79 (dd) : $H_7$ ; 5,30 (m) : $CH_2N^⊕$ ; 5,64-5,65 (s) : O-C<u>H</u>-Φ ; 6,25 (m) : CH=C<u>H</u>- ; 6,91-6,93 (d) : C<u>H</u>=CH-$CH_2$ ; 6,57-6,74 (m) : $H_5$" et $H_6$" ; 6,77 (s)-6,79 (s) : $H_5$ thiazole ; 7,57-8,05 (dd) : $H_5$' et $H_6$' ; 8,22 (d)-8,98 (d,d) : $H_4$', $H_7$' ; 8,30-8,46 (m) : $H_2$', $H_3$' ; 9,48 (d)-9,55 (d) : CH-NH-C=O ; 7,32-9,13-9,64 (m) : H mobiles.

**EXEMPLE 39 : Sel interne de (6R-(3-(E) 6alpha, 7béta-(Z))) 1-(3-(7-(((2-amino 4-thiazolyl) ((carboxy (2,5-difluoro 3,4-dihydroxyphényl) méthoxy) imino) acétyl) amino) 2-carboxy 8-oxo 5-thia 1-azabicyclo[4,2,0]oct-2-èn-3-yl) 2-propényl) imidazo(1,2-a) pyridinium**

**Stade A : [2,5-difluoro 3,4bis-dihydroxy] phényl hydroxy acétate de diphénylméthyle**

On opère comme au stade B de l'exemple 1, à partir de 3,30 g d'acide [2,5-difluoro 3,4bis-dihydroxy] phényl hydroxy acétique et 2,77 g de diphényl diazométhane. Après chromatographie sur silice (éluant cyclohexane-acétone (6-4)), on obtient 3,75 g du produit recherché.
Spectre IR :

| Absorptions complexes région OH/NH | |
|---|---|
| C=O<br>Aromatique | 1752 $cm^{-1}$<br>1630-1535-1485 $cm^{-1}$ |

**Stade B : [2,5-difluoro 3,4bis-[(2-méthoxy éthoxy) méthoxy) phényl] hydroxy acétate de diphénylméthyle**

A une solution de 4,21 g du produit obtenu comme au stade A, dans 45 $cm^3$ de chlorure de méthylène, on ajoute 2,96 g de diisopropyléthylamine et 10 $cm^3$ de chlorure de méthylène.

On refroidit à -5°C et ajoute lentement 2,71 g de chlorure de méthoxyéthoxyméthyle et 5 $cm^3$ de chlorure de méthylène. On agite 30 minutes à -5°C et verse sur de l'acide chlorhydrique 0,1N (30 $cm^3$) on décante et lave avec une solution saturée d'hydrogénocarbonate de sodium (pH 8), sèche, filtre et concentre à sec sous pression réduite. On obtient 6,9 g de produit que l'on chromatographie sur silice (éluant chlorure de méthylène-acétate d'éthyle (85-15)), on obtient 3,577 g du produit recherché.
Spectre IR : $CHCl_3$
~ 3600 $cm^{-1}$ (f) - 3530 $cm^{-1}$ complexe : OH
1734 $cm^{-1}$ : C=O
1624-1600-1589-1492 $cm^{-1}$ : aromatique.
Spectre RMN $^{19}F$

- 140,6 $F^{2,}$ $J_{F1^{-}F4}$ = 14, $J_{F1H5}$ = 6,5
- 135,2 $F^5$, $J_{F4-H5}$ = 11.

**Stade C : 2,5-difluoro 3,4bis-[(2-méthoxy éthoxy) méthoxy) phényl] phtalimidoxy acétate de diphénylméthyle**

On opère comme au stade C de l'exemple 1, à partir de 4,419 g de produit obtenu comme ci-dessus, en utilisant 1,41 g d'hydroxyphatlimide et 4,12 g de triphénylphosphine. Après chromatographie sur silice (éluant chlorure de méthylène-acétone (97-3) puis cyclohexane-acétate d'éthyle (1-1)), on obtient 2,56 g du produit recherché.
Spectre RMN : $CDCl_3$ 300 MHz
3,33 (s)-3,36 (s) : $OCH_3$ ; 3,50 (m)-3,91 (m) : O-$CH_2$-$CH_2$-O ; 5,20 (système A,B)-5,26 (système A,B) : $OCH_2O$ ; 6,26 (s) :

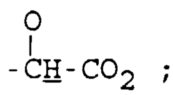

6,94 (s) : $CO_2CH$ $Ph_2$- ; 7,15-7,3 (m)-7,76 (m) : H aromatiques.

**Stade D : aminoxy [2,5-difluoro 3,4bis-[(2-méthaxy éthoxy) méthoxy) phényl] acétate de diphénylméthyle**

On opère comme au stade D de l'exemple 1, à partir de 2,537 g du produit obtenu ci-dessus, en utilisant 0,21 $cm^3$ d'hydrate d'hydrazine. Après chromatographie sur silice (éluant cyclohexane-acétate d'éthyle (1-1)) on obtient 1,67 g du produit recherché.

| Spectre RMN $^{19}F$ : $CDCl_3$ | |
|---|---|
| 135,5 (dd) | $F_5$ |
| 139,8 (dd) | $F_2$ |

$J_{F5-F2}$ = 14
$J_{F5-H6}$ = 10,5
$J_{F2-H6}$ = 6

**Stade E : Acide [[[1-[2,5-difluoro 3,4bis-[(2-méthoxy éthoxy) méthoxy) phényl] 2-oxo 2-(diphénylméthoxy) éthyl] oxy] imino] [2-[(triphénylméthyl) amino] thiazol-4-yl] acétique**

On opère comme au stade E de l'exemple 1, à partir de 909 mg du produit obtenu au stade précédent en utilisant 652 mg d'acide oxo [2-[(triphénylméthyl) amino] thiazol] 4-yl] acétique (décrit dans la demande belge n° 864 828). Après chromatographie sur silice (éluant chlorure de méthylène-méthanol (96-4)) on obtient 1,138 g du produit recherché.

| Spectre IR : | |
|---|---|
| =C-NH + absorption générale OH/NH | ~ 3405 $cm^{-1}$ |
| C=O C=N | 1740 - 1727 $cm^{-1}$ |
| Aromatique + Hétéroaromatique | 1615 - 1597 - 1526 - 1495 $cm^{-1}$ |

**Stade F : 7béta-[[[[[1-[2,5-difluoro 3,4bis-[(2-méthoxy éthoxy) méthoxy] phényl] 2-oxo 2-(diphénylméthoxy) éthyl] oxy] imino] [2-[(triphénylméthyl) amino] thiazol-4-yl] acétyl] amino] 3-[(Z) 3-chloro 1-propényl] 8-oxo 5-thia 1-azabicyclo[4,2,0]oct-2-èn-2-carboxylate de 4-méthoxy benzyle**

On opère comme au stade F de l'exemple 1, à partir de 1,12 g du produit obtenu ci-dessus. Après chromatographie sur silice (éluant chlorure de méthylène-acétate d'éthyle (9-1)), on obtient 979 mg du produit attendu.
Spectre RMN : $CDCl_3$ 400 MHz
3,08-3,20-3,40 : $CH_2S$ ; 3,34-3,35 (s) : les C-O-$CH_3$ ; 3,54-3,92 (m) : les $CH_2$ centraux ; 3,80-3,81 (s) : Ar-O-$CH_3$ ; 3,70-4,00 : CH=C$\underline{H}_2$Cl ; 4,95-5,03 : $H_6$ céphalo ; 5,16-5,25 : OC$\underline{H}_2$O, $CO_2$C$\underline{H}_2$Ar ; 5,75 (m) : =C$\underline{H}$-$CH_2$ (ΔZ) ; 5,85-6,00 : $H_7$ céphalo, =CH-$CH_2$ ; 6,26 (d,J = 11)-6,35 (m) : C-C$\underline{H}$=CH-$CH_2$Cl, ArCHO ; 6,70-6,78 (m) : $H_5$ thiazole ; ~ 7,00 à 7,35 : H aromatiques ; 7,80-8,30 : N$\underline{H}$-CH.

**Stade G : 7béta-[[[[[1-[2,5-difluoro 3,4bis-[(2-méthoxy éthoxy) méthoxy] phényl] 2-oxo 2-(diphénylméthoxy) éthyl] oxy] imino] [2-[(triphénylméthyl) amino] thiazol-4-yl] acétyl] amino] 3-[(Z) 3-iodo 1-propényl] 8-oxo 5-thia 1-azabicyclo[4,2,0]oct-2-èn-2-carboxylate de 4-méthoxy benzyle**

On opère comme au stade G de l'exemple 1, à partir de 969 mg du produit obtenu ci-dessus. On obtient 725 mg du produit attendu.

**Stade H : Iodure de 1-[3-[7béta-[[[[[1-[2,5-difluoro 3,4bis-[(2-méthoxy éthoxy) méthoxy] phényl] 2-oxo 2-(diphénylméthoxy) éthyl] oxy] imino] [2-[(triphénylméthyl) amino] thiazol-4-yl] acétyl] amino] 2-[(4-méthoxy benzyloxy) carbonyl] 8-oxo 5-thia 1-azabicyclo[4,2,0]oct-2-èn-3-yl]2-propényl] imidazo(1,2-a)pyridinium**

On opère comme au stade H de l'exemple 1, à partir de 717 mg du produit obtenu ci-dessus, en utilisant 313 mg d'imidazo(1,2-a) pyridine. Après chromatographie sur silice (éluant chlorure de méthylène-méthanol (95-5)), on obtient 350 mg de produit recherché.
Spectre RMN : $CDCl_3$ 400 MHz
3,32-3,33-3,34 (s) : C-O-$CH_3$ ; 3,53-3,92 (m) : $CH_2$ centraux ; 3,30-3,80 : $CH_2S$ ; 3,79-3,80 (s) : Ar-O-C$\underline{H}_3$ ; 4,94 (dd) : $H_6$ céphalo ; 5,80-5,88 (d) après échange : $H_7$ céphalo ; O (td)-6,31 (td) : =C$\underline{H}$-$CH_2N^{\oplus}$ ; 6,31 (s)-6,37 (s) : Ar-C$\underline{H}$-$CO_2$ ; 7,84-8,18 (d) : CO-NH-CH ; 7,86-8,02 (m) : hétérocycle ; 8,32 à 9,18 : imidazopyridine ; 6,75-7,45 (m) : aromatique, $H_5$ thiazole, C$\underline{H}$=CH-$CH_2$, $CO_2$-C$\underline{H}$-$\Phi_2$ ; 5,18-5,30-5,43 : C$\underline{H}_2$-$N^{\oplus}$, $OCH_2O$,

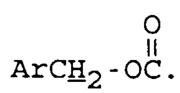

**Stade I : Sel interne de (6R-(3-(E) 6alpha, 7béta-(Z))) 1-(3-(7-(((2-amino 4-thiazolyl) ((carboxy (2,5-difluoro 3,4-dihydroxyphényl) méthoxy) imino) acétyl) amino) 2-carboxy 8-oxo 5-thia 1-azabicyclo[4,2,0]oct-2-èn-3-yl) 2-propényl)imidazo(1,2-a) pyridinium**

On opère comme au stade I de l'exemple 1, à partir de 344 mg du produit obtenu ci-dessus. On recueille 170 mg du produit attendu.
Spectre RMN : DMSO 300 MHz
5,16 (dd) : $H_6$ céphalo ; 5,77 (m) : $H_7$ céphalo ; 5,29 (m) : C$\underline{H}_2$-$N^{\oplus}$ ; 5,64 (s,d) :

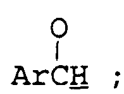

6,70 (dd) : H aromatique couplé à 2F ; 6,80 (s,d) : $H_5$ thiazole ; 6,87 (d,d) : C<u>H</u>=CH-$CH_2$ (ΔE) : 7,33 (sl) : $NH_2$ ; 8,28-8,44 (m) : $H_2$' et $H_3$' ; 7,18 à 8,96 (4H) imidazopyridine ; 9,03-9,63 (d) : C-N<u>H</u>-CH ; 9,84 autres H mobiles.

**<u>PREPARATION DE L'EXEMPLE 39</u> : Acide 2,5-difluoro 3,4-dihydroxy] phényl hydroxy acétique**

**Stade A : 2,5-difluorophénol**

A une solution de 62,19 g de 1,4-difluorobenzène dans 500 $cm^3$ de tétrahydrofuranne, on ajoute à -65°C, 325 $cm^3$ de n-butyl lithium en solution 1,6 M dans l'hexane. On agite 2 heures 30 puis ajoute en 30 minutes à -65°C une solution de 53,8 g de triméthylborate dans 250 $cm^3$ d'éther. On agite 1 heure 30 en laissant la température remonter à -15°C. On agite 15 minutes et ajoute 350 $cm^3$ d'acide chlorhydrique à 10 %. On décante, lave à l'eau, sèche et évapore à sec sous pression réduite. On obtient : 63,5 g de produit que l'on reprend avec-400 $cm^3$ de toluène. On chauffe jusqu'à 108°C et ajoute goutte à goutte, 180 $cm^3$ d'eau oxygénée à 30 %, on chauffe 2 heures à 90°C, on laisse refroidir et filtre. On décante le filtrat, lave la phase organique à l'eau puis avec une solution de sulfate d'ammonium ferreux à 10 % puis à l'eau, on extrait la phase organique avec 2 fois 300 $cm^3$ de soude 2N. On acidifie avec de l'acide chlorhydrique concentré, extrait avec du chlorure de méthylène, sèche et évapore à sec sous pression réduite.
<u>Spectre RMN</u> : $CDCl_3$
5,21 (sl) : O<u>H</u> ; 6,55 (dddd) : $H_4$, $JH_4$-$H_2$ = 9, $JH_4$-$F_2$ = 3,5, $JH_4$-$H_6$ = 3, $JH_4F_5$ = 8 ; 6,74 (ddd) : $H_6$, $JH_6$-$H_4$ = 3, $JH_6$-$F_2$ = 7 ; 7,01 (ddd) : $H_3$, $JH_3H_4$ = 9, $JH_3$-$F_2$ = 10, $JH_3$-$F_5$ = 5.

**Stade B : 2,5-difluoroanisole**

On chauffe pendant 3 heures 30 un mélange de 35,0 g du produit obtenu ci-dessus, 350 $cm^3$ d'acétone, 44,6 g de carbonate de potassium et 40,7 g de sulfate de diméthyle (neutralisé sur hydrogénocarbonate de potassium). On laisse revenir à température ambiante, ajoute de l'eau, extrait avec de l'éther, lave, sèche et concentre à sec sous pression réduite, on obtient 39,8 g de produit recherché.

| <u>Spectre RMN</u> : $^{19}F$ $CDCl_3$ 188 MHz | | |
|---|---|---|
| 119,1 (dddd)<br>144,1 (dddd)<br>$J_{F5-H3}$ = 5<br>$J_{F5-H4}$ = 8<br>$J_{F5-H6}$ = 10 | $F_5$<br>$F_2$ | <br>$J_{F2-F5}$ = 15<br>$J_{F2-H3}$ = 10,5<br>$J_{F2-H4}$ = 3,5<br>$J_{F2-H6}$ = 7 |

**Stade C : 3,6-difluoroguaiacol**

On opère comme au stade A ci-dessus, à partir de 55,15 g du produit obtenu au stade B ci-dessus, en utilisant 220 $cm^3$ de n-butyl lithium, 1,6 M dans l'hexane, 36,4 g de borate de triméthyle et 200 $cm^3$ d'eau oxygénée à 30 %. On obtient 44,7 g du produit recherché.

| Spectre RMN : $CDCl_3$ 200 MHz | |
|---|---|
| 4,02 (d, J = 2) | $OCH_3$ |
| 5,56 (sl) | OH |
| 6,57 (ddd) | $H_4$ |
| 6,75 (dt) | $H_5$ |

$J_{H5-H4}$ = 9,5 ; $J_{H5-F3}$ = 5 ; $J_{H5-F6}$ = 9,5 ; $J_{H4-F3}$ = 10,5 ; $J_{H4-F6}$ = 5.

**Stade D : 3,6-difluorocatéchol**

On opère comme au stade B de la préparation 2 à partir de 21,15 g du produit obtenu au stade C ci-dessus, en utilisant 260 $cm^3$ d'une solution molaire de tribromure de bore. On obtient 17,62 g du produit recherché.

**Stade E : Acide 2,5-difluoro 3,4-dihydroxyl phényl hydroxy acétique**

A une solution de 11,7 g du produit obtenu au stade ci-dessus et 7,37 g d'acide glyoxylique monohydraté dans 40 $cm^3$ d'eau, on ajoute à +10°C, 7,69 g de soude en solution dans 80 $cm^3$ d'eau. Agite 30 minutes à 10°C puis 3 heures 30 à température ambiante. On ajoute 0,74 g d'acide glyoxylique monohydraté et agite 1 heure à température ambiante. On ajoute de l'acide chlorhydrique concentré jusqu'à pH 1. On extrait avec de l'acétate d'éthyle, sèche, filtre et concentre à sec sous pression réduite. On obtient 17,0 g de produit recherché.
Spectre IR : Nujol

| Absorption complexe région OH/NH | |
|---|---|
| C=O<br>Aromatique | 1700 $cm^{-1}$<br>1640 - 1612 - 1526 - 1488 $cm^{-1}$ |

**EXEMPLE 40 : Sel interne de (6R-(3-(E) 6alpha, 7béta-(Z))) 1-(3-(7-(((2-amino 4-thiazolyl) ((carboxy (2-chloro 4,5-dihydroxy 3-méthoxy phényl) méthoxy) imino) acétyl) amino) 2-carboxy 8-oxo 5-thia 1-azabicyclo[4,2,0] oct-2-èn-3-yl) 2-propényl) imidazo(1,2-a) pyridinium**

**Stade A : [2-chloro 4,5bis-[(2-méthoxy éthoxy) méthoxy] 3-méthoxy] benzaldéhyde et 2-chloro 3,4bis-[(2-méthoxy éthoxy) méthoxy] 5-méthoxy benzaldéhyde**

On agite 2,17 g de 3,4bis-[(2-méthoxy éthoxy) méthoxy] 5-méthoxy benzaldéhyde avec 21,7 $cm^3$ de chlorure de méthylène et 4 $cm^3$ d'une solution de 1,76 g d'hypochlorite de calcium (à 65 %) dans 10 $cm^3$ d'eau, on agite à 0/+5°C, pendant 20 minutes, extrait avec du chlorure de méthylène, lave à l'eau, sèche et évapore à sec sous pression réduite. On obtient 2,34 g de mélange d'isomères (7/3).
Spectre RMN : $CDCl_3$ 250 MHz
3,37 : les $OCH_3$ ; 3,7 à 4,1 : les $CH_2$ centraux ; 5,29 à 5,33 : $OCH_3$ ; 3,91 : $OCH_3$ ; 7,54 et 7,30 : $H_6$ ; 10,38 et 10,40 : CHO.

**Stade B : [2-chloro 4,5bis-[(2-méthoxy éthoxy) méthoxy] 3-méthoxy] styryle carboxylate et isomère 5-méthoxy correspondant**

A un mélange, agité à +5/+10°C, de 2,162 g de bromure de lithium avec 60 $cm^3$ de tétrahydrofuranne et 4,03 $cm^3$ de triphénylphosphoroacétate, on ajoute 2,97 $cm^3$ de triéthylamine puis, une solution de 6,72 g du mélange d'isomères obtenus au stade précédent, en solution dans 60 $cm^3$ de tétrahydrofuranne. On agite 16 heures à température ambiante, on concentre à sec sous pression réduite. On reprend le résidu avec du chlorure de méthylène, lave avec de l'acide chlorhydrique 1N puis à l'eau, sèche et évapore à sec sous pression réduite, on obtient 8,8 g du produit attendu (mélange de 2 isomères # 7/3).
Spectre RMN : $CDCl_3$ 250 MHz
1,35 (t,d) 4,28 (q,d) : $CO_2Et$ ; 3,37-3,38 : les $OCH_3$ ; 3,57 (m)-3,84 à 4,04 (m) : les $CH_2$ centraux ; 3,88 (s)-3,86 (s) : $OCH_3$ ; 5,24 à 5,28 : les $OCH_2$ ; 6,34 (d)-6,38 (d) : $CH=C\underline{H}-CO_2$ ; 8,03-8,05 (d) : $-C\underline{H}=CH-CO_2$ ; 6,93-7,30 : H aromatique.

**Stade C : [2-chloro 4,5bis-[(2-méthoxy éthoxy) méthoxy] 3-méthoxy] styrole et isomère 5-méthoxy correspondant**

A une solution refroidie à -70°C de 2,83 g du mélange d'isomères, obtenu au stade précédent, dans 20 $cm^3$ de chlorure de méthylène, on ajoute 11,7 $cm^3$ d'une solution molaire d'hydrure de diisobutyl aluminium dans l'hexane. On agite 20 minutes à -70°C et on ajoute 2 $cm^3$ d'eau. On agite 30 minutes à 20°C, sèche, filtre et amène à sec sous pression réduite, on obtient 1,95 g du produit recherché (mélange d'isomères 7/3).
Spectre RMN : $CDCl_3$ 250 MHz
3,37-3,38 : les $OCH_3$ ; 3,54-3,84-3,97 (m) : les $CH_2$ centraux ; 3,87 (s) : $OCH_3$ (en 3) ; 4,34 (d) : $-CH_2-OH$ ; 6,29 (t) : $CH=C\underline{H}-CH_2$ ; 6,93 (d) : $-C\underline{H}=CH-CH_2$ ; 7,19 (s) : H aromatique.

**Stade D : [2-chloro 4,5bis-[(2-méthoxy éthoxy) méthoxy] 3-méthoxy] (1,2-époxy) styrole (isomère A) et [2-chloro 3,4bis-[(2-méthoxy éthoxy) méthoxy] 5-méthoxy] (1,2-époxy) styrole (isomère B)**

A une solution de 4,78 g du mélange d'isomères obtenu au stade C, dans 50 $cm^3$ de chlorure de méthylène,

on ajoute, à +5°C, une solution de 2,79 g d'acide métachloroperbenzoïque dans 50 $cm^3$ de chlorure de méthylène. On agite 16 heures à 20°C et ajoute 20 $cm^3$ d'une solution saturée d'hydrogéno carbonate de sodium et 50 $cm^3$ de chlorure de méthylène, décante, lave à l'eau, sèche et évapore à sec sous pression réduite. On chromatographie le résidu sur silice (éluant chlorure de méthylène-acétone 85-15), on obtient 2,12 g du produit isomère A et 716 mg de l'isomère B.
Spectre RMN : $CDCl_3$ 250 MHz 3,07 (t,d) :

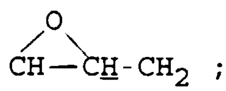

3,37-3,38 (s) : les $OCH_3$ ; 3,57- 3,83-3,97 (m) : les $CH_2$ centraux ; 3,88 (s) : $OCH_3$ (en 3) ; 3,83-4,06 (ddd) : -$C\underline{H}_2OH$ ; 4,19 (d,J = 2) :

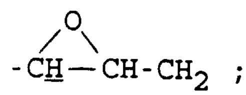

5,2-5,26 (s) : les $OCH_2$ ; 1,94 (d,d,J = 5,5-7,5) : OH.

**Stade E : [[2-chloro 4,5bis-[(2-méthoxy éthoxy) méthoxy] 3-méthoxy] phényl] chloro acétate de diphényl méthyle**

**a)** Ouverture de l'époxyde

A une solution préparée à +5°C de 1,306 g de chlorure cuivrique 20 $cm^3$ de tétrahydrofuranne et 1 g de chlorure de lithium, on agite cinq minutes puis, ajoute à 20°C, 2,055 g de l'époxyde isomère A (obtenu au stade D ci-dessus) en solution dans 10 $cm^3$ de tétrahydrofuranne, on agite 5 heures à 20°C, ajoute 10 $cm^3$ d'eau décante, lave, sèche et évapore à sec sous pression réduite. On obtient 2,09 g de diol intermédiaire.

**b)** Oxydation

A une solution de 2,035 du diol ci-dessus avec 6 $cm^3$ de tétrachlorure de carbone et 6 $cm^3$ d'acétonitrile, on ajoute à +5°C/+10°C, 9 $cm^3$ d'eau et 3,79 g de périodate de sodium et 50 mg de chlorure de ruthénium hydraté (à 35/40 % en Ru) on agite 1 heure à 20°C, on ajoute 20 $cm^3$ de chlorure de méthylène, 10 $cm^3$ d'eau, décante, lave, sèche et évapore à sec sous pression réduite. On obtient 1,8 g d'acide intermédiaire.

**c)** Estérification :

A une solution de 1,8 g de produit ci-dessus, dans 20 $cm^3$ de chlorure de méthylène, on ajoute 790 mg de diphényl diazométhane. On agite 1 heure à 20°C, et concentre à sec sous pression réduite. Après chromatographie sur silice (éluant chlorure de méthylène (9-1)), on obtient 1,525 g du produit recherché.
Spectre RMN : $CDCl_3$ 250 MHz
3,34-3,37 : les $OCH_3$ ; 3,47-3,57-3,74-3,97 (m) : les $CH_2$ centraux ; 3,87 (s) : 3-méthoxy ; 5,12-5,24 : les $OCH_2$ ; 5,94 :

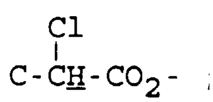

6,89 (s) : $CO_2$-$C\underline{H}$

**Stade F : Aminoxy [[2-chloro 4,5bis-[(2-méthoxy éthoxy) méthoxy] 3-méthoxy] phényl acétate de diphényl méthyle**

**a)** Phtalimidoxylation

On agite 5 heures, 1,48 g du produit obtenu au stade précédent, 7,5 $cm^3$ de diméthylformamide avec 363 mg d'acétate de potassium et 604 mg de N-hydroxyphtalimide.

**b)** Hydrazinolyse

On ajoute à 20°C 180 µl d'hydrate d'hydrazine, on agite 20' à 20°C, on sépare le phtalylhydrazide, le lave avec de l'acétonitrile et évapore à sec les fractions organiques. Après chromatographie sur silice (éluant : chlorure de méthylène-acétate d'éthyle (3-1)), on obtient 1,11 g du produit recherché.
Spectre RMN : $CDCl_3$ 250 MHz
3,33-3,37 (s) : les $OCH_3$ ; 3,44-3,57-3,71-3,97 (m) : les $CH_2$ centraux ; 3,88 (s) : 3-méthoxy ; 5,09 et 5,23 : les $OCH_2$ ; 5,73 (s) :

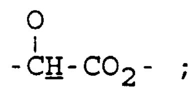

5,84 : $NH_2$ ; 6,91 (s) -6,94 : $CO_2C\underline{H}\text{-}\Phi_2$ ; 7,07 à 7,35 : les aromatiques.

**Stade G : Acide [[[1-[2-chloro 4,5bis-[(2-méthoxy éthoxy) méthoxy] 3-méthoxy] phényl] 2-oxo 2-(diphénylméthoxy) éthyl] oxy] imino] [2-[(triphénylméthyl) amino] thiazol 4-yl] acétique**

On opère comme au stade E de l'exemple 1, à partir de 1,1 g du produit obtenu au stade F ci-dessus, en utilisant 835 mg d'acide oxo [2-[(triphénylméthyl) amino] thiazol-4-yl] acétique (décrit dans la demande belge n° 864828). Le produit n'est pas isolé et traité tel quel pour le stade suivant.

**Stade H : 7béta-[[[[[1-[2-chloro 4,5bis-[(2-méthoxy éthoxy) méthoxy] 3-méthoxy] phényl] 2-oxo 2-(diphénylméthoxy) éthyl] oxy] imino] [2-[(triphénylméthyl) amino] thiazol 4-yl] acétyl] amino] 3-[(Z) 3-chloro 1-propényl] 8-oxo 5-thia 1-azabicyclo[4,2,0]oct-2-èn-2-carboxylate de 4-méthoxy benzyle**

On opère comme au stade F de l'exemple 1, à partir du produit brut obtenu au stade G ci-dessus, en utilisant 781 mg de chlorhydrate de 7béta-amino 3-[(Z) 3-chloro 1-propényl] 8-oxo 5-thia 1-azabicyclo[4,2,0]oct-2-èn-2-carboxylate de 4-méthoxy benzyle (EP 0 333 154). Après chromatographie sur silice, (éluant chlorure de méthylène-acétate d'éthyle (9-1)) on obtient 1,36 g du produit recherché.
Spectre RMN : $CDCl_3$ 300 MHz
3,25-3,29-3,36-3,37-3,81 : les $OCH_3$ ; 3,2 à 4,0 : $-SCH_2-$, $C\text{-}CH_2Cl$, les $CH_2$ centraux ; 4,94 (d) à 5,25 : $CO_2C\underline{H}\Phi_2$, NH-CH-C$\underline{H}$-S, les $OCH_2O$ ; 5,75 (m) : -CH=C$\underline{H}$-$CH_2$ ($\Delta Z$) ; 5,9 (m) : NH-C$\underline{H}$-CH-S ; 6,28 (d) : -C$\underline{H}$=CH-$CH_2$ ($\Delta Z$) ; 6,8 à 7,15 : aromatiques + $H_5$ thiazole.

**Stade I : 7béta-[[[[[1-[2-chloro 4,5bis-[(2-méthoxy éthoxy) méthoxy] 3-méthoxy] phényl] 2-oxo 2-(diphénylméthoxy) éthyl] oxy] imino] [2-[(triphénylméthyl) amino] thiazol 4-yl] acétyl] amino] 3-[(Z) 3-iodo 1-propényl] 8-oxo 5-thia 1-azabicyclo[4,2,0]oct-2-èn-2-carboxylate de 4-méthoxy benzyle**

On opère comme au stade G de l'exemple 1, à partir de 1,33 g du produit obtenu ci-dessus. Après chromatographie sur silice (éluant chlorure de méthylène-acétate d'éthyle (9-1)). On obtient 990 mg du produit recherché.
Spectre RMN : $CDCl_3$ 400 MHz
3,24 (s)-3,26 (s)-3,35 (s)-3,36 (s) : les 2 $OCH_3$ ; 3,2 à 4,0 (m) : -SC$\underline{H}_2$-C et les $CH_2$ centraux ; 3,8 (s,d) 3,85-3,88 (s) : les 2 ≡C-$OCH_3$ ; 4,9 à 5,07 (m) 5,18 à 5,25 (m) : les O-C$\underline{H}_3$ ; 4,97-5,02 (d) : CH-CH-S- ; 5,87 (ddd) NH-CH-CH-S- ; 6,51-6,53 (s) : =NH-O-C$\underline{H}$= ; 6,79-6,80 (s) : $H_5$ thiazole ; 6,84 à 7,37 (m) : aromatiques, autre C$\underline{H}$=C et $CO_2$-C$\underline{H}$-$\Phi_2$ ; 7,78-8,3 (d) :

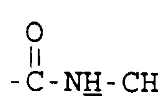

**Stade J : Iodure de 1-[3-[7béta-[[[[1-[2-chloro 4,5bis-[(2-méthoxy éthoxy) méthoxy] 3-méthoxy] phényl] 2-oxo 2-(diphénylméthoxy) éthyl] oxy] imino] [2-[(triphénylméthyl) amino] thiazol-4-yl] acétyl] amino] 2-[(4-méthoxy benzyloxy) carbonyl] 8-oxo 5-thia 1-azabicyclo[4,2,0]oct-2-èn-3-yl]2-propényl] imidazo(1,2-a)pyridinium**

On opère comme au stade H de l'exemple 1, à partir de 735 mg du produit obtenu ci-dessus, en utilisant 260 micro-litres d'imidazo(1,2-a)pyridine, on obtient 750 mg du produit attendu.
Spectre RMN : $CDCl_3$ 300 MHz
3,14 à 3,38 : -S-$CH_2$-C≡ , les -C-O-$CH_3$ ; 3,78-3,79-3,85-3,87 : =C-O$CH_3$ ; 3,3 à 4,0 : les $CH_2$ centraux ; 4,9 à 5,02 : NH-CH-C$\underline{H}$-S- ; 5,8 (m) : =NH-C$\underline{H}$-CH-S ; 6,27 (m) : -CH=C$\underline{H}$-$CH_2$ (ΔE) ; 6,53-6,54 : =N-O-C$\underline{H}$ ; 6,76 à 7,3 : $CO_2$-C$\underline{H}$-$\Phi_2$, -C$\underline{H}$=CH-$CH_2$ et les aromatiques ; 7,6 à 9,10 : imidazopyridine.

**Stade K : Sel interne de (6R-(3-(E) 6alpha, 7béta-(Z))) 1-(3-(7-(((2-amino 4-thiazolyl) ((carboxy (2-chloro 4,5-dihydroxy 3-méthoxy phényl) méthoxy) imino) acétyl) amino) 2-carboxy 8-oxo 5-thia 1-azabicyclo[4,2,0]oct-2-èn-3-yl) 2-propényl) imidazo(1,2-a) pyridinium**

On opère comme au stade I de l'exemple 1, à partir de 750 mg du produit obtenu ci-dessus, on obtient 463 mg du produit recherché.
Spectre RMN : DMSO 300 MHz
5,12 (d,d) : =NH-CH-C$\underline{H}$-S- ; 5,28 à 5,3 : =CH-C$\underline{H_2}$-N$^\oplus$ ; 5,74 (m) : =NH-C$\underline{H}$-CH-S ; 5,75 : =N-O-C$\underline{H}$- ; 6,23 (m) : ≡C-CH=C$\underline{H}$- ; 6,78 (s) : $H_5$ thiazole ; 6,84 (d,J = 15) : ≡C-C$\underline{H}$=CH- (ΔE) ; 7,36 : $NH_2$ ; 7,58 à 8,93 : imidazopyridine ; 9,55 : H mobiles.

**<u>EXEMPLE 41</u> : Sel interne de (6R-(3-(E) 6alpha, 7béta-(Z))) 1-(3-(7-(((2-amino 4-thiazolyl) ((carboxy (2-chloro 3,4-dihydroxy 5-méthoxy phényl) méthoxy) imino) acétyl) amino) 2-carboxy 8-oxo 5-thia 1-azabicyclo[4,2,0] oct-2-èn-3-yl) 2-propényl) imidazo(1,2-a) pyridinium**

En opérant comme aux stades E à K de l'exemple 40, à partir de l'isomère B obtenu au stade D de l'exemple 40, on a obtenu le produit recherché.
Spectre RMN : DMSO 300 MHz
3,5 à 3,7 (m) : S-$CH_2$-C≡ ; 3,73-3,74 (s) : $OCH_3$ ; 5,18 : NH-CH-C$\underline{H}$-S ; 5,29 : -$CH_2N^\oplus$ ; 5,79 (ddd) : NH-C$\underline{H}$-CH- ; 5,83-5,85 (s) : =N-O-CH= ; 6,25 (m) : CH=C$\underline{H}$- ; 6,6 (s)-6,78 (s)-6,82 (s) : aromatique, $H_5$ thiazole ; 6,88 (d,d) J=16) :-C$\underline{H}$=CH-$CH_2$ (ΔE) ; 7,58 à 8,96 (6H) imidazopyridine.

**<u>EXEMPLE 42</u> : Sel interne de (6R-(3-(E) 6alpha, 7béta-(Z))) 1-(3-(7-(((2-amino 4-thiazolyl) ((carboxy (2-chloro 4,5-dihydroxy 3-méthoxy phényl) méthoxy) imino) acétyl) amino) 2-carboxy 8-oxo 5-thia 1-azabicyclo[4,2,0] oct-2-èn-3-yl) 2-propényl) quinolinium**

**Stade A : Iodure de 1-[3-[7béta-[[[[1-[2-chloro 4,5bis-[(2-méthoxy éthoxy) méthoxy] 3-méthoxy] phényl] 2-oxo 2-(diphénylméthoxy) éthyl] oxy] imino] [2-[(triphénylméthyl) amino] thiazol-4-yl] acétyl] amino] 2-[(4-méthoxy benzyloxy) carbonyl] 8-oxo 5-thia 1-azabicyclo[4,2,0]oct-2-èn-3-yl]2-propényl] quinolinium**

On opère comme au stade J de l'exemple 40 à partir de 700 mg du produit obtenu au stade I de l'exemple 40, en utilisant 282 microlitres de quinoléine. On obtient 706 mg du produit recherché.

**Stade B : Sel interne de (6R-(3-(E) 6alpha, 7béta-(Z))) 1-(3-(7-(((2-amino 4-thiazolyl) ((carboxy (2-chloro 4,5-dihydroxy 3-méthoxy phényl) méthoxy) imino) acétyl) amino) 2-carboxy 8-oxo 5-thia 1-azabicyclo[4,2,0]oct-2-èn-3-yl) 2-propényl) quinolinium**

On opère comme au stade K de l'exemple 40, à partir de 690 mg du produit obtenu au stade A ci-dessus. On obtient 434 mg du produit recherché.
Spectre RMN : DMSO 300 MHz
3,4 à 3,8 (m) : S-$CH_2$- ; 3,65-3,73 (s) : ≡C-O$CH_3$ ; 5,1-5,14 (d) : -NH-CH-C$\underline{H}$- ; 5,73 : -NH-C$\underline{H}$-CH- ; 5,75 (s) : =N-O-C$\underline{H}$= ; 5,89 (m) : -CH=CH-C$\underline{H_2}$ ; 6,75-6,76-6,78-6,79 (s) : $H_5$ thiazole, H aromatique ; 6,35 (m) : -CH=C$\underline{H}$-C (ΔE) ; 6,97 (dl J = 16) : -CH=CH-C (ΔE) ; 8,07 à 9,58 (7H) quinoline ; 9,53 : -NH-C$\underline{H}$-CH- ; 7,35-9,24-13,0 : H mobiles.

**<u>EXEMPLE 43</u> : Sel interne de (6R-(3-(E) 6alpha, 7béta-(Z))) 1-(3-(7-(((2-amino 4-thiazolyl) ((carboxy (2-chloro 3,4-dihydroxy 5-méthoxy phényl) méthoxy) imino) acétyl) amino) 2-carboxy 8-oxo 5-thia 1-azabicyclo[4,2,0] oct-2-èn-3-yl) 2-propényl) quinolinium**

**Stade A : Iodure de 1-[3-[7béta-[[[[[1-[2-chloro 3,4-dihydroxy 5-méthoxy] phényl] 2-oxo 2-(diphénylméthoxy) éthyl] oxy] imino] [2-[(triphénylméthyl) amino] thiazol-4-yl] acétyl] amino] 2-[(4-méthoxy benzyloxy) carbonyl] 8-oxo 5-thia 1-azabicyclo[4,2,0]oct-2-èn-3-yl]2-propényl] quinolinium**

On opère comme au stade J de l'exemple 40 à partir de 450 mg du produit obtenu au stade E de l'exemple 41, on obtient 440 Mg du produit recherché.

**Stade B : Sel interne de (6R-(3-(E) 6alpha, 7béta-(Z))) 1-(3-(7-(((2-amino 4-thiazolyl) ((carboxy (2-chloro 3,4-dihydroxy 5-méthoxy phényl) méthoxy) imino) acétyl) amino) 2-carboxy 8-oxo 5-thia 1-azabicyclo[4,2,0]oct-2-èn-3-yl) 2-propényl) quinolinium**

On opère comme au stade K de l'exemple 40, à partir de 440 mg du produit obtenu au stade A ci-dessus, on obtient 256 mg de produit recherché.
<u>Spectre RMN</u> : DMSO 300 MHz
3,48 à 3,84 (m) : S-$CH_2$- ; 3,71-3,73 (s) : =C-$OCH_3$ ; 5,17 (d,d) : -NH-CH-CH-C ; 5,8 (m) : -NH-CH-CH- ; 5,82-5,84 (s) : N-O-CH= ; 5,88 (m) : -CH=CH-C<u>H</u>$_2$- ; 6,38 (d,m) : CH=C<u>H</u>-$CH_2$ ; 6,97 (d,d) dJ = 16) : -C<u>H</u>=CH- (ΔE) ; 6,59 (s)-6,77 (s)-6,81 (s) : $H_5$ thiazole, aromatique ; 8,06 à 9,58 : quinoline (7H) ; 9,53-9,64 (d) : C-NH- ; 7,54 (m)-9,25 (m)-13,70 (m) : H mobiles.

**<u>EXEMPLE 44</u> : Sel interne de (6R-(3-(E) 6alpha, 7béta-(Z(S*)))) 1-(3-(7-(((2-amino 4-thiazolyl) ((carboxy (3,4-dihydroxy 5-fluorophényl) méthoxy) imino) acétyl) amino) 2-carboxy 8-oxo 5-thia 1-azabicyclo[4,2,0]oct-2-èn-3-yl) 2-propényl) quinoléinium**

On chromatographie 10 mg du produit obtenu à l'exemple 3 sur colonne MICROBONDAPACK $C_{18}$ (éluant eau (à pH 2,7 avec de l'acide trifluoro acétique)-acétonitrile (contenant 0,025 % acide trifluoro acétique) 86-14), on obtient 1,5 mg isomère (S) attendu et 1,5 mg isomère (R).
<u>Spectre RMN</u> : DMSO 400 MHz
3,40 à 3,60 (m) : $CH_2S$ ; 5,13 (d,J = 5) : $H_6$ ; 5,77 (dd,J = 5 et 8) : $H_7$ (d, après échange) ; 5,32 (s) Φ-CH-O ; 5,87 (m) : $CH_2N^{\oplus}$ ; 6,31 (m) : CH=C<u>H</u>-$CH_2$ ; 7,00 (d,J = 16) : C<u>H</u>=CH-$CH_2$ ; 6,77 (s) : $H_5$ thiazole ; 6,72 (m) : $H_4$' $H_6$' ; 7,25 (s) : 2H mobiles ; 8,06 (dd, J = 7 et 8) et 8,28 (ddl) J = 7 et 9) : $H_6$", $H_7$" ; 8,22 (dd J = 6 et 8) : $H_3$" ; 8,50 (d, J = 8) et 8,55 (d, J = 9) : $H_4$" et $H_5$" ; 9,33 (d, J = 8) : $H_8$" ; 9,57 (d, J = 6) : $H_2$".

**<u>EXEMPLE 45</u> : Sel interne de (6R-(3-(E) 6alpha, 7béta-(Z(R*)))) 1-(3-(7-(((2-amino 4-thiazolyl) ((carboxy (3,4- dihydroxy 5-fluorophényl) méthoxy) imino) acétyl) amino) 2-carboxy 8-oxo 5-thia 1-azabicyclo[4,2,0]oct-2-èn-3-yl) 2-propényl) quinoléinium**

Isomère R isolé au cours de la chromatographie à l'exemple 44.
<u>Spectre RMN</u> : DMSO 400 MHz
3,39 à 3,75 (m) : $CH_2S$ ; 5,16 (d, J = 5) : $H_6$ ; 5,32 (s) Φ-CH-O ; 5,74 (dd, J = 5 et 8) $H_7$ ; 5,88 (m) : $CH_2N^{\oplus}$ ; 6,36 (dt) : CH=C<u>H</u>-$CH_2$ ; 6,99 (d, J = 16) : C<u>H</u>=CH-$CH_2$ ; 6,73 (m) (3H) : $H_5$ thiazole et $H_2$' et $H_6$' ; 7,26 (s) : 2H mobiles ; 8,07 (t, J = 8) et 8,28 (t, J = 8) : $H_6$" et $H_7$" ; 8,22 (dd, J = 6 et 8) : $H_3$" ; 8,50 (d, J = 8) et 8,55 (d, J = 9) : $H_5$" et $H_4$" ; 9,33 (d, J = 8) : $H_8$" ; 9,59 (d, J = 6) : $H_2$" ; 9,59 (d,m) : CON<u>H</u>-CH ; 9,14 (s) : 1H mobile.

**<u>EXEMPLE 46</u> : Sel interne de [[6R-[3(E), 6alpha, 7béta(Z) (S*)]] 1-[3-[7-[[(2-amino 4-thiazolyl) [[carboxy (2,5-dichloro 3,4-dihydroxy phényl) méthoxy] imino] acétyl] amino] 2-carboxy 8-oxo 5-thia 1-azabicyclo[4,2,0]oct-2-en-3-yl] 2-propènyl] 4-(méthylthio) pyridinium correspondant à la séparation des isomères du produit obtenu à l'exemple 14**

<u>Spectre RMN</u> : DMSO 300 MHz
2,71 (s) : Φ-SMe ; ~ 3,57 : =C-$CH_2S$ ; ~ 5,15 à 5,25 : $H_6$ et $N^{\oplus}$-C<u>H</u>$_2$-CH= ; ~ 5,80 (sl) : O-CH-Φ et $H_7$ ; 6,25 (m) : =C<u>H</u>-$CH_2$ ; ~ 6,98 (d) : =C-C<u>H</u>=CH ; 6,81 (s) : $H_5$ thiazole ; 7,00 : $H_6$ ; 7,32 (1) 2H : $NH_2$ ; 7,96-8,70 (d) : H pyridine ; 9,58 (d) :

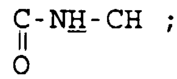

9,84 (s)-9,92 (s) : les OH ; 13,28-13,78 : les autres H mobiles.

**<u>EXEMPLE 47</u> : Sel interne de (6R-(3-(E) 6alpha, 7béta-(Z) (S*))) 1-(3-(7-(((2-amino 4-thiazolyl) ((carboxy (2,5-dichloro 3,4-dihydroxy phényl) méthoxy) imino) acétyl) amino) 2-carboxy 8-oxo 5-thia 1-azabicyclo[4,2,0]oct-2-èn-3-yl) 2-propényl) imidazo(1,2-a) pyridinium**

Spectre RMN : DMSO 300 MHz
5,15 (d,J = 5) : $H_6$ ; 5,29 5(m) : $C\underline{H}_2$-N⊕ ; 5,79 (s) : O-C$\underline{H}$-Φ ; 5,79 (dd, d après éch.) : $H_7$ ; 6,24 (dt,J= 15,5 et 6,5) : =C$\underline{H}$-$CH_2$, ~ 3/4 H ; 6,81 (s)-6,99 (s) : $H_5$ thiazole, 1H aromatique ; 6,86 (d,J= 15,5) =C-C$\underline{H}$=CH ; 7,58 (t)-8,06 (t) : $H_5'$, $H_6'$ ; 8,20 (d) : $H_7'$ ; 8,96 (d) : $H_4'$ ; 8,28 (d)-8,44 (d) : $H_2'$, $H_3'$ ; 9,56 (d) : CH-N$\underline{H}$-CO ; 7,35 (m) : ~ 2H ; 9,87 (sl) : 1H ; 9,96 (sl) : 1H ; H mobile.

**<u>EXEMPLE 48</u> : Sel interne de (6R-(3-(E) 6alpha, 7béta-(Z) (R*))) 1-(3-(7-(((2-amino 4-thiazolyl) ((carboxy (2,5-dichloro 3,4-dihydroxy phényl) méthoxy) imino) acétyl) amino) 2-carboxy 8-oxo 5-thia 1-azabicyclo[4,2,0]oct-2-èn-3-yl) 2-propényl) imidazo(1,2-a) pyridinium**

Spectre RMN : DMSO 300 MHz

5,15 (d,J= 5) : $H_6$ ; 5,27 (m) : $C\underline{H}_2$-N⊕ **;** 5,78 (d,J= 5) : $H_7$ ; 5,81 (s) : O-C$\underline{H}$-Φ, ~ 1,7 H ; 6,24 (dt,J= 16 et 6) : =C$\underline{H}$-$CH_2$, ~ 0,85 H ; 6,84 (s)-6,99 (s) : ~ 1,7 H, $H_5$ et 1H aromatique ; 6,85 (d,J= 16) : =C-C$\underline{H}$=CH ; 7,57 (dd,J= 6 et 7) : $H_5'$ ; 8,05 (dd, J= 7 et 8,5) : $H_6'$ ; 8,17 (d,J= 8,5) : $H_7'$ ; 8,94 (d,J= 6) : $H_4'$ : 8,26 (d,J= 2)-8,42 (d,J= 2) : $H_2'$, $H_3'$.

En opérant de manière analogue à celle décrite dans les exemples précédents, à partir des intermédiaires appropriés, on a obtenu les produits suivants :

**<u>EXEMPLE 49</u> : Sel interne de (6R-(3-(E) 6alpha, 7béta-(Z))) 1-(3-(7-(((2-amino 4-thiazolyl) ((carboxy (2,3-dihydroxy 4-méthoxy phényl) méthoxy) imino) acétyl) amino) 2-carboxy 8-oxo 5-thia 1-azabicyclo[4,2,0]oct-2-èn-3-yl) 2-propényl) quinolinium**

Spectre RMN : DMSO 300 MHz

3,52 (d)-3,70 : $CH_2S$ ; 3,72-3,74 : C-OMe ; 5,15 (d, dédoublé) : $H_6$ ; 5,57 (dd)-5,80 (m) : $H_7$ ; 5,83-5,87 :

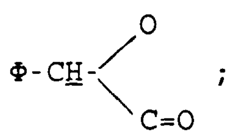

5,70 à 6,0 : $CH_2$-N⊕ ; 6,42 (d, dédoublé)-6,75 (d, dédoublé) H aromatique ; 6,40 (m)-6,99 (d, dédoublé, J~16) : éthylène (ΔE) ; 6,78-6,82 : $H_5$ thiazole ; 8,07 (t)-8,26 : $H_3'$, $H_6'$, $H_7'$ ; 8,53 (d)-8,56 (d)-9,34 (d) : $H_4'$, $H_5'$; 9,53 (d) : $H_8'$ ; 9,45 (d)-9,56 (d) :

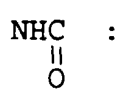

7,45 et 8,84 : H mobile.

**EXEMPLE 50 : Sel interne de (6R-(3-(E) 6alpha, 7béta-(Z))) 7-(3-(7-(((2-amino 4-thiazolyl) ((carboxy (2,3-dihydroxy 4-méthoxy phényl) méthoxy) imino) acétyl) amino) 2-carboxy 8-oxo 5-thia 1-azabicyclo[4,2,0)oct-2-èn-3-yl) 2-propényl) thiéno(2,3-b) pyridinium**

Spectre RMN : DMSO 400 MHz

3,54 (d)~3,68 (d) : =C-$CH_2$S ; 5,18 (m) : les $H_6$ ; 5,76 (m)-5,96 (m) : les $H_7$ ; 5,72 (s)-5,74 (s) : les Φ-OMe ; 5,67 (m) : $C\underline{H}_2$-CH= ; 5,83 (s)-5,87 (s) : C-C$\underline{H}$-Φ ; ~ 6,80 (s) dédoublé : les H thiazole ; 6,42 (m)-6,73 (m) : $H_5$, $H_6$ ; 6,30 (m)-7,15 (d) dédoublé : =C-CH=C$\underline{H}$-CH (ΔE) ; 7,88 (d) : $H_3$' ; 8,29 (d) : $H_2$' ; 8,15 (m) $H_5$' ; 9,08 (d) : $H_4$' ; 9,22 (d) : $H_6$' ; 9,44 (d)-9,53 (d) : =C-N$\underline{H}$-CH ; ~ 7,44 (m)-8,60 à 8,90 : H mobiles.

**EXEMPLE 51 : Sel interne de (6R-(3-(E) 6alpha, 7béta-(Z))) 1-(3-(7-(((2-amino 4-thiazolyl) ((carboxy (2,3-dihydroxy 4-méthoxy phényl) méthoxy) imino) acétyl) amino) 2-carboxy 8-oxo 5-thia 1-azabicyclo[4,2,0]oct-2-èn-3-yl) 2-propényl) imidazo(1,2-a) pyridinium**

Spectre RMN : DMSO 300 MHz

7,35 (m)-8,69 (m)-8,82 (m) : H mobiles ; 3,52 (m)~3,70 (m) : $CH_2$S ; 3,74 (s)-3,75 (s) : $C\underline{H}_3$O-Φ ; 5,15 (d, dédoublé) : $H_6$ ; 5,74 (dd, d après éch.)-5,81 (dd, d après éch.) : $H_7$ ; 5,29 (m) : $C\underline{H}_2$-N⊕ : 5,83 (s)-5,86 (s) : O-C$\underline{H}$-Φ ; 6,25 (m)-6,90 (d, dédoublé) : CH=C$\underline{H}$-$CH_2$ ; 6,43 (d) : $H_6$' ; 6,72 à 6,80 (m) : $H_5$' et $H_5$ thiazole ; 7,58 (t) : $H_5$" ; 8,06 (t) : $H_6$" ; 8,21 (d)-8,96 (d) : $H_4$", $H_7$" ; 8,29 (sl) : $H_3$" ; 8,44 (sl) : $H_2$" ; 9,44 (d)-9,54 (d) : CO-N$\underline{H}$-CH.

**EXEMPLE 52 : Sel interne de (6R-(3-(E) 6alpha, 7béta-(Z))) 1-(3-(7-(((2-amino 4-thiazolyl) ((carboxy (2,3,4-trihydroxy phényl) méthoxy) imino) acétyl) amino) 2-carboxy 8-oxo 5-thia 1-azabicyclo[4,2,0]oct-2-èn-3-yl) 2-propényl) imidazo(1,2-a) pyridinium**

Spectre RMN : DMSO (300 MHz)

3,54 (m) : $CH_2$S ; 5,15 (d) : $H_6$ ; 5,82 (m) : $H_7$ et O-C$\underline{H}$-Φ ; 5,28 (m) : =CH-C$\underline{H}_2$-N⊕ ; 6,26 (m) : C$\underline{H}$= ; 6,5 à 7,4 (m) : 6H aromatique ; 7,58 (t) : $H_5$' ; 8,06 (t) : $H_6$' ; 8,20 (d), 8,96 (d) : $H_4$', $H_7$' ; 8,29 (sl)-8,44 (sl) : $H_2$', $H_3$' ; 9,41 (d)-9,53 (d) : CO-NH-CH ; 8,68 (m)-9,27 (m)-7,36 (m) : H mobiles.

**EXEMPLE 53 : Sel interne de (6R-(3-(E) 6alpha, 7béta-(Z))) 1-(3-(7-(((2-amino 4-thiazolyl) ((carboxy (2,3-difluoro 4,5-dihydroxy phényl) méthoxy) imino) acétyl) amino) 2-carboxy 8-oxo 5-thia 1-azabicyclo [4,2,0]oct-2-èn-3-yl) 2-propényl) imidazo(1,2-a) pyridinium**

Spectre RMN : DMSO (300 MHz)

~ 3,70 : $CH_2$S ; 5,09 (d)-5,14 (d) : $H_6$ ; 5,29 (m) : $CH_2$-N⊕ ; 5,58 (s)-5,59 (s) : O-CH-Φ ; 5,72 (m) : $H_7$ ; 6,22 (m) : CH=C$\underline{H}$-$CH_2$ ; 6,68 (m) : $H_6$' ; 6,77 (s)-6,78 (s) : $H_5$ thiazole ; 6,85 (d,J=16) : =C-C$\underline{H}$=CH- ; 7,58 (t)-8,06 (t) : $H_5$", $H_6$" ; 8,19 (d)-8,96 (d) : $H_4$", $H_7$" ; 8,28 (m)-8,44 (sl) : $H_2$", $H_3$" ; 7,32 (m) : 9,5 à 9,9 (m) : H mobiles.

**EXEMPLE 54 : Sel interne de (6R-(3-(E) 6alpha, 7béta-(Z))) 1-(3-(7-(((2-amino 4-thiazolyl) ((carboxy (2,3-difluoro 4,5-dihydroxy phényl) méthoxy) imino) acétyl) amino) 2-carboxy 8-oxo 5-thia 1-azabicyclo[4,2,0]oct-2-èn-3-yl) 2-propényl) quinoléinium**

Spectre RMN : DMSO (300 MHz)

3,45 (m) : $CH_2$S ; 5,09 (d)-5,14 (d) : $H_6$ ; 5,72 (m) : $H_7$ ; 5,58 (s)-5,59 (s) : O-C$\underline{H}$-CO ; 5,88 (m) : $C\underline{H}_2$-N⊕ ; 6,34 (m) : CH=C$\underline{H}$-$CH_2$ ; 6,94 (d,J=16)-6,97 (d,J=16) : C$\underline{H}$=CH-$CH_2$ ; 6,67 (m) : $H_6$" ; 6,76 (s)-6,77 (S) : $H_5$ thiazole ; 8,07 (m)-8,26 (m)-8,53 (m)-9,34 (d)-9,57 (d) : quinoléine ; 7,32 (m)-9,5 à 9,9 (m) : H mobiles.

**<u>EXEMPLE 55</u> : Sel interne de (6R-(3-(E) 6alpha, 7béta-(Z))) 1-(3-(7-(((2-amino 4-thiazolyl) ((carboxy (2,5-difluoro 3,4-dihydroxy phényl) méthoxy) imino) acétyl) amino) 2-carboxy 8-oxo 5-thia 1-azabicyclo[4,2,0]oct-2-èn-3-yl) 2-propényl) pyridinium**

<u>Spectre RMN</u> : DMSO (300 MHz)

3,58 (m) : $CH_2S$ ; 5,18 (d,d) : $H_6$ ; 5,79 (m) : $H_7$ ; 5,41 (sl) : $CH_2N^{\oplus}$ ; 5,64 (s)-5,65 (s) : O-C$\underline{H}$-C= ; 6,30 (m) : CH=CH-$CH_2$ ; 7,02 (dd,J=16) : C$\underline{H}$=CH-$CH_2$ ; 6,70 (dd) : $H_6'$ ; 6,78 (s)-6,80 (s) : $H_5$ thiazole ; 8,18 (m) : $H_3''$, $H_5''$ ; 8,63 (m) : $H_4''$ ; 9,05 (d) : $H_2''$, $H_6''$ ; 9,35 (d)-9,62 (d) : CO-N$\underline{H}$CH ; 7,33 (m)-9,80 (m) : H mobile.

**<u>EXEMPLE 56</u> : Sel interne de (6R-(3-(E) 6alpha, 7béta-(Z))) 1-(3-(7-(((2-amino 4-thiazolyl) ((carboxy (2,5-difluoro 3,4-dihydroxy phényl) méthoxy) imino) acétyl) amino) 2-carboxy 8-oxo 5-thia 1-azabicyclo[4,2,0]oct-2-èn-3-yl) 2-propényl) 6,7-dihydro 5H-pyrindinium**

<u>Spectre RMN</u> : DMSO (300 MHz)

2,24 (m) : $CH_2$ en 6' ; 3,14 (m)-3,37 (m) : $CH_2$ en 5' et 7' ; 3,56 (m) : $CH_2S$ ; 5,15 (d)-5,18 (d) : $H_6$ ; 5,79 (m) : $H_7$ ; 5,33 (d) : =CH-C$\underline{H_2}$-$N^{\oplus}$ ; 5,63 (s)-5,65 (s) : O-C$\underline{H}$-Φ ; 6,23 (m) : CH=C$\underline{H}$-$CH_2$ ; 6,85 (d)-6,88 (d, J=16) : C$\underline{H}$=CH-$CH_2$ ; 6,71 (dd,J = 10,5 et 6) : $H_6''$ ; 6,78 (s)-6,81 (s) : $H_5$ thiazole ; 7,91 (m) : $H_3'$ ; 8,43 (d,J=8) : $H_4'$ ; 8,76 (d,J=6) : $H_2'$ ; 9,54 (d)-9,63 (d) : CH-NH-CO ; 7,35 (m)-9,85 (m) : H mobiles.

**<u>EXEMPLE 57</u> : Sel interne de (6R-(3-(E) 6alpha, 7béta-(Z))) 1-(3-(7-(((2-amino 4-thiazolyl) ((carboxy (2,5-difluoro 3,4-dihydroxy phényl) méthoxy) imino) acétyl) amino) 2-carboxy 8-oxo 5-thia 1-azabicyclo[4,2,0]oct-2-èn-3-yl) 2-propényl) quinoléinium**

<u>Spectre RMN</u> : DMSO (300 MHz)

3,4 à 3,7 (m) : $CH_2S$ ; 5,15 (dd) : $H_6$ ; 5,79 (m) : $H_7$ ; 5,62 (s)-5,64 (s) : -O-C$\underline{H}$-Φ ; 5,89 (m) : =CH-C$\underline{H_2}$-$N^{\oplus}$ ; 6,37 (m) : CH=C$\underline{H}$-$CH_2$ ; 6,97 (dd,J=15,5) : : C$\underline{H}$=CH-$CH_2$ ; 6,76 (s)-6,80 (s) : $H_5$ thiazole ; 6,70 (dd,J= 11 et 6) : $H_6''$ ; 8,07 (m)-8,27 (m)-8,53 (m)-9,34 (d) : quinoléïne ; 9,58 (d) : $H_2$ ; 9,53 (d)-9,62 (d) : CO-N$\underline{H}$-CH ; 7,30 (m)-9,83 (m) : H mobile.

**<u>EXEMPLE 58</u> : Sel interne de (6R-(3-(E) 6alpha, 7béta-(Z))) 3-(3-(7-(((2-amino 4-thiazolyl) ((carboxy (2,5-difluoro 3,4-dihydroxy phényl) méthoxy) imino) acétyl) amino) 2-carboxy 8-oxo 5-thia 1-azabicyclo[4,2,0]oct-2-èn-3-yl) 2-propényl) thiazolium**

<u>Spectre RMN</u> : DMSO (300 MHz)

3,50 à 3,75 (m) : $CH_2S$ ; 5,16 à 5,19 (d) : $H_6$ ; 5,81 (m) : $H_7$ ; 5,33 (m) : =CH-C$\underline{H_2}$-$N^{\oplus}$ ; 5,63 (s)-5,65 (s) : O-C$\underline{H}$-Φ ; 6,26 (m) : CH=C$\underline{H}$-$CH_2$ ; 6,96 (dd,J=16) : C$\underline{H}$=CH-$CH_2$ ; 6,71 (dd,J=6 et 11) : $H_6''$ ; 6,78 (s)-6,81 (s) : $H_5$ thiazole ; 8,37 (m)-8,51 (m) : $H_4'$, $H_5'$ ; 10,20 (sl) : $H_2'$ ; 7,30 (m) : 9,5 à 9,9 (m)-13,25 (m)-13,75 (m) : H mobiles.

**<u>EXEMPLE 59</u> : Sel interne de (6R-(3-(E) 6alpha, 7béta-(Z))) 2-(3-(7-(((2-amino 4-thiazolyl) ((carboxy (2,5-difluoro 3,4-dihydroxy phényl) méthoxy) imino) acétyl) amino) 2-carboxy 8-oxo 5-thia 1-azabicyclo[4,2,0]oct-2-èn-3-yl) 2-propényl) isoquinoléinium**

<u>Spectre RMN</u> : DMSO (300 MHz)

3,50 à 3,75 (m) : $CH_2S$ ; 5,18 (dd) : $H_6$ ; 5,80 (m) : $H_7$ ; 5,53 (d) : =CH-C$\underline{H_2}$-$N^{\oplus}$ ; 5,63 (s)-5,65 (s) : O-C$\underline{H}$-Φ ; 6,37 (m) : CH=C$\underline{H}$-$CH_2$ ; 7,09 (dd,J=15,5) : C$\underline{H}$=CH-$CH_2$ ; 6,71 (dd,J=11 et 6) : $H_6''$ ; 6,78 (s)-6,80 (s) : $H_5$ thiazole ; 8,09 (t)-8,28 (t) : $H_6'$, $H_7'$ ; 8,37 (d)-8,53 (d) : $H_5'$, $H_8'$ ; 8,61 (d)-8,74 (d) : $H_3'$, $H_4'$ ; 10,06 (s) : $H_1'$ ; 7,35 (m)-9,84 (m) : H mobiles ; 9,56 (d)-9,65 (d) : CH-N$\underline{H}$-CO.

**EXEMPLE 60 : Sel interne de (6R-(3-(E) 6alpha, 7béta-(Z))) 1-(3-(7-(((2-amino 4-thiazolyl) ((carboxy (2,5-difluoro 3,4-dihydroxy phényl) méthoxy) imino) acétyl) amino) 2-carboxy 8-oxo 5-thia 1-azabicyclo[4,2,0]oct-2-èn-3-yl) 2-propényl) 4-(méthylthio) pyridinium**

Spectre RMN : (DMSO) 300 MHz

2,71 (s) : ΦSMe ; 3,54 (d), 3,66 (d) : CH-S ; 5,17 (m) : les $H_6$ ; 5,22 (s,l) : =CH-$CH_2$-$N^+$ ; 5,63 (s) dédoublé : OCH-ϕ ; 5,79 (m) : les $H_7$ ; 6,26 (m) CH=CH-$CH_2$ ΔE, 6,98 (d,J=16) dédoublé : CH=CH-$CH_2$ ; 6,71 (dd, J=6 et 11) : $H_6$" ; 6,78 (s) dédoublé : $H_5$ thiazole ; 7,30 (s,l) : $NH_2$ ; 7,95 (d), 8,70 (d): N -S ; 9,62-9,67 (d), 9,85 : les H mobiles ; 19,49.

**EXEMPLE 61 : Sel interne de (6R-(3-(E) 6alpha, 7béta-(Z))) 4-(3-(7-(((2-amino 4-thiazolyl) ((carboxy (2,5-difluoro 3,4-dihydroxy phényl) méthoxy) imino) acétyl) amino) 2-carboxy 8-oxo 1-azabicyclo 5-thia 1-azabicyclo[4,2,0] oct-2-èn-3-yl) 2-propényl) 2-amino thiazolo (5,4-b)-pyridinium**

Spectre RMN : (DMSO) 300 MHz

~3,60 : $CH_2$ ; 5,19 (d) dédoublé : $H_6$ ; 5,81 (d) : $H_7$ ; 5,47 (d) : =CH-$CH_2$-$N^+$ ; 5,63 (s), 5,65 (s) : O-CH-Φ ; 6,21 (m) : CH=CH-$CH_2$ ; 7,08 (d) dédoublé J=16 : CH=CH-$CH_2$ ; 6,78 (s) : $H_5$ thiazole ; 6,70 (dd, J=6 et 11) : $H_6$" ; 7,83 (m) : $H_5$' ; 8,29 (d, J=8) : $H_4$' ; 8,64 (d, J=6) : $H_6$' ; 9,55 (d), 9,64 (d) : CH-NH-CO ; 8,73 (s), 9,84 (m), 7,33 (m) : H mobile.

**EXEMPLE 62 : Sel interne de (6R-(3-(E) 6alpha, 7béta-(Z))) 7-(3-(7-(((2-amino 4-thiazolyl) ((carboxy (2,5-difluoro 3,4-dihydroxy phényl) méthoxy) imino) acétyl) amino) 2-carboxy 8-oxo 5-thia 1-azabicyclo[4,2,0]oct-2-èn-3-yl) 2-propényl) thiéno (2,3-b)-pyridinium**

Spectre RMN : (DMSO)

3,4 à 3,8 : $CH_2S$ ; 5,18 (d, dédoublé) : $H_6$ ; 5,81 (dd, dédoublé) : $H_7$ ; 5,66 (m) 3H =CH-$CH_2$-$N^+$ et O-CH-Φ ; 6,30 (m) : CH=CH-$CH_2$ ; 7,14 (d, dédoublé, J=16 : CH=CH-$CH_2$ ; 6,70 (dd, J=6 et 11) : $H_6$" ; 6,77 (s), 6,81 (s) : $H_5$ thiazole ; 7,89 (d, J=6) : $H_3$' ; 8,28 (d, J=6) : $H_2$' ; 8,16 (m) : $H_5$' ; 9,09 (d, J=8) : $H_4$' 9,23 (d, J=6) : $H_6$' ; 9,35 (d), 9,65 (d) : CO-NH-CH ; 7,32 (m), 9,83 (m) : H mobiles.

**EXEMPLE 63 : Sel interne de (6R-(3-(E) 6alpha, 7béta-(Z))) 7-(3-(7-(((2-amino 4-thiazolyl) ((carboxy (3,4-dihydroxy 2,5-difluorophényl) méthoxy) imino) acétyl) amino) 2-carboxy 8-oxo 5-thia 1-azabicyclo[4,2,0]oct-2-èn-3-yl) 2-propényl) imidazo (2,1-b)-thiazolium**

**EXEMPLE 64 : Sel interne de (6R-(3-(E) 6alpha, 7béta-(Z))) 3-(3-(7-(((2-amino 4-thiazolyl) ((carboxy (3,4-dihydroxy 2,5-difluorophényl) méthoxy) imino) acétyl) amino) 2-carboxy 8-oxo 5-thia 1-azabicyclo[4,2,0]oct-2-èn-3-yl) 2-propényl) 1-méthyl 1H-benzimidazalium**

**EXEMPLE 65 : Sel interne de (6R-(3-(E) 6alpha, 7béta-(Z))) 7-(3-(7-(((2-amino 4-thiazolyl) ((carboxy (3,4-dihydroxy 2,5-difluorophényl) méthoxy) imino) acétyl) amino) 2-carboxy 8-oxo 5-thia 1-azabicyclo[4,2,0]oct-2-èn-3-yl) 2-propényl) 4-méthyl thieno (2,3-b)-pyridinium**

En plus des produits décrits ci-dessus dans les exemples, les produits suivants constituent des produits pouvant être obtenus selon les méthodes de l'invention :

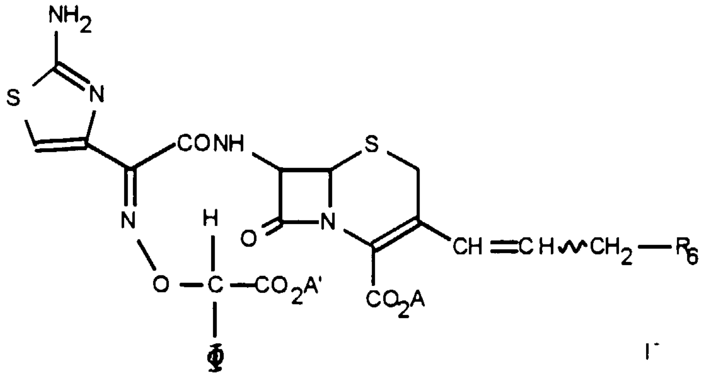

$I^-$

| Φ | R6 | Φ | $R_6$ |
|---|---|---|---|
| 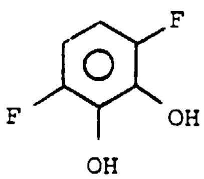 | 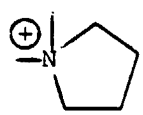 | 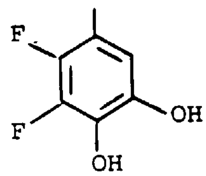 | 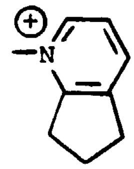 |
| " | 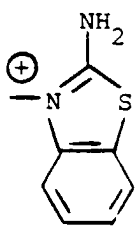 | " | 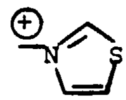 |
| 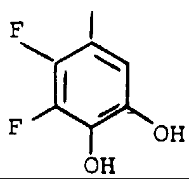 | 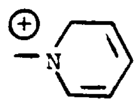 | " | 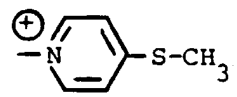 |

| Φ | $R_6$ | Φ | $R_6$ |
|---|---|---|---|
| 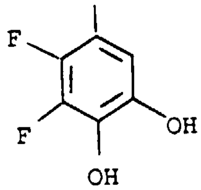 | 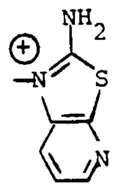 | 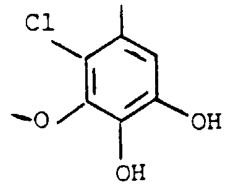 | 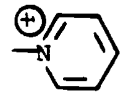 |
| " | 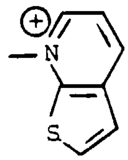 | " | 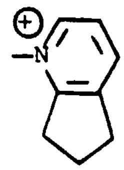 |
| " | 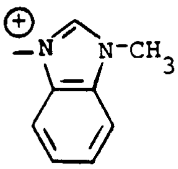 | " | 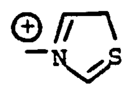 |
| " | 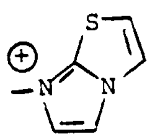 | " | 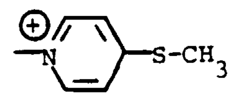 |
| " | 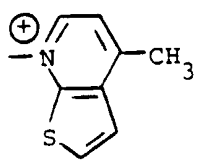 | " | 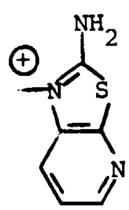 |
| " | | " | 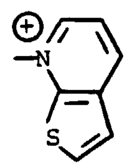 |
| " | 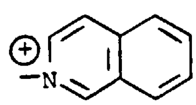 | " | 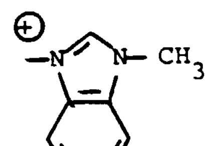 |
| " | | " | 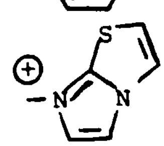 |
| | | " | 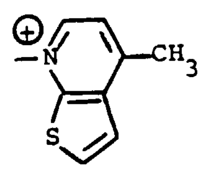 |

| Φ | $R_6$ | Φ | $R_6$ |
|---|---|---|---|
| 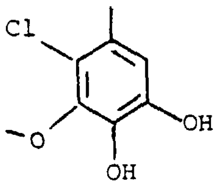 | 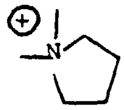 | 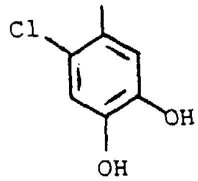 | 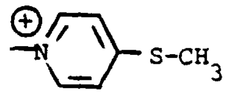 |
| " | 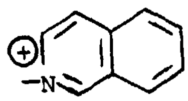 | " | 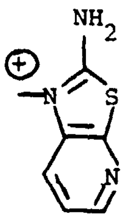 |
| " | 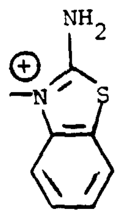 | " | 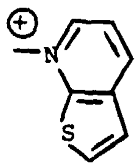 |
| 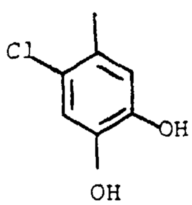 | 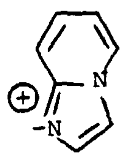 | " | 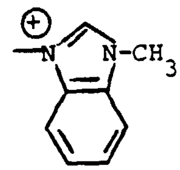 |
| " | 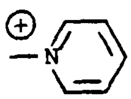 | " | 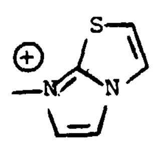 |
| " | 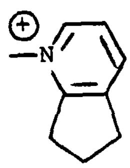 | " | 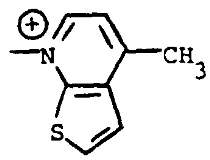 |
| " | 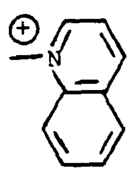 | " | 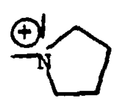 |
| " | 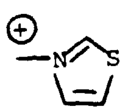 | " | 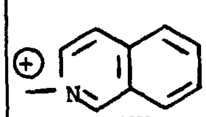 |
| | | " | 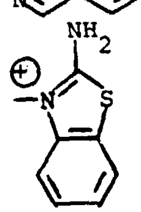 |

| Φ | $R_6$ | Φ | $R_6$ |
|---|---|---|---|
| 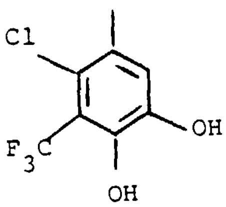 | 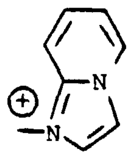 | 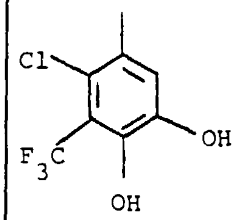 | 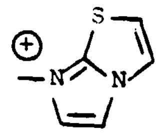 |
| " | 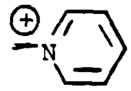 | " | 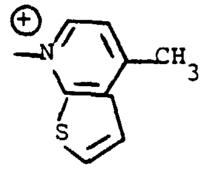 |
| " | 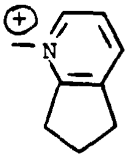 | " | 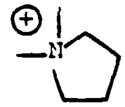 |
| " | 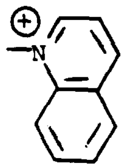 | " | 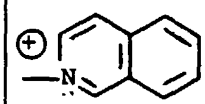 |
| " | 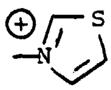 | " | 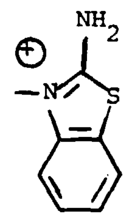 |
| " | 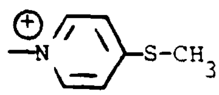 | 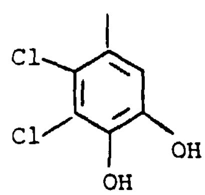 | 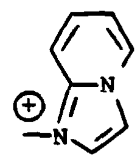 |
| " | 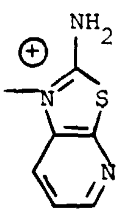 | " | 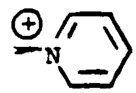 |
| " | 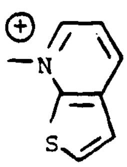 | " | 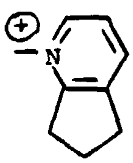 |
| " | 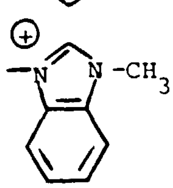 | " | 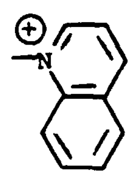 |

| Φ | $R_6$ | Φ | $R_6$ |
|---|---|---|---|
| 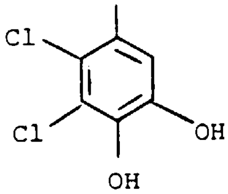 | 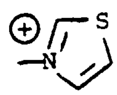 | 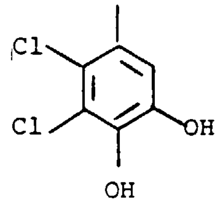 | 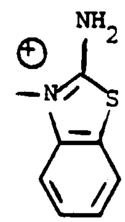 |
| " | 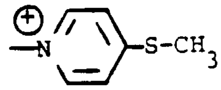 | | |
| " | 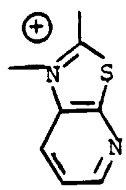 | 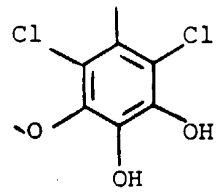 | 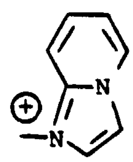 |
| " | 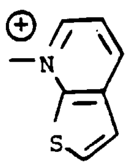 | " | 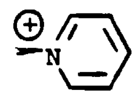 |
| " | 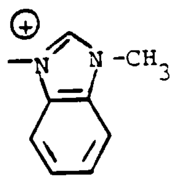 | " | 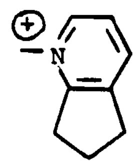 |
| | | " | 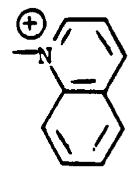 |
| " | 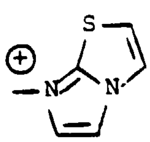 | " | 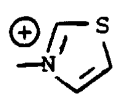 |
| " | 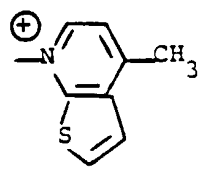 | " | 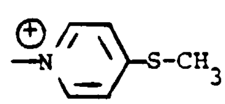 |
| " | 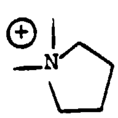 | " | 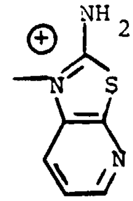 |
| " | 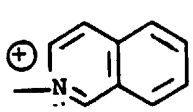 | | |

| Φ | $R_6$ | Φ | $R_6$ |
|---|---|---|---|
| 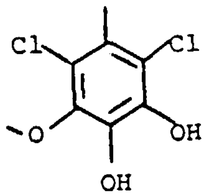 | 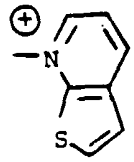 | 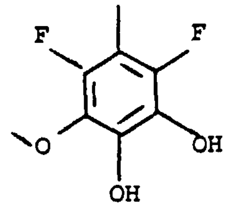 | 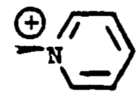 |
| " | 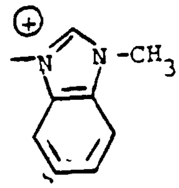 | " | 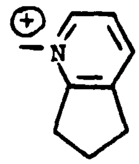 |
| " | 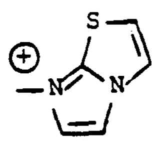 | " | 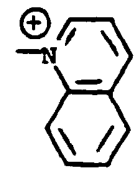 |
| " | 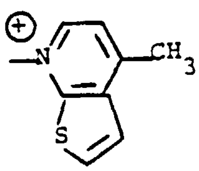 | " | 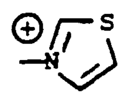 |
| " | 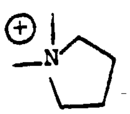 | " | 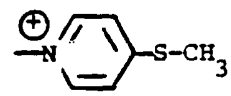 |
| " | 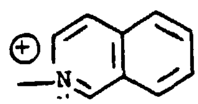 | " | 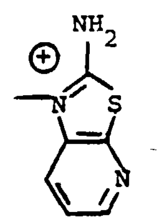 |
| " | 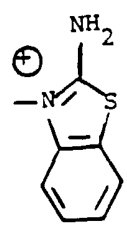 | " | 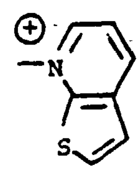 |
| 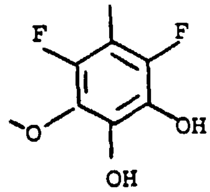 | 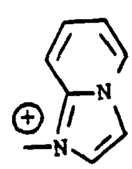 | " | 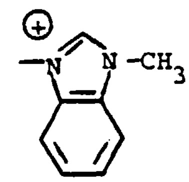 |
| | | " | 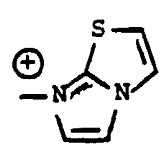 |

| Φ | $R_6$ | Φ | $R_6$ |
|---|---|---|---|
| 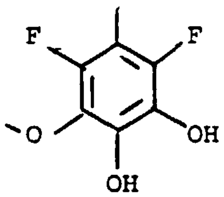 | 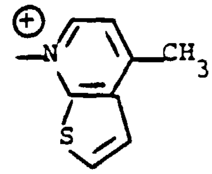 | 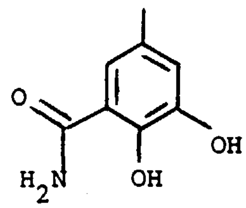 | 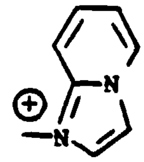 |
| " | 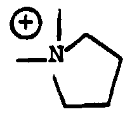 | " | 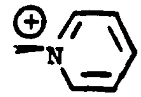 |
| " | 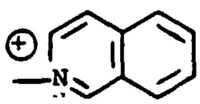 | " | 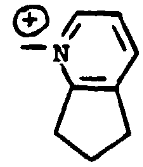 |
| " | 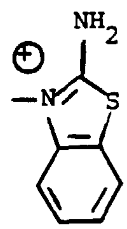 | " | 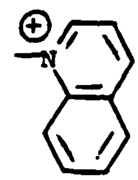 |

| Φ | $R_6$ | Φ | $R_6$ |
|---|---|---|---|
| 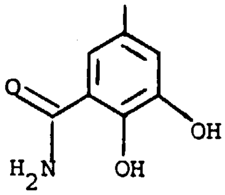 | 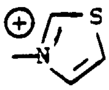 | 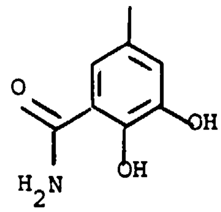 | 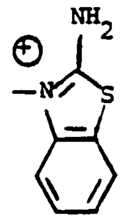 |
| " | 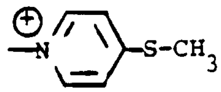 | 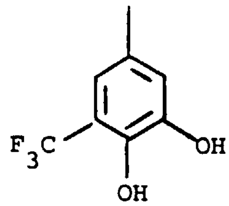 | 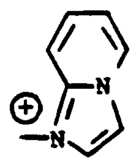 |
| " | 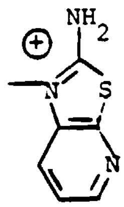 | " | 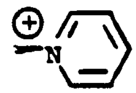 |
| " | 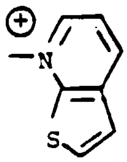 | " | 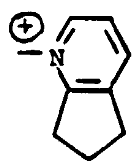 |
| " | 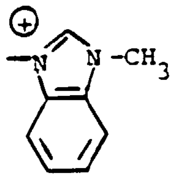 | " | 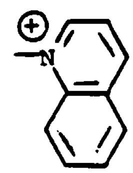 |
| " | 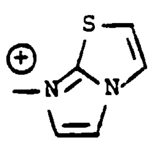 | " | 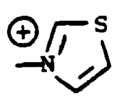 |
| " | 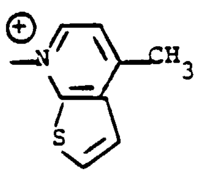 | " | 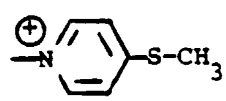 |
| " | 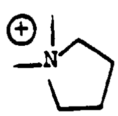 | " | 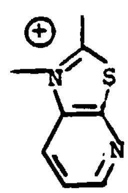 |
| " | 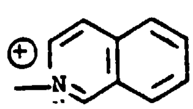 | | |

| Φ | $R_6$ | Φ | $R_6$ |
|---|---|---|---|
| 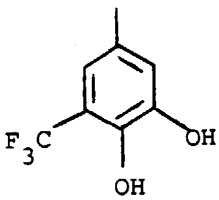 | 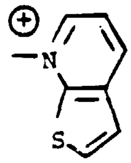 | 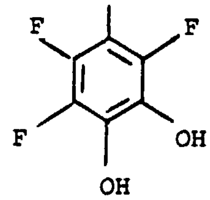 | 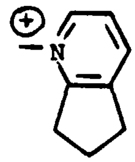 |
| " | 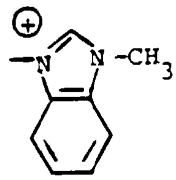 | " | 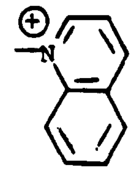 |
| " | 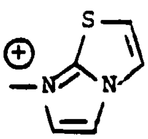 | " | 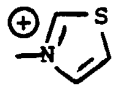 |
| " | 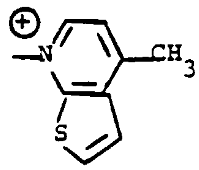 | " | 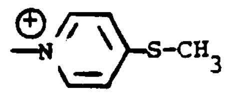 |
| " | 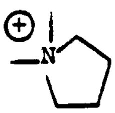 | " | 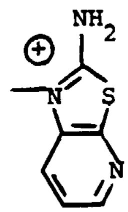 |
| " | 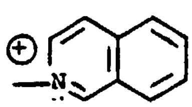 | " | 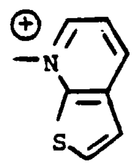 |
| " | 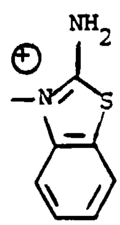 | " | 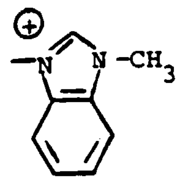 |
| 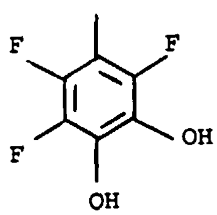 | 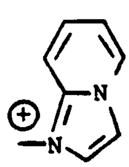 | " | 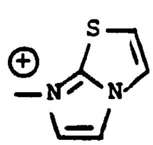 |
| | 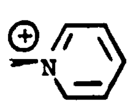 | | |

| Φ | $R_6$ | Φ | $R_6$ |
|---|---|---|---|
| 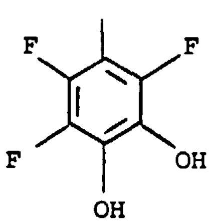 | 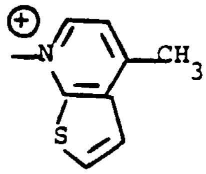 | 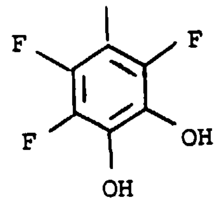 | 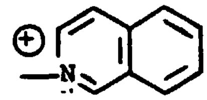 |
| " | 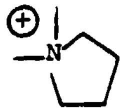 | " | 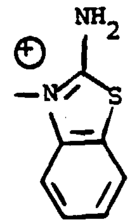 |

**<u>EXEMPLE 66</u> : On a réalisé des préparations pour injections de formule :**

- Produit de l'exemple 2 500 mg
- Excipient aqueux stérile q.s.p 5 $cm^3$

**<u>ETUDE PHARMACOLOGIQUE DES PRODUITS DE L'INVENTION</u>**

**Activité in vitro, méthode des dilutions en milieu liquide.**

On prépare une série de tubes dans lesquels on répartit une même quantité de milieu nutritif stérile. On distribue dans chaque tube des quantités croissantes du produit à étudier, puis chaque tube est ensemencé avec une souche bactérienne. Après incubation de vingt-quatre heures à l'étuve à 37°C, l'inhibition de la croissance est appréciée. par transillumination ce qui permet de déterminer les concentrations minimales inhibitrices (C.M.I.) exprimées en $\mu g/cm^3$ (TABLEAU I).

**Activité in vitro, méthode des dilutions en milieu solide.**

On prépare une série de boites dans lesquelles on répartit une même quantité de milieu nutritif stérile, contenant des quantités croissantes du produit à étudier puis chaque boite est ensemencée avec plusieurs souches bactériennes.
Après incubation de 24 heures en étuve à 37°C, l'inhibition de la croissance est appréciée par l'absence de tout développement bactérien ce qui permet de déterminer les concentrations minimales inhibitrices (CMI) exprimées en microgrammes/$cm^3$.
Les résultats sont exprimés en $CMI_{90}$ qui est la concentration minimum d'antibiotique permettant d'inhiber la croissance de 90 % des souches étudiées (TABLEAU II).
On a obtenu les résultats suivants :

CMI

TABLEAU I

| Produit de l'exemple | Enterobacteries Cloacae 1321E | Staphylocoques aureus SG 5 11 | Proteus A 235 | Pseudomonas Aeruginosa 1771 m |
|---|---|---|---|---|
| 9 | 0,04 | 0,08 | 0,04 | 0,3 |
| 3 | 0,08 | 0,15 | 0,04 | 0,6 |
| 1 | 0,08 | 0,15 | 0,04 | 0,6 |
| 2 | 0,15 | 0,30 | 0,02 | 0,3 |

$CMI_{90}$

TABLEAU II

| Produit de l'exemple | Staphylocoques aureus (20 souches) pénicilline resis-tants oracilline sensible | Entérobactéries producteurs de céphalospori-nases (20 souches) | Entérobactéries producteurs de ß lactamase à spectre élargi (16 souches) | Pseudomonas Aeruginosa (39 souches) |
|---|---|---|---|---|
| 39 | 2,5 | 0,3 | 2,5 | 0,08 |
| 43 | 0,3 | 2,5 | 1,2 | 1,2 |
| 27 | 5 | 0,3 | 10 | 0,08 |

**Revendications**

**1.** Les produits de formule générale (I) :

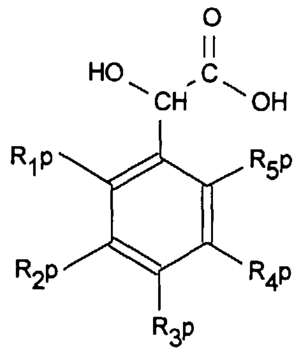

(III)

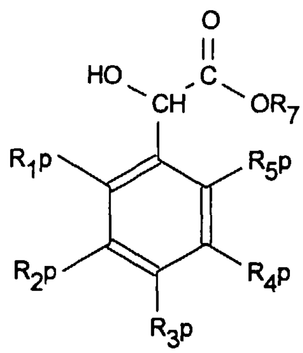

(IV)

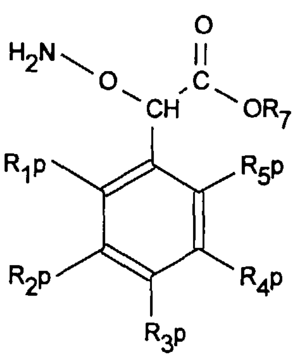

(V)

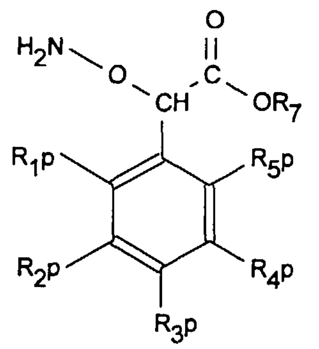

(VI)

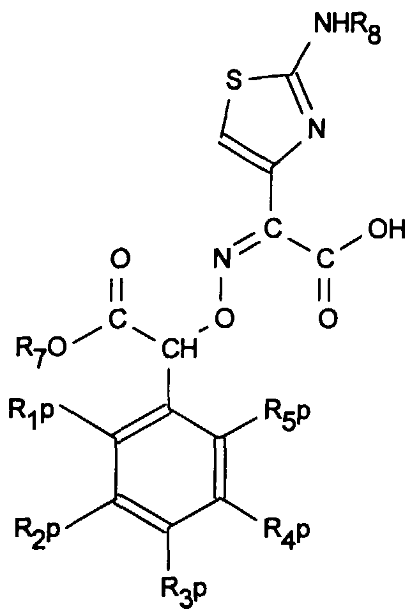

(VIII)

formules dans lesquelles $R_{1p}$, $R_{2p}$, $R_{3p}$, $R_{4p}$ et $R_{5p}$ représentent respectivement les valeurs de $R_1$, $R_2$, $R_3$, $R_4$ et $R_5$ telles que définies ci-après ou une fonction réactive protégée, $R_1$, $R_2$, $R_3$ et $R_5$, identiques ou différents, représentent un atome d'hydrogène, un atome d'halogène ou un des radicaux choisis parmi le radical hydroxy, alkyle renfermant de 1 à 4 atomes de carbone, éventuellement substitué par un ou plusieurs atomes d'halogène, alkyloxy renfermant de un à quatre atomes de carbone, mercapto, alkylthio renfermant de 1 à 4 atomes de carbone, nitro, cyano, amino, alkylamino renfermant de 1 à 4 atomes de carbone, dialkylamino renfermant de 2 à 8 atomes de carbone, carbamoyle, (alkylamino) carbonyle renfermant de 2 à 5 atomes de carbone, (dialkylamino) carbonyle renfermant de trois à neuf atomes de carbone, carboxy, alcoxycarbonyle renfermant de 2 à 5 atomes de carbone, acyloxy renfermant de 1 à 8 atomes de carbone et

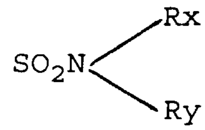

dans lequel Rx et Ry,identiques ou différents, représentent un atome d'hydrogène ou un radical alkyle renfermant de 1 à 4 atomes de carbone,

$R_4$ représente un radical hydroxy ou un radical acyloxy renfermant de 1 à 8 atomes de carbone, $R_3$ ou $R_5$ représentant nécessairement le même radical Hydroxy ou Acyloxy que $R_4$, étant entendu que :

- soit $R_1$, $R_2$ ou $R_3$, soit $R_2$, $R_4$ ou $R_5$ représente nécessairement un atome de Fluor, de Brome, d'Iode ou un

radical Cyano,

- soit $R_1$ et $R_5$ sont tous les deux différents d'hydrogène,
- soit $R_2$ et $R_5$ représentent tous les deux un atome de chlore,

$R_7$ représente le reste d'un ester aisément clivable et $R_8$ représente un atome d'hydrogène ou un groupe protecteur de la fonction amine.

Office européen des brevets

# RAPPORT DE RECHERCHE EUROPEENNE

Numéro de la demande
EP 00 20 0918

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | PATENT ABSTRACTS OF JAPAN vol. 015, no. 163 (C-0826), 24 avril 1991 (1991-04-24) & JP 03 034986 A (HISAYASU ISHIMARU), 14 février 1991 (1991-02-14) * abrégé * | 1 | C07C59/48 C07C59/64 C07C69/732 C07C239/20 C07C255/57 C07D277/40 |
| X | EP 0 131 174 A (BEECHAM GROUP PLC) 16 janvier 1985 (1985-01-16) * page 28 * | 1 | |

DOMAINES TECHNIQUES RECHERCHES (Int.Cl.7)
C07C
C07D

Le présent rapport a été établi pour toutes les revendications

| Lieu de la recherche | Date d'achèvement de la recherche | Examinateur |
|---|---|---|
| LA HAYE | 10 mai 2000 | Pauwels, G |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 00 20 0918

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

10-05-2000

| Document brevet cité au rapport de recherche | Date de publication | Membre(s) de la famille de brevet(s) | Date de publication |
|---|---|---|---|
| JP 03034986 A | 14-02-1991 | AUCUN | |
| EP 0131174 A | 16-01-1985 | AU 3032184 A | 10-01-1985 |
| | | CA 1057730 A | 03-07-1979 |
| | | DE 3479731 D | 19-10-1989 |
| | | ES 534066 D | 16-09-1985 |
| | | ES 8507485 A | 16-12-1985 |
| | | ES 542291 D | 16-12-1985 |
| | | ES 8603494 A | 16-04-1986 |
| | | ES 542292 D | 16-12-1985 |
| | | ES 8603493 A | 16-04-1986 |
| | | ES 542293 D | 16-01-1987 |
| | | ES 8702886 A | 01-04-1987 |
| | | GR 82197 A | 13-12-1984 |
| | | JP 60089487 A | 20-05-1985 |
| | | US 4684639 A | 04-08-1987 |
| | | ZA 8405162 A | 29-05-1985 |

EPO FORM P0460

Pour tout renseignement concernant cette annexe : voir Journal Officiel de l'Office européen des brevets, No.12/82